# EUROPEAN PATENT SPECIFICATION

(11) **EP 1 175 417 B1**
(45) Date of publication and mention of the grant of the patent: **07.01.2004**
(21) Application number: 00914335.5
(22) Date of filing: 13.04.2000
(51) Int. Cl.: C07D 401/12, C07D 453/02, C07D 413/12, C07D 417/12, C07D 215/22, C07D 209/34, A61K 31/4709, A61K 31/404, A61P 25/00

(54) **SUBSTITUTED BENZOLACTAM COMPOUNDS**
SUBSTITUIERTE BENZOLACTAMVERBINDUNGEN
COMPOSES DE BENZOLACTAME SUBSTITUES

(30) Priority: 06.05.1999 US 132858 P
(43) Date of publication of application: 30.01.2002
(73) Proprietor: Pfizer Products Inc., Groton, Connecticut 06340 (US)
(72) Inventor: SOBOLOV-JAYNES, S. B., Groton, CT 06340 (US)
(74) Representative: Wood, David John
(86) International application number: PCT/IB2000/000461
(87) International publication number: WO 2000/068224

(56) References cited:
- EP-A- 0 780 375
- WO-A-94/13663
- WO-A-97/03066
- WO-A-97/08144
- WO-A-99/25714

## Description

This invention relates to substituted benzolactam and cyclic thioamide compounds of interest to those in the field of medical chemistry and chemotherapy. More particularly, it is concerned with a series of substituted benzolactam and cyclic thioamide compounds, including their pharmaceutically acceptable salts, which are of special value in view of their ability to antagonize the action of substance P. These compounds are of use in treating gastrointestinal disorders, central nervous system (CNS) disorders, inflammatory diseases, emesis, urinary incontinence, pain, migraine, angiogenesis or the like, especially CNS disorders in a mammalian subject, especially humans.

Substance P is a naturally occurring undecapeptide belonging to the tachykinin family of peptides, the latter being so-named because of their prompt stimulatory action on smooth muscle tissue. More specially, substance P is a pharmaceutically active neuropeptide that is produced in mammals (having originally been isolated from the gut) and possesses a characteristic amino acid sequence that is illustrated by D. F. Veber *et al* in U.S. Patent No. 4,680,283. The wide involvement of substance P and other tachykinins in the pathophysiology of numerous diseases has been amply demonstrated in the art. For instance, substance P has recently been shown to be involved in the transmission of pain or migraine, as well as in central nervous system disorders such as anxiety and schizophrenia, in respiratory and inflammatory diseases such as asthma and rheumatoid arthritis, respectively, and in gastrointestinal disorders and diseases of GI tract, like ulcerative colitis and Crohn's diseases, *etc*. It is also reported that the tachykinin antagonists are useful for the treatment of allergic conditions, immunoregulation, vasodilation, bronchospasm, reflex or neuronal control of the viscera and senile dementia of the Alzheimer type, emesis, sunburn and *Helicobacter pylori* infection.

International Publication No. WO 94/13663 discloses a wide variety of azaheterocyclic compounds as tachykinin antagonists such as substance P antagonists. Compounds of the formula (I), as defined below, are referred to generically in World Patent Publication WO 97/03066, which was published on January 30, 1997.

According to the present invention, there is provided the use of a compound of the chemical formula (I): 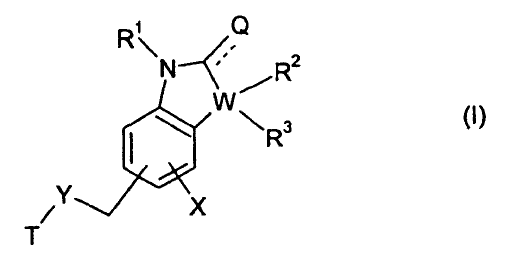 wherein W is methylene, ethylene, propylene, vinylene, -CH₂O-, -OCH₂-, -CH₂S-, or -SCH₂-;
R¹, R² and R³ are independently hydrogen, (C₁-C₃)alkyl, (C₁-C₃)alkoxy(C₁-C₃)alkyl, or halo(C₁-C₃)alkyl, provided that when W is methylene, R² and R³ cannot both be hydrogen;
or one of R² or R³ may be hydroxy;
X is halo, (C₁-C₃)alkoxy, (C₁-C₃)alkyl, halo(C₁-C₃)alkoxy, or (C₁-C₃)alkenyl;
Y is -NH- or -O-;
Q is oxygen or sulfur and is double bonded to the carbon to which it is attached or Q is CH₃ and is single bonded to the carbon to which it is attached;
T is (*2S,3S*)-2-diphenylmethylquinuclidin-3-yl, (*2S,3S*)-2-diphenylmethyl-1-azanorboman-3-yl, or (*2S,3S*)-2-phenylpiperidin-3-yl, wherein the phenyl group of said (2S, 3S)-2-phenylpiperidine-3-yl may optionally be substituted with one or more substituents, preferably with from zero to three substituents independently selected from halo, (C₁-C₆)alkyl optionally substituted with from one to seven fluorine atoms, (C₁-C₆)alkoxy optionally substituted with from one to seven fluorine atoms, amino, cyano, nitro, (C₁-C₆)alkylamino and di(C₁-C₆)alkylamino; and
the dashed line represents an optional double bond;
with the proviso that R¹ cannot be (C₁-C₃)alkoxy-CH₂- or halo-CH₂-;
or a pharmaceutically acceptable salt thereof,
in the manufacture of a medicament for treating a disorder or condition selected from obsessive-compulsive disorder, panic disorder, social phobia, agoraphobia, post traumatic stress disorder, borderline personality disorder, inflammation of the urinary tract, conduct disorder, disruptive behavior disorder, bipolar disorder, movement disorders associated with Tourette's syndrome, akinetic-rigid syndrome, movement disorders associated with Parkinson's disease, tardive dyskinesia, eating disorders selected from anorexia nervosa and bulimia nervosa, attention deficit hyperactivity disorder, chronic fatigue syndrome, premature ejaculation, premenstrual syndrome, premenstrual dysphoric disorder, gastroesophageal reflux disease, fibromyalgia, post-herpetia neuralgia, cystitis and irritable bowel syndrome, in a mammal.

Preferred compounds of formula (I) for use in the manufacture of a medicament in accordance with the invention include those wherein
Y is -NH-;
T is (2S,3S)-2-phenylpiperidin-3-yl, where the phenyl group of said (2S,3S)-2-phenylpiperidine-3-yl may optionally be substituted with fluoro;
Q is oxygen and is double bonded to the carbon atom to which it is attached;
X is methoxy or ethoxy;
R¹ is hydrogen, methyl, or halo(C₁-C₂)alkyl;
W is methylene, ethylene, or vinylene; and
R² and R³ are independently hydrogen or methyl, or one of R² or R³ may be hydroxy, when W is ethylene, or R² and R³ are both methyl, when W is methylene, or R² and R³ are both hydrogen, when W is vinylene.

Particularly preferred compounds of formula (I) for use in the manufacture of a medicament in accordance with the invention include the following and their pharmaceutically acceptable salts:

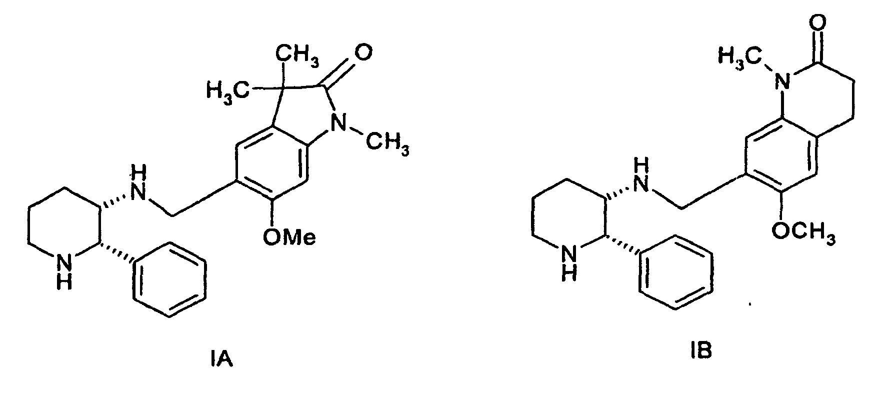

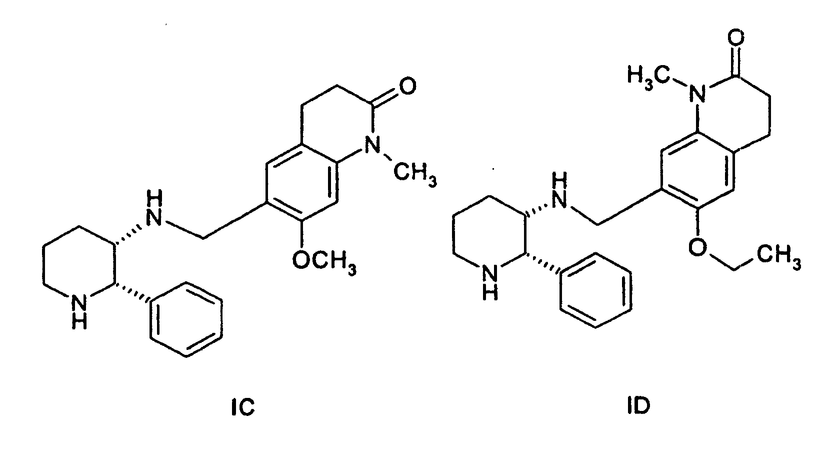

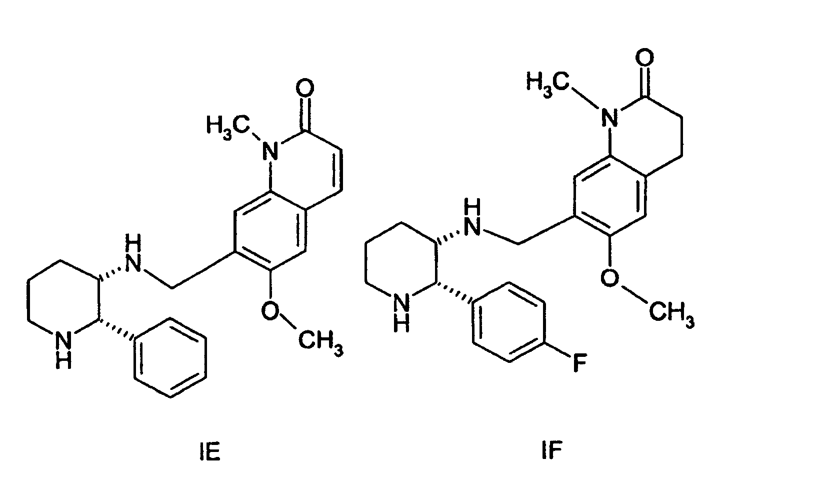

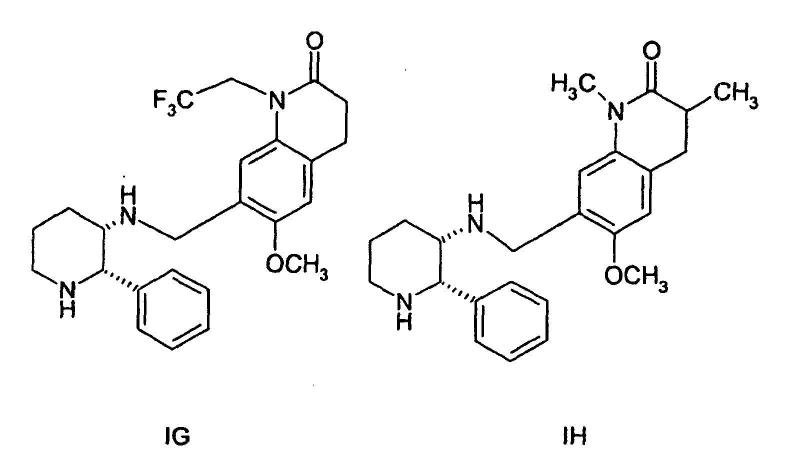

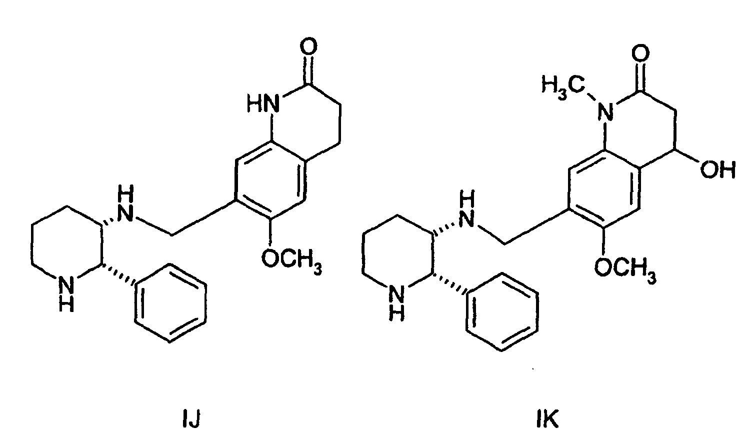

Compounds of the formula (I) are especially valuable for use in the manufacture of medicaments for the treatment of the disorders and conditions enumerated above because, in addition to their ability to antagonise substance P at its receptor sites, their major clearance mechanism in humans is not CYP2D6-mediated oxidative biotransformation. These compounds, therefore, when administered as drugs, do not have the variable plasma levels associated with drugs for which the major clearance mechanism in humans is CYP2D6-mediated biotransformation. Also, because these compounds are only weak inhibitors of CYP2D6, their administration to humans will not result in the drug-drug interactions typical of strong CYP2D6 inhibitors.

Protection is also sought for compounds of formula (I) ID, IF, IG, IH, IJ and IK and their pharmaceutically acceptable salts, for the use of these compounds and their pharmaceutically acceptable salts in the manufacture of a medicament for treating a disorder or condition selected from dysthymia, major depressive order, pediatric depression, generalized anxiety disorder, obsessive-compulsive disorder, panic disorder, phobias, social phobia, agoraphobia, post traumatic stress disorder, borderline personality disorder, cardiovascular disorders, ophthalmic disorders, inflammation of the urinary tract, conduct disorder, disruptive behavior disorder, bipolar disorder, movement disorders associated with Tourette's syndrome, akinetic-rigid syndrome, movement disorders associated with Parkinson's disease, tardive dyskinesia, memory disorders, eating disorders selected from anorexia nervosa and bulimia nervosa; attention deficit hyperactivity disorder, chronic fatigue syndrome, premature ejaculation, premenstrual syndrome, premenstrual dysphoric disorder, chemical dependencies and addictions, gastroesophageal reflux disease, fibromyalgia, post-herpetia neuralgia, cystitis and irritable bowel syndrome, in a mammal, and for pharmaceutical compositions comprising these compounds or their pharmaceutically acceptable salts and a pharmaceutically acceptable carrier.

Finally, there are provided three intermediate compounds of formula (I), *viz*. 5-methoxy-3,3-dimethyl-2-oxo-2,3-dihydro-1H-indole-6-carbaldehyde, 6-ethoxy-1-methyl-2-oxo-1,2,3,4-tetrahydroquinoline-7-carbaldehyde and 6-methoxy-1,3-dimethyl-2-oxo-1,2,3,4-tetrahydroquinoline-7-carbaldehyde.

The term "alkyl", as used herein, unless otherwise indicated, includes saturated monovalent hydrocarbon radicals having straight, branched or cyclic moieties or combinations thereof.

The term "halo-C₁-C₃ alkyl" as used herein to mean a C₁-C₃ alkyl radical substituted with one or more halogens (*e.g.* Cl, F, I, or Br) including, but not limited to, chloromethyl, trifluoromethyl, 2,2,2-trichloroethyl and the like.

The term "alkoxy", as used herein, means "alkyl-O-", wherein "alkyl" is defined as above.

Unless otherwise indicated, the term "one or more halogens", as used herein, refers to from one to the maximum number of halogen substituents possible based on the number of available bonding sites.

The term "treating", as used herein, refers to reversing, alleviating, inhibiting the progress of, or preventing the disorder or condition to which such term applies, or one or more symptoms of such condition or disorder. The term "treatment", as used herein, refers to the act of treating, as "treating" is defined immediately above.

The compounds of formula (I) may have optical centers and therefore may occur in different enantiomeric configurations. Formula (I), as depicted above, includes all enantiomers, diastereomers, and other stereoisomers of the compounds depicted in structural formula (I), as well as racemic and other mixtures thereof.

Formula (I) also includes all radiolabeled forms of the compounds depicted in structural formula (I). Preferred radiolabeled compounds of formula (I) are those wherein the radiolabels are selected from as ³H, ¹¹C, ¹⁴C, ¹⁸F, ¹²³I and ¹²⁵I. Such radiolabeled compounds are useful as research and diagnostic tools in metabolism pharmacokinetics studies and in binding assays in both animals and man.

The compounds of the formula (I) of this invention may be prepared as described in the following reaction schemes. Unless otherwise indicated, in the reaction schemes that follow, R¹, R², R³, X, Y, Z, W, Q and T are defined as above.

Scheme A-I illustrates a method for preparing compounds of the formula (I) by reductive amination of a compound of the formula (II) with a compound of the formula: T-NH₂, *i.e*. where Y is -NH-. The reduction can be carried out by catalytic hydrogenation, or with several hydride reagents in a reaction-inert solvent. The catalytic hydrogenation may be carried out In the presence of a metal catalyst such as palladium or Raney nickel. Suitable hydride reagents include borohydrides such as sodium borohydride (NaBH₄), sodium cyanoborohydride (NaBH₃CN) and sodium triacetoxyborohydride (NaB(OAc)₃H), boranes, aluminum-based reagents and trialkylsilanes. Suitable solvents include polar solvents such as methanol, ethanol, methylene chloride, tetrahydrofuran (THF), dioxane and ethyl acetate. This reaction is typically carried out at a temperature from -78°C to reflux temperature of the solvent, preferably from 0°C to 25°C for 5 minutes to 48 hours, preferably from 0.5 to 12 hours.

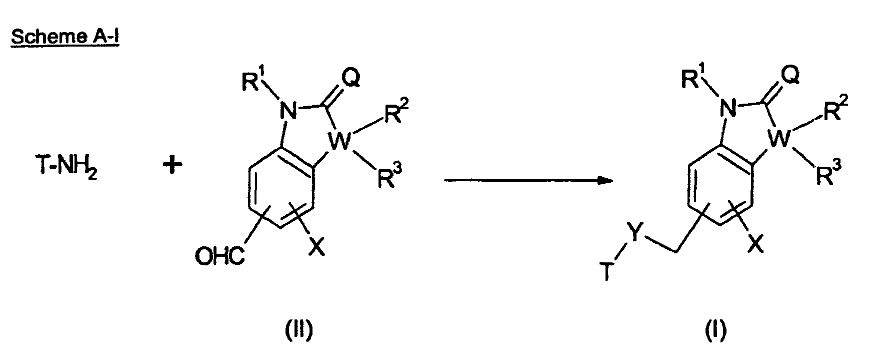

Alternatively, the compounds of the formula (I) of this invention may be prepared as shown in the following scheme A-II. 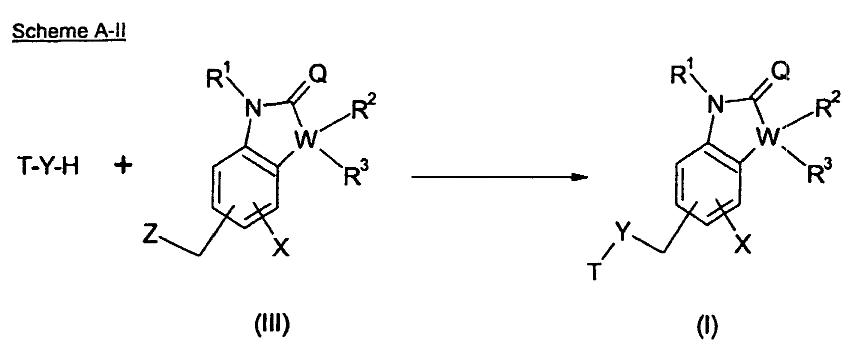 wherein Z is a leaving group such as halo or sulfonate including tosylate or mesylate.

Referring to Scheme A-II, the compounds of the formula (I) of this invention may be prepared by a reaction of a compound of the formula (III) with a compound of the formula: T-Y-H. When Y is NH, the compound (III) may be treated with T-NH₂ in the presence of a base (*e.g*. K₂CO₃ or Na₂CO₃) in a polar solvent (*e*.*g*. methanol, ethanol, isopropyl alcohol, THF, dioxane, dimethylformamide (DMF) or dimethylsulfoxide (DMSO)). When Y is O, the compound (III) may be treated with T-OH in the presence of a base (*e.g*. NaH or KH) in a polar solvent (*e.g*. THF, dioxane, DMF, or DMSO). This reaction is typically carried out at a temperature from -78°C to reflux temperature of the solvent, preferably from 0°C to 25°C for 5 minutes to 48 hours, preferably from 0.5 to 12 hours.

The compounds (III) may be prepared by reduction of an aldehyde of the formula (II), followed by conversion of a hydroxy group of the resultant compound into a leaving group, Z. Reduction of the aldehyde (II) may be accomplished using a variety of reducing agents in a reaction-inert solvent. Suitable reducing agent/solvent systems include sodium tetrahydroborate (NaBH₄) in methanol or ethanol; lithium tetrahydroborate (LiBH₄) in THF or diethyl ether; lithium aluminium hydride (LiAlH₄), lithium triethoxyaluminum hydride (LiAl(OEt)₃H) lithium *tert*-butoxyaluminium hydride (LiAl(O*t*-Bu)₃H) or aluminium trihydride (AlH₃) in THF or diethyl ether; and *iso*-butyl aluminium hydride(*i*-BuAlH₂) or diisopropyl aluminum hydride (DIBAL-H) in dichloromethane, THF, or *n*-hexane. This reaction is generally carried out at a temperature from -20°C to 25°C for 5 minutes to 12 hours. Then, the hydroxy group of the resultant compound is converted to a leaving group, Z (*e.g*. halo such as chloro, bromo, iodo or fluoro, or sulfonate including tosylate or mesylate). Conversion of the hydroxy group into the leaving group, Z may be accomplished according to methods known to those skilled in the art. For example, when Z is sulfonate such as tosylate or mesylate, the hydroxy compound is reacted with sulfonate in the presence of pyridine or triethylamine in dichloromethane. When Z is halo such as chloro or bromo, the hydroxy compound may be treated with SOX₂ (X is Cl or Br) in the presence of pyridine.

The compounds of the formula (II) can be prepared as illustrated in the following scheme B.

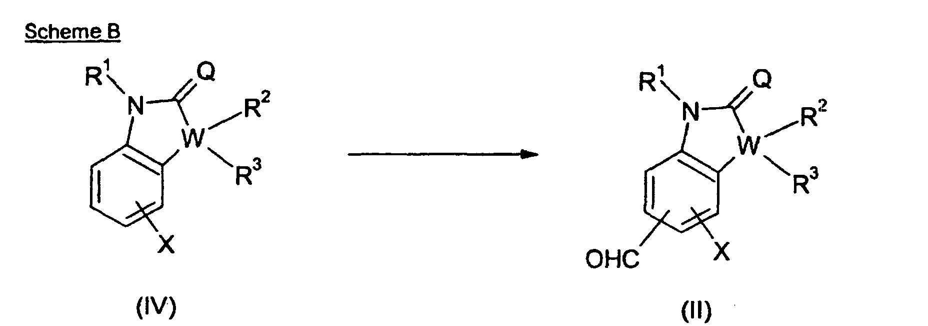

The compounds of the formula (II) may be prepared by direct or indirect formylation of a compound of the formula (IV). Any formylation methods known to those skilled in the art may be used, to introduce a formyl group into a benzene ring. For example, direct formylation may be accomplished by contacting the compound (IV) with a suitable formylating agent in the presence of a suitable catalyst. Suitable formylating agent/catalyst systems include dichloromethyl methyl ether/titanium (IV) chloride (Cl₂CHOCH₃/TiCl₄), trifluoroacetic acid (CF₃CO₂H)/hexamethylenetetramine (modified Duff's conditions) and phosphoryl trichloride (POCl₃)/DMF (Vilsmeier's conditions). Indirect formylation may be achieved by halogenating the compound (IV), displacing the halogen atom introduced with a cyano group, and then subjecting the resultant cyano-substituted compound to reduction. The halogenation as used herein may be carried out according to the procedure reported in G. A. Olah *et al*, J. Org. Chem., **58,** 3194 (1993). The displacement of the halogen atom with a cyano group may be performed according to the methods reported in D. M. Tschaem *et al*, Synth. Commun., **24,** 887 (1994), K. Takagi *et al*, Bull. Chem. Soc. Jpn., **64,** 1118 (1991). The reduction as used herein may be performed in the presence of diisopropyl aluminium hydride (DIBAL-H) in dichloromethane or Raney nickel in formic acid.

In addition, the compound (II) wherein W is vinylene can be prepared by dehydrogenation of the formula (II) wherein W is ethylene in a suitable solvent such as dioxane.

The starting materials of the formula (IV) are known compounds which are commercially available, or can be prepared by known methods. For example, compounds of the formula (IV) wherein R¹ is alkyl can be prepared by *N*-alkylation of the corresponding compounds (IV) wherein R¹ is hydrogen, in the presence of a base (*e.g.* NaH or KH) in a suitable solvent (*e.g*. DMSO, DMF and THF). Compounds of the formula (IV) wherein R² or R³ is not hydrogen, can be also prepared from the corresponding compounds (IV) wherein R² or R³ is hydrogen, using similar techniques as described above. Compounds (IV) can be also prepared by other methods as described in European Patent No. 385 662 and in C. Crestini *et al*, Synth. Commun., **24,** 2853 (1994) or G. W. Rewcastie *et al*, J. Med. Chem., **37,** 2033 (1994). Compound (IV) wherein Q is S, can be prepared by thionation of the corresponding compound (IV) wherein Q is O. Suitable thionation agents are Lawesson's reagent (Tetrahedron, **41,** 5061 (1985)) and P₄S₁₀ (Chem. Pharm. Bull., **10**, 647 (1962)).

Alternatively, compounds of the formula (I) wherein T is 2-phenylpireridinyl and Y is NH, may be prepared as shown in the following Scheme A-III.

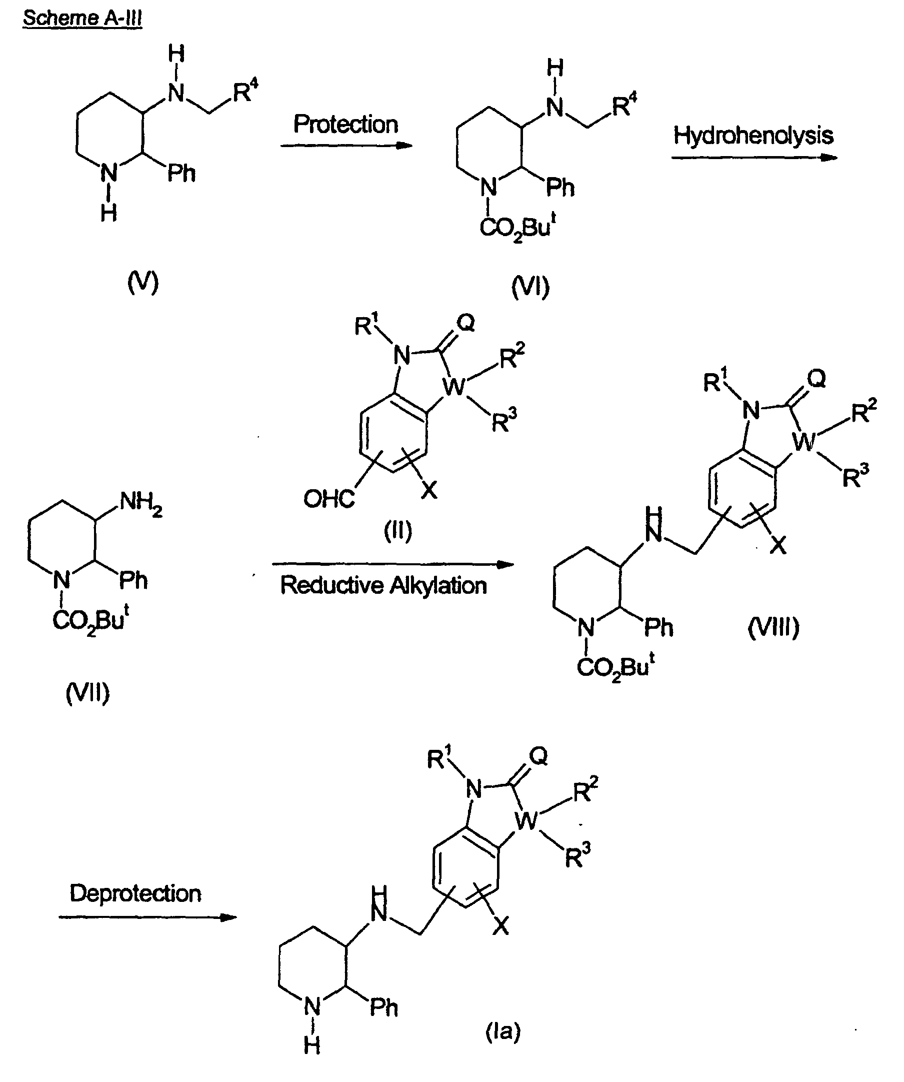

Scheme A-III illustrates the preparation of compounds of the formula (Ia) (corresponding to Compound (I) wherein T is 2-phenylpiperidinyl and Y is NH).

Referring to Scheme A-III, N-protection of a compound of the formula (V) (R⁴ is phenyl or the like) may be carried out by treatment with (*t*-BuOCO)₂O (Boc₂O) in the presence of a base such as sodium bicarbonate (NaHCO₃) or triethylamine (Et₃N) to obtain a compound of the formula (VI). Compound (VI) is subjected to hydrogenolysis to obtain a compound of the formula (VII) wherein R⁵ is phenyl and R⁶ is *t*-butoxycarbonyl. An alternative route for N-protection of a compound of the formula (V) may be carried out by treatment with carbobenzoxy chloride (Cbz-Cl) in the presence of a base such as sodium bicarbonate (NaHCO₃) or triethylamine (Et₃N), wherein R⁵ is phenyl; and R⁶ is benzyloxycarbonyl. The hydrogenolysis may be carried out by treatment with H₂ or ammonium formate (HCO₂NH₄) in the presence of a metal catalyst such as a palladium on charcoal (*e.g*. 20% palladium on charcoal) in a suitable solvent. Then, the compound (VII) is subjected to the reductive amination as described in Scheme A-I. The compound (VIII) may be converted into a compound of the formula (la) by treatment with acid catalyst such as hydrochloride (HCl) in methanol, concentrated HCl in ethyl acetate or CF₃CO₂H in dichloroethane.

Certain of the compounds of the invention may be prepared by biotransformation, as shown in certain of the Examples below. Biotransformations may be achieved by those skilled in the art by contacting the substance to be transformed, and other necessary reactants, with the enzymes derived from a variety of living organisms under conditions suitable for a chemical interaction to occur. Subsequently, the products of the reaction are separated and those of interest are purified for elucidation of their chemical structure and physical and biological properties. The enzymes can be present as purified reagents, be in crude extracts or lysates, or be in intact cells and can be in solution, be in suspension (*e.g*. intact cells), be covalently attached to a supporting surface, or be imbedded in a permeable matrix (*e.g*. agarose or alginate beads). The substrate and other necessary reactants (*e.g.* water, air) are supplied as the chemistry dictates. Generally, the reaction is carried out in the presence of one or more liquid phases, aqueous and/or organic, to promote mass transfer of the reactants and products. The reaction can be conducted aseptically or not. The conditions for monitoring the progress of the reaction and the isolation of the products of the reaction will vary according to the physical properties of the reaction system and the chemistry of the reactants and products.

The compounds of formula (I), and the intermediates shown in the above reaction schemes can be isolated and purified by conventional procedures, such as recrystallization or chromatographic separation.

As the compounds of formula (I) of this invention possess at least two asymmetric centers, they are capable of occurring in various stereoisomeric forms or configurations. Hence, the compounds can exist in separated (+)- and (-)-optically active forms, as well as mixtures thereof.. The present invention includes all such forms within its scope. Individual isomers can be obtained by known methods, such as optical resolution, optically selective reaction, or chromatographic separation in the preparation of the final product or its intermediate.
In so far as the compounds of formula (I) of this invention are basic compounds, they are all capable of forming a wide variety of different salts with various inorganic and organic acids. Although such salts must be pharmaceutically acceptable for administration to animals, it is often desirable in practice to initially isolate the base compound from the reaction mixture as a pharmaceutically unacceptable salt and then simply convert to the free base compound by treatment with an alkaline reagent and thereafter convert the free base to a pharmaceutically acceptable acid addition salt. The acid addition salts of the base compounds of this invention are readily prepared by treating the base compound with a substantially equivalent amount of the chosen mineral or organic acid in an aqueous solvent or in a suitable organic solvent, such as methanol or ethanol. Upon careful evaporation of the solvent, the desired solid salt is readily obtained. The acid which are used to prepare the pharmaceutically acceptable acid addition salts of the aforementioned base compounds of this invention are those which form non-toxic acid addition salts, *i.e*. salts containing pharmaceutically acceptable anions, such as the hydrochloride, hydrobromide, hydroiodide, nitrate, sulfate or bisulfate, phosphate or acid phosphate, acetate, lactate, citrate or acid citrate, tartrate or bi-tartrate, succinate, maleate, fumarate, gluconate, saccharate, benzoate, methanesulfonate, ethanesulfonate, benzenesulfonate, *p*-toluenesulfonate and pamoate (*i*.*e*. 1.1'-methylene-bis-(2-hydroxy-3-naphthoate)) salts.

The compounds of the invention which have also acidic groups are capable of forming base salts with various pharmaceutically acceptable cations. Examples of such salts include the alkali metal or alkaline-earth metal salts and particularly, the sodium and potassium salts. These salts are all prepared by conventional techniques.

The chemical bases which are used as reagents to prepare the pharmaceutically acceptable base salts of this invention are those which form non-toxic base salts with the herein described acidic derivatives. These particular non-toxic base salts include those derived form such pharmaceutically acceptable cations as sodium, potassium, calcium and magnesium, etc. These salts can easily be prepared by treating the aforementioned acidic compounds with an aqueous solution containing the desired pharmaceutically acceptable cation, and then evaporating the resulting solution to dryness, preferably under reduced pressure. Alternatively, they may also be prepared by mixing solutions of the acidic compounds and the desired alkali metal alkoxide together, and then evaporating the resulting solution to dryness in the same manner as before. In either case, stoichiometric quantities of reagents are preferably employed in order to ensure completeness of reaction and maximum production of yields of the desired final product.

The active compounds of the present invention exhibit significant substance P receptor-binding activity and therefore, are of value in the treatment of a wide variety of clinical conditions which are characterized by the presence of an excess of said substance P activity. Such conditions include gastrointestinal disorders, central nervous system disorders, inflammatory diseases, emesis, urinary incontinence, pain, migraine or angiogenesis in a mammalian subject, especially humans.

The active compounds of the formula (I) of this invention can be administered via either the oral, parenteral or topical routes to mammals. In general, these compounds are most desirably administered to humans in doses ranging from about 0.3 mg up to 750 mg per day, although variations will necessarily occur depending upon the weight and condition of the subject being treated and the particular route of administration chosen. However, a dosage level that is in the range of from about 0.06 mg to about 2 mg per kg of body weight per day is most desirably employed. Nevertheless, variations may still occur depending upon the species of animal being treated and its individual response to said medicament, as well as on the type of pharmaceutical formulation chosen and the time period and interval at which such administration is carried out. In some instances, dosage levels below the lower limit of the aforesaid range may be more than adequate, while in other cases still larger doses may be employed without causing any harmful side effects provided that such higher dose levels are first divided into several small doses for administration throughout the day.

The compounds of the present invention may be administered alone or in combination with pharmaceutically acceptable carriers or diluents by either of the above routes previously indicated, and such administration can be carried out in single or multiple doses. More particularly, the novel therapeutic agents of the invention can be administered in a wide variety of different dosage forms, *i.e*. they may be combined with various pharmaceutically acceptable inert carriers in the form of tablets, capsules, lozenges, troches, hard candies, powders, sprays, creams, salves, suppositories, jellies, gels, pastes, lotions, ointments, aqueous suspensions, injectable solutions, elixirs, syrups, and the like. Such carriers include solid diluents or fillers, sterile aqueous media and various nontoxic organic solvents, *etc*. Moreover, oral pharmaceutical compositions can be suitably sweetened and/or flavored. In general, the therapeutically-effective compounds of this invention are present in such dosage forms at concentration levels ranging about 5.0% to about 70% by weight.

For oral administration, tablets containing various excipients such as microcrystalline cellulose, sodium citrate, calcium carbonate, dicalcium phosphate and glycine may be employed along with various disintegrants such as starch and preferably corn, potato or tapioca starch, alginic acid and certain complex silicates, together with granulation binders like polyvinylpyrrolidone, sucrose, gelatin and acacia. Additionally, lubricating agents such as magnesium stearate, sodium lauryl sulfate and talc are often very useful for tabletting purposes. Solid compositions of a similar type may also be employed as fillers in gelatin capsules; preferred materials in this connection also include lactose or milk sugar as well as high molecular weight polyethylene glycols. When aqueous suspensions and/or elixirs are desired for oral administration, the active ingredient may be combined with various sweetening or flavoring agents, coloring matter or dyes, and, if so desired, emulsifying and/or suspending agents as well, together with such diluents as water, ethanol, propylene glycol, glycerin and various like combinations thereof.

For parenteral administration, solutions of a compound of the present invention in either sesame or peanut oil or in aqueous propylene glycol may be employed. The aqueous solutions should be suitably buffered (preferably pH>8) if necessary and the liquid diluent first rendered isotonic. These aqueous solutions are suitable for intravenous injection purposes. The oily solutions are suitable for intra-articular, intra-muscular and subcutaneous injection purposes. The preparation of all these solutions under sterile conditions is readily accomplished by standard pharmaceutical techniques well-known to those skilled in the art. Additionally, it is also possible to administer the compounds of the present invention topically when treating inflammatory conditions of the skin and this may preferably be done by way of creams, jellies, gels, pastes, ointments and the like, in accordance with standard pharmaceutical practice.

The activity of the compounds of the present invention, as substance P antagonists, is determined by their ability to inhibit the binding of substance P at its receptor sites in IM-9 cells employing radioactive ligands. The substance P antagonist activity of the herein described compounds is evaluated by using the standard assay procedure described by D. G. Payan *et al* as reported in J. Immunology, **133,** 3260 (1984). This method essentially involves determining the concentration of the individual compound required to reduce by 50% the amount of radiolabeled substance P ligands at their receptor sites in said isolated cow tissues or IM-9 cells, thereby affording characteristic IC₅₀ values for each compound tested. More specifically, inhibition of [³H]SP binding to human IM-9 cells by compounds are determined in assay buffer (50 mM Tris-HCl (pH 7.4), 1 mM MnCl₂, 0.02 % bovine serum albumin, bacitracin (40 µg/ml), leupeptin (4 µg/ml), chymostatin (2 µg/ml) and phosphoramidon (30 µg/ml)). The reaction is initiated by the addition of cells to assay buffer containing 0.56 nM [³H]SP and various concentrations of compounds (total volume; 0.5 ml) and allowed to incubate for 120 min at 4 °C. Incubation is terminated by filtration onto GF/B filters (presoaked in 0.1 % polyethylenamine for 2 hours). Nonspecific binding is defined as the radioactivity remaining in the presence of 1 µM SP. The filters are placed into tubes and counted using liquid scintillation counter.

The adverse effect on Ca²⁺ channel binding affinity is determined by verapamil binding study in rat heart membrane preparation. More specifically, verapamil binding study is performed as previously described by Reynolds *et al*, J. Pharmacol. Exp. Ther., **237,** 731 (1986). Briefly, incubations are initiated by the addition of tissue to tubes containing 0.25 nM [³H]desmethoxyverapamil and various concentrations of compounds (total volume 1 ml). Nonspecific binding is defined as radioligand binding remaining in the presence of 3-10 µM methoxyverapamil.

The activity of the compounds of this invention against generalized anxiety disorder is determined by inhibition of GR73632-induced tapping test in gerbils. More specifically, gerbils are lightly anesthetized with ether and the skull surface is exposed. GR73632 or vehicle (PBS, 5 µl) are administered directly into the lateral ventricles via a 25 gauge needle inserted 4.5 mm below bregma (preceded by pretreatment with an antagonist, 0.1-32.0 mg/kg, s.c. or p.o.). Following injection, gerbils are placed in 1 L beaker individually and monitored for repetitive hind paw tapping. Some compounds prepared in the following Examples were tested in accordance with these testing methods. As a result, it was found that the compounds of the present inventions have good antagonist activity toward substance P, particularly good activity against CNS disorders with decreased side effects.

The present invention is illustrated by the following examples. However, it should be understood that the invention is not limited to the specific details of these examples. Melting points were taken with a Büchi micro melting point apparatus and uncorrected. Infrared absorption spectra (IR) were measured by a Shimazu infrared spectrometer (IR-470). ¹H and ¹³C nuclear magnetic resonance spectra (NMR) were measured in CDCl₃ by a JEOL NMR spectrometer (JNM-GX270, 270MHz for ¹H, 67.5MHz for ¹³C) unless otherwise indicated and peak positions are expressed in parts per million (ppm) downfield from tetramethylsilane. The peak shapes are denoted as follows: s, singlet; d, doublet; t, triplet; m, multiplet; br, broad.

### Example 1

### Preparation of (2S,3S)-2-Diphenylmethyl-3-(6-methoxy-1,3,3-trimethyloxindol-5-yl)-methylamino-1-azabicyclo[2.2.2]octane monomesylate (Compound 6)

### (i). 6-Methoxyoxindole (Compound 1)

This compound was prepared according to Quallich and Morrissey's procedures (Synthesis 51(1993)).

### (ii). 6-Methoxy-1,3,3-trimethyloxindole (Compound 2)

To a stirred and ice-cooled suspension of NaH [60% in oil, 3.93g, 98.1mmol; washed with *n*-pentane (15.0ml) three times prior to use) in dry DMF (50.0ml) was added compound **1** (4.00g, 24.5mmol) portionwise. To this gray suspension was added neat Mel (6.11ml, *d*2.280, 98.1mmol) dropwise with ice-cooling. The mixture was stirred at room temperature for 45 minutes. After the mixture was ice-cooled, H₂O (90.0ml) was added. The mixture was extracted with ethyl acetate/toluene (ethyl acetate/toluene) (2:1; 70.0ml x3). The combined ethyl acetate/toluene (2:1) extracts were washed with Na₂S₂O₃ aq. (x1), H₂O (x1), and sat. NaCl aq. (x1), dried (MgSO₄), treated with activated charcoal, and concentrated *in vacuo* to give a red syrup (5.18g). This was purified by medium pressure silica gel chromatography [Merck Kieselgel 60, 100g; n-hexane/ethyl acetate (20:1-15:1-10:1)] to give compound **2** (4.74g, 94.2%) as white crystals. ¹H-NMR (270MHz) δ (CDCl₃) 7.09 (d, *J* = 8.1 Hz, 1H), 6.56 (dd, *J* = 8.1, 2.2Hz, 1H), 6.44 (d, *J* = 2.2Hz,. 1H), 3.83 (s, 3H), 3.19 (s, 3H), 1.34 (s, 6H) ppm.

### (iii). 5-Formyl-6-methoxy-1,3,3-trimethyloxindole (Compound 3)

The title compound **3** was prepared from compound **2** by applying the method reported by Rieche, A., Gross, H., and Höft, E. (Org. Synth. Coll. Vol., V, 49). To a stirred and ice-cooled solution of compound **2** (1.00g, 4.87mmol) in dry CH₂Cl₂ (30.0ml) was added neat TiCl₄ (1.60ml, *d*1.730, 14.6mmol) followed by dichloromethyl methyl ether (Cl₂CHOMe) (0.66ml, *d*1.271, 7.31mmol). After the addition was complete, the resultant dark green mixture was stirred at room temperature for 45 minutes. H₂O (60.0ml) was added with ice-cooling, and the layers were separated. The aqueous layer was extracted with CH₂Cl₂ (20.0ml x3). The combined CH₂Cl₂ layer and extracts were washed with sat. NaCl aq. (x1), sat. NaHCO₃ aq. (x1), and sat. NaCl aq., dried (MgSO₄), treated with activated charcoal, and concentrated *in vacuo* to give a white solid. This was recrystallized from *i*-PrOH/*i*-Pr₂O to give compound **3** (1.07g, 94.1%). mp 190.8-193.2°C; IR vₘₐₓ (nujol) 1718(s), 1709(s) cm⁻¹ ; ¹H-NMR (270MHz) δ (CDCl₃) 10.37 (s, 1H), 7.70 (s, 1H), 6.43 (s, 1H), 3.99 (s, 3H), 3.27 (s, 3H), 1.36 (s, 6H) ppm. Analysis: % Calculated: C₁₃H₁₅NO₃: C; 66.94, H; 6.48, N; 6.00. Found: C; 66.84, H; 6.47, N; 6.04.

### (iv). (2S,3S)-3-Amino-2-diphenylmethyl-1-azabicyclo[2,2,2]octane (Compound 4)

This compound was prepared according to the procedure (J. Med. Chem., *18*, 587(1975))

### (v). (2S,3S)-2-Diphenylmethyl-3-(6-methoxy-1,3,3-trimethyloxindol-5-yl)methylamino -1-azabicyclo[2,2,2]octane (Compound 5)

Reductive alkylation of compound **4** with compound **3** was carried out according to the procedures reported by Abdel-Magid, A. F., Maryanoff, C.A., and Carson, K.G. (Tetrahedron Lett., *31*, 5595 (1990)). To a stirred solution of compound **4** (1.28g, 4.36mmol), compound **3** (1.07g, 4.58mmol) and acetic acid (0.50ml, *d*1.049, 8.72mmol) in dry CH₂Cl₂ was added sodium triacetoxyborohydride (NaB(OAc)₃H) (1.39g, 6.54mmol) at room temperature. The mixture was stirred at room temperature for 3.5 hours. The mixture was made basic with 10% NaOH aq. (ca. 10.0ml), and the layers were separated. The aqueous layer was extracted with CH₂Cl₂ (8.0ml x3). The combined CH₂Cl₂ layer and extracts were washed with sat. NaCl aq. (x1), dried (K₂CO₃), and concentrated *in vacuo* to give a colorless glass. This was recrystallized from *i*-PrOH/*i*-Pr₂O to give compound **5** (1.89g, 85.0%) as a white powder. mp 184.7-189.1°C; IR vₘₐₓ (nujol) 1710(s), 1620(m), 1600(w), 1497(m), 1125(m), 1062(m), 830(w), 800(w), 756(w), 744(w), 706(w), 693(m) cm⁻¹; ¹H-NMR (270MHz) δ (CDCl₃) 7.41-7.30 (m, 2H), 7.32-7.21 (m, 2H), 7.24-7.12 (m, 5H), 6.54 (s, 1H), 6.28 (s, 1H), 4.49 (d, *J* = 12.1Hz, 1H), 3.70 (dd, *J* = 12.1, 8.1 Hz, 1H), 3.61 (s, 3H), 3.61 (d, *J* = 12.6Hz), 3.27-3.06 (m, 1 H), 3.19 (s, 3H), 3.14 (d, *J* = 12.6Hz, 1H), 2.94 (ddd, *J* = 3.9, 3.9, 3.9Hz, 1H), 2.77 (br, dd, *J* = 7.5, 7.5Hz, 2H), 2.60 (br, dd, *J* = 11.5, 115 Hz), 2.13-2.03 (m, 1H), 2.02-1.86 (m, 1H), 1.75-1.40 (m, 3H), 1.40-1.16 (m,1H), 1.32 (s, 3H), 1.31 (s, 3H) ppm. Analysis: % Calculated for C₃₃H₃₉N₃O₂: C; 77.77, H; 7.71, N; 8.24. Found: C; 77.62, H; 7.81, N; 8.15.

### (vi). (2S,3S)-2-Diphenylmethyl-3-(6-methoxy-1,3,3-trimethyloxindol-5-yl)methylamino -1-azabicyclo[2.2.2]octane monobesylate (Compound 6)

After compound **5** (0.200g, 0.392mmol) was dissolved in acetone with warming, to this solution was added a solution of PhSO₃H•H₂O (69.1mg, 0.392mmol) in acetone. When this mixture was allowed to cool to room temperature, precipitation of white solids took place. After the mixture was left to stand in a refrigerator at 4°C overnight, the crystals precipitated were collected by filtration, washed with ice-chilled acetone (x2), and dried *in vacuo* at room temperature to give compound **6** (0.198g, 75.7%) as a white powder. mp 242.7-247.7°C (decamp.); IR vₘₐₓ (nujol) 1713(s), 1699(s), 1620(s), 1600(m), 1500(s), 1220(s), 1175(s), 1124(s), 853(m), 820(m), 755(s), 725(s), 725(s), 707(s), 698(s) cm⁻¹; ¹H-NMR (270MHz) δ (CDCl₃) 7.76-7.63 (m, 2H), 7.43-7.20 (m, 9H), 7.27-7.12 (m, 3H), 7.13-7.01 (m, 1H), 6.52 (s, 1H), 6.30 (s, 1H), 4.54 (d, *J* = 12.1Hz, 1H), 4.60-4.45 (m, 1H), 3.70-3.40 (m, 4H), 3.60 (s, 3H), 3.53 (d, *J* = 12.3Hz, 1H), 3.32-3.20 (m, 1H), 3.20 (s, 3H), 3.16 (d, *J* = 12.3Hz, 1H), 2.46-2.35 (m, 1H), 2.35-2.18 (m, 1H), 2.13-1.95 (m, 2H), 2.05-1.65 (m, 2H), 1.65-1.45 (m, 1H), 1.31 (s, 6H) ppm. Analysis: % Calculated for C₃₉H₄₅N₃O₅S: C; 70.14, H; 6.79, N; 6.29. Found: C; 70.09, H; 6.88, N; 6.21.

### Example 2

### Preparation of (2S,3S)-3-(6-Methoxy-1,3,3-trimethyloxindol-5-yl)methylamino-2-phenylpiperidine dihydrochloride (Compound 13)

### (i) (2S,3S)-3-(2-Methoxybenzyl)amino-2-phenylpiperidine (Compound 7)

This compound was prepared according to the procedures disclosed in WO-93-01170.

### (ii). (2S,3S)-1-tert-Butoxycarbonyl-3-(2-methoxybenzyl)amino-2-phenylpiperidine (Compound 8)

To a stirred and ice-cooled mixture of compound **7** (10.0g, 27.1mmol), 3.0M NaOH aq. (36.1ml, 108.4mmol) and *tert*-butanol (15.0ml) was added (*tert*-BuOCO)₂O (Boc₂O, 7.39g, 33.8mmol) in one portion. After stirring at room temperature overnight, the mixture was extracted with ethyl acetate (50ml x3). The combined ethyl acetate extracts were washed with H₂O (x3), and sat. NaCl (x1), dried (Na₂SO₄), and concentrated *in vacuo* to give compound **8** (11.27g, quantitative) as a pale yellow syrup. IR vₘₐₓ (film) 3350(w), 1693(s), 1605(s), 1590(m), 1492(s), 755(m) cm⁻¹; ¹H-NMR (270MHz) δ (CDCl₃) 7.58 (br, d, *J* = 7.3Hz, 2H), 7.36-7.16 (m, 5H), 6.89 (ddd, *J* = 7.5, 7.5, 1.1Hz, 1H), 6.81 (dd, *J* = 8.4. 0.8Hz, 1H), 5.47 (br, s, 1H), 3.96 (dm, *J* = 13.4Hz, 1H), 3.87 (d, *J* = 13.6Hz, 1H), 3.79 (d, *J* = 13.6Hz, 1H), 3.70 (s, 3H), 3.10-2.99 (m, 1H), 2.94 (dd, *J* = 12.5, 3.4Hz, 1H), 1.87-1.74 (m, 2H), 1.74-1.40 (m, 3H), 1.41 (s, 9H) ppm. This was employed in the next step without further purification.

### (iii). (2S,3S)-3-Amino-1-tert-butoxycarbonyl-2-phenylpiperidine (Compound 9)

A mixture of compound **8** (11.27g), 20% Pd(OH)₂ / C (Pearlman's catalyst, 3.10g), and MeOH (90ml) was stirred under an atmosphere of H₂ (balloon) at room temperature overnight. After an additional amount of 20% Pd(OH)₂ / C (0.55g) was added, the stirring was continued under an atmosphere of H₂ (balloon) at room temperature for three days. The catalyst was filtered off by the aid of Celite, and washed with MeOH thoroughly. The combined MeOH filtrate and washings were concentrated *in vacuo* to give crude compound **9** (8.59g, quantitative). This was dissolved in ethanol (20.0ml), and then a warmed solution of fumaric acid (1.57g, 13.5mmol) in ethanol (20.0ml)was added in one portion to this solution at room temperature. When the mixture was scratched with a spatula, there took place precipitation of white solids with ease. After the mixture was left to stand at 4 °C in a refrigerator overnight, the crystals precipitated were collected by filtration, washed with ice-chilled ethanol (x1), and dried *in vacuo* at 50 °C to give a first crop of (2S,3S)-3-amino-1-(*tert*-butoxycarbonyl)-2-phenylpiperidine semifumarate compound **10** (6.14g, 67.8%) as white short needles. The combined filtrate and washing were concentrated *in vacuo* to give a residual solid (4.56g), which was recrystallized from ethanol and *i*-Pr₂O to give a second crop of compound **10** (1.25g, 13.7%). mp 165.7-168.8°C; Analysis: % Calculated for C₁₈H₂₆N₂O₄•0.4H₂O: C; 63.29, H; 7.91, N; 8.20. Found: C; 63.64, H; 8.22, N; 7.79. After a suspension of compound **10** (1.24g, 3.71mmol) in H₂O was ice-cooled, 20% NaOH aq. was added until the mixture became basic. The mixture was then extracted with ethyl acetate (x3). The combined ethyl acetate extracts were washed with sat. NaCl aq. (x1), dried (Na₂SO₄), and concentrated *in vacuo* to give pure compound **9** (0.95g. 93.1%). IR vₘₐₓ (film) 3370(w), 3310(w), 1695(s), 1682(s), 1807(m), 1590(w, shoulder), 1494(s), 1250(s), 1180(s), 1150(s), 756(m), 703(s) cm⁻¹; ¹H-NMR (270MHz) δ (CDCl₃) 7.47-7.39 (m, 2H), 7.37.7.23 (m, 5H), 5.19 (br, d, *J* = 6.2Hz, 1H), 4.00 (dm, *J* = 13.0Hz, 1H), 3.25-3.05 (m, 2H), 1.94-1.83 (m, 1H), 1.83-1.56 (m, 4H), 1.36 (s, 9H), 1.32 (br, s, 2H) ppm.

### (iv). (2S, 3S)-1-tert-Butoxycarbonyl-3-(6-methoxy-1,3,3-trimethyloxindol-5-yl) methylamino-2-phenylpiperidine (Compound 11)

Reductive alkylation of compound **9** with compound **3** was carried out according to the procedures reported by Abdel-Magid, A. F., Maryanoff, C.A., and Carson, K.G. (Tetrahedron Lett., *31*, 5595 (1990)). To a stirred and ice-cooled solution of compound **9** (0.504g, 1.82mmol) and compound **3** (0.468g, 2.01mmol) in dry CH₂Cl₂ (17.0ml) was added NaB(OAc)₃H (0.579g, 2.73mmol) in one portion. After the mixture was stirred at room temperature for eight hours, NaB(OAc)₃H (0.30g, 1.42mmol) and acetic acid (0.104ml, *d*1.049, 1.82mmol) were added, and the stirring was continued at room temperature for additional three nights. The mixture was made basic to pH 9-10 with 10% NaOH aq. (9.0ml), and the layers were separated. The aqueous layer was extracted with CH₂Cl₂ (x3). The combined CH₂Cl₂ layer and extracts were washed with NaCl aq. (x1), dried (K₂CO₃), and concentrated *in vacuo* to give a colorless syrup (1.06g). This was flash-chromatographed over silica gel (Merck Kieselgel 60, 30g). Elution with CH₂Cl₂-MeOH (200:1-150:1-100:1) gave compound **11** (0.644g, 71.9%) as a colorless syrup. IR vₘₐₓ (film) 3345(w), 1715(s), 1695(s), 1681(s), 1625(s), 1604(s), 1508(s), 886(m), 820(m), 732(s), 703(s) cm⁻¹; ¹H-NMR (270MHz) δ (CDCl₃) 7.60 (br, d, *J* = 7.0Hz, 2H), 7.38-7.23 (m, 3H), 7.00 (s, 1H), 6.36 (s, 1H), 5.57-5.45 (m, 1H), 4.03-3.89 (m, 1H), 3.84 (d, *J* = 12.8Hz, 1H), 3.78 (d, *J* = 12.8Hz, 1H), 3.74 (s,3H), 3.20 (s, 3H), 3.11-2.90 (m, 2H), 1.90-1.74 (m,2H), 1.74-1.45 (m, 3H), 1.41 (s, 9H), 1.32 (s, 3H), 1.31 (s, 3H) ppm.

### (v). (2S,3S)-3-(6-Methoxy-1,3,3-trimethyloxindol-5-yl)methylamino-2-phenylpiperidine (Compound 12)

To a stirred and ice-cooled solution of compound **11** (0.64g, 1.31mmol) in ethyl acetate (5.0ml) was added *conc*. HCl (2.0ml) dropwise. The mixture was stirred at room temperature for 45 minutes. The mixture was ice-cooled, and then made basic with 20% NaOH aq. (ca. 8.0ml). The layers were separated, and the aqueous layer was extracted with ethyl acetate (x3). The combined ethyl acetate layer and extracts were washed with sat. NaCl aq. (x1), dried (K₂CO₃), and concentrated *in vacuo* to give compound **12** (0.48g, 93.6%) as a colorless syrup. IR vₘₐₓ(film) 3330(m), 1710(s), 1620(s), 1600(s), 1500(s), 1250(s), 1125(s), 1060(m) cm⁻¹; ¹H-NMR (270MHz) δ (CDCl₃) 7.38-7.17 (m, 5H), 6.79 (s, 1H), 6.24 (s, 1H), 3.90 (d, *J* = 2.4Hz, 1H), 3.63 (d, *J* = 13.7Hz, 1H), 3.54 (s, 3H), 3.40 (d, *J* = 13.7Hz, 1H), 3.31-3.21 (m, 1H), 3.17 (s, 3H), 2.85 (ddd, *J* = 2.4, 2.4, 2.4Hz, 1H), 2.80 (ddd, *J* = 12.6, 12.6, 3.1Hz, 1H), 2.14 (dm, *J* = 12.6Hz, 1H), 1.93 (ddddd, *J* = 12.6, 12.6, 12.6, 4.0, 4.0Hz, 1H), 1.69 (br, s, 2H), 1.61 (dddd, *J* = 12.6, 12.6, 3.7, 3.7Hz, 1H), 1.42 (dm, *J* = 12.6Hz, 1H), 1.29 (s, 3H), 1.28 (s, 3H) ppm. This was employed in the next salt formation step without further purification.

### (vi). (2S,3S)-3-(6-Methoxy-1,3,3-trimethyloxindol-5-yl)methylamino-2-phenylpiperidine dihydrochloride (Compound 13)

To a solution of compound **12** (0.48g, 1.22mmol) in MeOH (0.5ml) was added an excess amount of Hydrogen Chloride, Methanol Reagent 10 (Tokyo Kasei, 6.0ml). After the solvent MeOH was evaporated *in vacuo*, the residual solid was recrystallized from MeOH-Et₂O. The recrystallization mixture was left to stand at 4°C in a refrigerator for three nights. The crystals precipitated were collected by filtration, washed with Et₂O (x2), and dried in vacuo at 50°C to give compound **13** (0.401g, 70.4%) as a white powder. mp 223.7 - 239.7 °C; IR vₘₐₓ(nujol) 2900-2200(br., s), 1718(s), 1710(s), 1630(s), 1605(m), 1565(s), 1505(s), 1250(s), 1180(s), 1129(s), 1060(m), 900(m), 850(m), 824(m), 765(m), 750(s), 692(s) cm⁻¹. Analysis: % Calculated for C₂₄H₃₃N₃O₂Cl₂: C; 61.80, H; 7.13, N; 9.01. Found: C; 61.67, H; 7.13, N; 9.00.

### Example 3

### Preparation of (2S,3S)-2-Diphenylmethyl-3-(6-methoxy-1-methyl-2-oxo-1,2,3,4-tetrahydroquinolin-7-yl)methylamino-1-azabicyclo[2.2.2]octane (Compound 17)

### (i). 6-Methoxy-1-methyl-2-oxo-1,2-dihydroquinoline (Compound 14)

Preparation of compound **14** from 6-methoxyquinoline was carried out according to the procedures set forth in EP 385662. A mixture of 6-methoxyquinoline (16 g, 100 mmol) and dimethyl sulfate (13 g, 100 mmol) and in benzene (50 ml) was refluxed for 1 hour. After the reaction mixture was cooled down to room temperature, the orange solution was decanted off. The resulting salt was washed with benzene (30 ml x 3). This salt was dissolved in H₂O (50 ml) and the solution was washed with benzene (30 ml). This solution and a solution of NaOH (12 g, 300 mmol) in H₂O (50 mL) were added to a mixture of potassium ferricyanide (66 g, 200 mmol) in H₂O and CH₂Cl₂ at room temperature over 15 minutes. The resulting mixture was stirred at room temperature for 15hr. The organic layer was separated and the aqueous layer was extracted with CH₂Cl₂ (150 ml) three times. The combined extracts were dried over Na₂SO₄ and concentrated. The crude was purified by recrystallization from ethyl acetate/hexane to give compound **14** (12 g, 63 mmol, 63%) as a pale yellow crystal. ¹H-NMR (270MHz) δ (CDCl₃) 7.70-6.70 (m, 5H), 3.88 (s, 3H), 3.72 (s, 3H) ppm.

### (ii). 6-Methoxy-1-methyl-2-oxo-1,2,3,4-tetrahydroquinoline (Compound 15)

A mixture of compound **14** (3.0 g, 16 mmol), 10 % Pd-C (0.7 g) and ethanol (20 ml) was heated in an autoclave at 100°C under 50 atmospheres of H₂ for 15 hours. The catalyst was removed by filtration and the filtrate was concentrated *in vacuo* to give a crude solid. This was purified by recrystallization from MeOH to give compound **15** (1.8 g, 9.4 mmol, 53%) as a colorless crystal. ¹H-NMR (270 MHz) δ (CDCl₃) 6.96-6.68 (m, 3H), 3.79 (s, 3H), 3.33 (s, 3H), 2.95-2.56 (m, 4H) ppm.

### (iii). 6-Methoxy-1-methyl-2-oxo-1,2,3,4-tetrahydroquinoline-7-carboxaldehyde (Compound 16)

This compound was prepared from compound **15** in a procedure analogous to that used to make compound **3.** ¹H-NMR (270 MHz) δ (CDCl₃) 10.44 (s, 1H), 7.42 (s, 1H), 6.84 (s, 1H), 3.94 (s, 3H), 3.37 (s, 3H), 2.97 (t, 2H, *J* = 7Hz), 2.66 (t, 2H, *J* = 7Hz)

### (iv). (2S,3S)-2-Diphenylmethyl-3-(6-methoxy-1-methyl-2-oxo-1,2,3,4-tetrahydroquinolin-7-yl)methylamino-1-azabicyclo[2,2,2]octane (Compound 17)

This compound was prepared from compound **16** and compound **4** in a procedure analogous to that used to make compound **5.** mp 136 - 139°C. ¹H-NMR (270 MHz) δ (CDCl₃) 7.38-6.43 (m, 12H), 4.47 (d, *J* = 12 Hz, 1H), 3.75-2.55 (m, 13H), 3.54 (s, 3H), 3.27 (s, 3H), 2.16-1.20 (m, 4H) ppm. Analysis: % Calculated for C₃₂H₃₇N₃O₂•0.25H₂O: C; 76.84 %, H; 7.56 %, N; 8.40 %. Found: C; 76.81 %, H; 7.45 %, N, 8.41 %.

### Example 4

### Preparation of (2S,3S)-2-Diphenylmethyl-3-(6-methoxy-1-methyl-2-oxo-1,2-dihydroquinolin-7-yl)methylamino-1-azabicyclo[2.2.2]octane (Compound 19)

### (i). 6-Methoxy-1-methyl-2-oxo-1,2-dihydroquinoline-7-carboxaldehyde (Compound 18)

A mixture of compound **16** (2.0 g, 9.0 mmol), DDQ (9.0 g, 40 mmol) and dioxane (100 ml) was stirred and heated at 140°C for two days. After the reaction mixture was cooled down to room temperature, the mixture was filtered and the filtrate was concentrated *in vacuo*. The residue was purified by a column chromatography on silica gel to give a pale yellow solid, which was recrystallized to give compound **18** (0.7 g, 31 mmol, 35%) as a pale yellow crystal. ¹H-NMR (270 MHz) δ (CDCl₃) 10.57 (s, 1H), 7.84 (s, 1H), 7.64 (d, *J* = 10 Hz, 1H), 7.10 (s, 1H), 6.84 (d, *J* = 10 Hz, 1H), 4.00 (s, 3H), 3.75 (s, 3H) ppm.

### (ii). (2S,3S)-2-Diphenylmethyl-3-(6-methoxy-1-methyl-2-oxo-1,2-dihydroquinolin-7-yl)methylamino-1-azabicyclo[2.2.2]octane (Compound 19)

This compound was prepared from compound **18** and compound **4** in a procedure analogous to that used to make compound **5**. mp 125 - 128 °C. ¹H-NMR (270 MHz) δ (CDCl₃) 7.61-6.63 (m, 14H), 4.47 (d, *J* = 12 Hz, 1H), 3.72-2.55 (m, 9H), 3.66 (s, 3H), 3.62 (s, 3H), 2.14-1.20 (m, 4H) ppm. Analysis: % Calculated for C₃₂H₃₅N₃O₂•0.5H₂O•0.4(2-propanol): C; 75.71 %, H; 7.50 %, N; 7.98 %. Found: C; 75.41 %, H; 7.50 %, N; 7.91 %.

### Example 5

### Preparation of (2S,3S)-3-(6-methoxy-1-methyl-2-oxo-1,2,3,4-tetrahydroquinolin-7-yl)methylamino-2-phenylpiperidine dihydrochloride (Compound 21)

### (i). (2S,3S)-2-Phenylpiperidin-3-amine dihydrochloride (Compound 20)

This compound was prepared according to the procedures disclosed in EP-558156.

### (ii). (2S,3S)-3-(6-Methoxy-1-methyl-2-oxo-1,2,3,4-tetrahydroquinolin-7-yl)methylamino-2-phenylpiperidine dihydrochloride (Compound 21)

A mixture of compound **20** (250 mg, 1 mmol), compound **16** (220 mg, 1 mmol) and sodium triacetoxyborohydride (400 mg, 1.9 mmol) in CH₂Cl₂ (10 ml) was stirred at room temperature for 24 hours, quenched with NaHCO₃ aq , and extracted with CH₂Cl₂ three times. The combined extracts were dried over Na₂SO₄ and concentrated. The crude was purified by a column chromatography on silica gel to give a free base of compound **21.** This was derived to dihydrochloride salt with HCl-MeOH, which was washed with IPA to give compound **21** (100 mg, 0.22 mmol, 22%) as a colorless crystal. mp 265 - 268°C. IR vₘₐₓ(KBr) 3415, 2935, 1673, 1647, 1556, 1513, 1469, 1452, 1430, 1366, 1249, 1185, 1163, 1065, 1027 cm⁻¹. ¹H-NMR (270 MHz) δ (free base; CDCl₃) 7.43-6.52 (m, 7H), 4.00-3.28 (m, 4H), 3.51 (s, 3H), 3.22 (s, 3H), 2.96-1.45 (m, 10H) ppm. Analysis: % Calculated for C₂₃H₂₉N₃O₂•2HCl•0.5H₂O: C; 59.87 %, H, 6.99 %, N, 9.11 %. Found: C; 59.82%, H; 7.37 %, N, 9.23 %. The compound obtained was subjected to the IM-9 binding assay, the [Sar⁹, Met(O₂)¹¹]substance P-induced tapping test and the verapamil binding study as described before, with the results of less than 0.1 nM, 52% (% inhibition at 0.3mg/kg scoring) and more than 3000nM, respectively.

### Example 6

### Preparation of (2S,3S)-3-(6-Isopropoxy-1-methyl-2-oxo-1,2,3,4-tetrahydroquinolin-7-yl)methylamino-2-phenylpiperidine dihydrochloride (Compound 26)

### (i). 6-Hydroxy-1-methyl-2-oxo-1,2,3,4-tetrahydroquinoline (Compound 22)

To a stirred solution of compound **15** (500 mg, 2.61mmol) in CH₂Cl₂ (7 ml) was added BBr₃ (1.0 M in CH₂Cl₂, 5.74 ml, 5.74 mmol) at room temperature, and stirred for 3h. The mixture was poured into ice water. The aqueous layer was extracted with ethyl acetate (x 2). The combined organic layers were washed with sat. NaCl aq, dried (MgSO₄), filtered, and concentrated to give compound **22** (350 mg, 76%) as a colorless crystal. ¹H-NMR (270MHz) δ (CDCl₃) 6.85 (1H, d, *J* = 8.4 Hz), 6.75-6.70 (m, 2H), 3.34 (3H, s), 2.86 (2H, t, *J* = 7.1 Hz), 2.63 (2H, t, *J* = 7.1 Hz) ppm.

### (ii). 6-Isopropoxy-1-methyl-2-oxo-1,2,3,4-tetrahydroquinoline (Compound 23)

To a stirred solution of compound **22** (350 mg, 1.98 mmol) and 2-iodopropane (0.590 ml, 5.93 mmol) in acetone (16 ml) was added Cs₂CO₃ (2.90 g, 8.91 mmol), and heated at 55°C for 3h. The mixture was filtered over Celite and washed with acetone. The filtrate was concentrated to give crude compound **23.** This was diluted with ethyl acetate, washed with water and sat. NaCl aq, dried (MgSO₄), and concentrated. This was purified by SiO₂ chromatography to give compound **23** (382 mg, 88%) as a colorless oil. ¹H-NMR (270MHz) δ (CDCl₃) 6.88 (1 H, d, *J* = 8.4 Hz), 6.80-6.70 (m, 2H), 4.49 (1H, hep, *J* = 5.9 Hz), 3.33 (3H, s), 2.86 (2H, t, *J* = 7.2 Hz), 2.66-2.59 (2H, m), 1.33 (6H, d, *J* = 5.9 Hz) ppm.

### (iii). 6-Isopropoxy-1-methyl-2-oxo-1,2,3,4-tetrahydroquinoline-7-carboxaldehyde (Compound 24)

To a stirred solution of compound **23** (382 mg, 1.74 mmol) in CH₂Cl₂ (10 ml) was added TiCl₄ (0.42 ml, 3.83 mmol) at -20°C. After the reaction mixture was stirred for 10 minutes, Cl₂CHOMe (0.35 ml, 3.83 mmol) was added at -20°C; and stirred for 2 hours. After H₂O was added, the mixture was extracted with CH₂Cl₂ (x 3). The combined extracts were dried (MgSO₄), filtered, and concentrated. The residue was purified by SiO₂ chromatography to give compound **24** (391 mg, 91%) as a slight yellow crystal. ¹H-NMR (270 MHz) δ (CDCl₃) 10.44 (1H, s), 7.41 (1H, s), 6.84 (1H, s), 4.65 (1H, hep, *J* = 5.9 Hz), 3.36 (3H, s), 2.94 (2H, t, *J* = 7.3 Hz), 2.66 (2H, t, *J* = 7.3 Hz), 1.40 (6H, d, *J* = 5.9 Hz) ppm.

### (iv). (2S,3S)-1-tert-Butoxycarbonyl-3-(6-isopropoxy-1-methyl-2-oxo-1,2,3,4-tetrahydroquinolin-7-yl)methylamino-2-phenylpiperidine (Compound 25)

A mixture of compound **9** (436 mg, 1.58 mmol), compound **24** (390 mg, 1.58 mmol), sodium triacetoxyborohydride (670 mg, 3.16 mmol) and CH₂Cl₂ (8 ml) was stirred under nitrogen at room temperature for 2 hours. After NaHCO₃ aq. solution was added, the mixture was extracted with CH₂Cl₂ (x 3). The combined extracts were dried (MgSO₄), filtered and concentrated. This was purified by SiO₂ chromatography to give compound **25** (698 mg, 87%) as a colorless oil. ¹H-NMR (270 MHz) δ (CDCl₃) 7.58 (2H, d, *J* = 7.0 Hz), 7.37-7.22 (3H, m), 6.88 (1H, s), 6.64 (1H, s), 5.54-5.42 (1H, m), 4.45 (1H, hep, *J* = 5.9 Hz), 4.02-3.78 (3H, m), 3.30 (3H,s), 3.12-2.92 (2H, m), 2.83 (2H, t, *J* = 7.3 Hz), 2.61 (2H, t, *J* = 7.3 Hz), 1.95-1.30 (4H, m), 1.40 (9H, s), 1.26 (3H, d, *J* = 5.9 Hz), 1.24 (3H, d, *J* = 5.9 Hz) ppm.

### (v). (2S,3S)-3-(6-Isopropoxy-1-methyl-2-oxo-1,2,3,4-tetrahydro-quinolin-7-yl)methyl amino-2-phenylpiperidine dihydrochloride (Compound 26)

To a solution of compound **25** (312 mg, 0.615 mmol) in ethyl acetate (6 ml) was added an excess amount of Hydrogen Chloride, Methanol Reagent 10 (Tokyo Kasei, 3 ml). The mixture was stirred for 4 hours and then evaporated *in vacuo*, the residual solid was recrystallized from MeOH-Et₂O to give compound **26** (140 mg, 47%) as a white crystal. mp 249-251°C; ¹H-NMR (270 MHz) δ (free base; CDCl₃) 7.40-7.18 (5H, m), 6.63 (1H, s), 6.55 (1H, s), 4.31 (1H, hep, *J* = 6.2 Hz), 3.89 (1H, d, *J* = 2.2 Hz), 3.54 (1H, d, *J* = 13.7 Hz), 3.41 (1H, d, *J* = 13.7 Hz), 3.42-3.20 (1 H, m), 3.20 (3H,s), 2.95-2.75 (4H, m), 2.64-2.55 (2H, m) 2.22-2.10 (1H, m), 1.98-1.82 (1H, m), 1.72-1.56 (1H, m), 1.52-1.39 (1H, m), 1.15 (3H, d, *J* = 5.9 Hz), 1.12 (3H, d, *J* = 5.9 Hz) ppm. IR vₘₐₓ (KBr) 3420, 2915, 2650, 2460, 1666, 1513, 1468, 1436, 1404, 1372, 1130, 964 cm⁻¹. Analysis: % Calculated for C₂₅H₃₅N₃O₂Cl₂: C; 62.50%, H; 7.34%; N 8.75%; Found: C; 62.12%, H; 7.58%, N; 9.04%.

### Example 7

### Preparation of (2S,3S)-3-(6-Methoxy-1-methyl-2-oxo-1,2-dihydroquinolin-7-yl)-methylamino-2-phenylpiperidine dihydrochloride (Compound 28)

### (i). (2S,3S)-1-tert-Butoxycarbonyl-3-(6-methoxy-1-methyl-2-oxo-1,2-dihydroquinolin-7-yl)methylamino-2-phenylpiperidine (Compound 27)

This compound was prepared from compound **18** and compound **9** in a procedure analogous to that used to make compound **11.** ¹H-NMR (270 MHz) δ (CDCl₃) 7.70 - 6.66 (9H, m), 5.52 (1H, br), 4.10 - 3.62 (3H, m), 3.77 (3H,s), 3.68 (3H,s), 3.30 - 2.92 (2H, m), 2.10 - 1.30 (4H, m), 1.40 (9H, s) ppm. This was employed in the next step without further purification.

### (ii).(2S,3S)-3-(6-Methoxy-1-methyl-2-oxo-1,2-dihydroquinolin-7-yl)methylamino-2-phenylpiperidine dihydrochloride (Compound 28)

This compound was prepared from compound **27** in a procedure analogous to that used to make compound **26.** mp 260 - 263°C. ¹H-NMR (270 MHz) δ (free base; CDCl₃) 7.60 - 6.62 (9H, m), 3.95 - 2.75 (6H, m), 3.61 (3H,s), 3.54 (3H,s), 2.23 - 1.42 (4H, m) ppm. Analysis: % Calculated for C₂₃H₂₇N₃O₂•2HCl•H₂O: C; 58.98 %, H, 6.67 %, N, 8.97 %. Found: C; 58.71%, H; 6.97 %, N, 8.72 %.

### Example 8

### Preparation of (2S,3S)-3-(1-Isopropyl-6-methoxy-2-oxo-1,2,3,4-tetrahydroquinolin-7-yl)methylamino-2-phenylpiperidine dihydrochloride (Compound 33)

### (i). 6-Methoxy-2-oxo-1,2,3,4-tetrahydroquinoline (Compound 29)

This compound was prepared according to the procedure (J. Med. Chem, *30*, 295(1987)).

### (ii). 1-Isopropyl-6-methoxy-2-oxo-1,2,3,4-tetrahydroquinoline (Compound 30)

To a stirred solution of compound **29** (560 mg, 3.0 mmol) and 2-iodopropane (1.0 g, 6.0 mmol) in DMF (5 ml) was added NaH (240 mg, 6.0 mmol), and heated at 60°C for 3 h. The mixture was diluted with water, extracted with CH₂Cl₂ (50 ml) three times. The combined extracts were dried over Na₂SO₄ and concentrated. The crude product was purified by a column chromatography on silica gel to give compound **30** (290 mg, 1.3 mmol, 44%) as a pale yellow crystal. ¹H-NMR (270 MHz) δ (CDCl₃) 7.10-6.70 (m, 3H), 4.68 (hep, 1H, *J* = 7Hz), 3.79 (s, 3H), 2.84-2.50 (m, 4H), 1.50 (d, 6H, *J* = 7Hz) ppm.

### (iii). 1-Isopropyl-6-methoxy-2-oxo-1,2,3,4-tetrahydroquinoline-7-carboxaldehyde (Compound 31)

This compound was prepared from compound **30** in a procedure analogous to that used to make compound **3.** ¹H-NMR (270 MHz) δ (CDCl₃) 10.43 (s, 1H), 7.57 (s, 1H), 6.82 (s, 1H), 4.67 (hep, 1H, *J* = 7Hz), 3.93 (s, 3H), 2.87 (t, 2H, *J* = 7Hz), 2.57 (t, 2H, *J* = 7Hz), 1.51 (d, 6H, *J* = 7Hz) ppm.

### (iv). (2S,3S)-1-tert-Butoxycarbonyl-3-(1-isopropyl-6-methoxy-2-oxo-1,2,3,4-tetrahydroquinolin-7-yl)methylamino-2-phenylpiperidine (Compound 32)

This compound was prepared from compound **31** and compound **9** in a procedure analogous to that used to make compound **11.** ¹H-NMR (270 MHz) δ (CDCl₃) 7.65 - 6.60 (7H, m), 5.48 (1H, br), 4.62 (1H, hep, *J* = 7 Hz), 4.03 - 3.75 (3H, m), 3.70 (3H, s), 3.14 - 2.48 (6H, m), 1.95 - 1.40 (4H, m), 1.48 (3H, d, *J* = 7 Hz), 1.47 (3H, d, *J* = 7 Hz), 1.40 (9H, s) ppm. This was employed in the next step without further purification.

### (v). (2S,3S)-3-(1-Isopropyl-6-methoxy-2-oxo-1,2,3,4-tetrahydroquinolin-7-yl)methylamino-2-phenylpiperidine dihydrochloride (Compound 33)

This compound was prepared from compound **32** in a procedure analogous to that used to make compound **26.** mp 257 - 260°C. IR vₘₐₓ (KBr) 3440, 2960, 2925, 1665, 1554, 1506, 1464, 1455, 1435, 1412, 1372, 1355, 1326, 1316, 1234, 1196, 1141, 1033 cm⁻¹. ¹H-NMR (270 MHz) δ (free base; CDCl₃) 7.40 - 6.50 (7H, m), 4.55 (1H, hep, *J* = 7 Hz), 3.94 - 3.22 (4H, m), 3.49 (3H,s), 2.90 - 2.47 (6H, m), 2.20 - 1.38 (4H, m), 1.46 (3H, d, *J* = 7 Hz), 1.46 (3H, d, *J* = 7 Hz) ppm. Analysis: % Calculated for C₂₅H₃₃N₃O₂•2HCl•H₂O: C; 60.24 %, H, 7.48 %, N, 8.43 %. Found: C; 60.46%, H; 7.77 %, N, 8.13 %.

### Example 9

### Preparation of (2S,3S)-3-[(6-Difluoromethoxy-1-methyl-2-oxo-1,2,3,4-tetrahydroquinolin-7-yl)methyl]amino-2-phenylpiperidine dihydrochloride (Compound 37)

### (i). 6-Hydroxy-1-methyl-2-oxo-1,2,3,4-tetrahydroquinoline-7-carboxaldehyde (Compound 34)

This compound was prepared from compound **16** in a procedure analogous to that used to make compound **22.** ¹H-NMR (270MHz) δ (CDCl₃) 10.89 (1H, s), 9.88 (1H, s), 7.07 (1H, s), 6.85 (1H, s), 3.39 (3H, s), 2.98-2.88 (2H, m), 2.68-2.58 (2H, m) ppm.

### (ii). 6-Difluoromethoxy-1-methyl-2-oxo-1,2,3,4-tetrahydroquinoline-7-carboxaldehyde (Compound 35)

To a stirred solution of compound **34** (160 mg, 0.78 mmol) and NaOH (200 mg, 5.0 mmol) in dioxane - H₂O (10 ml) was bubbled ClCHF₂ at 100°C for 18 hours. The solvent was removed by evaporation, and the residue was diluted with CH₂Cl₂. This was washed with water and brine, dried over MgSO₄, filtered, and concentrated. The crude product was purified by a column chromatography on silica gel to give compound **35** (20 mg, 0.078 mmol, 10 %) as a white solid. ¹H-NMR (270MHz) δ (CDCl₃) 10.34 (1H, s), 7.49 (1H, s), 7.11 (1H, s), 6.64 (1H, t, *J* = 72.5 Hz), 3.40 (3H, s), 3.05 - 2.95 (2H, m), 2.73 -2.63 (2H, m) ppm.

### (iii). (2S,3S)-1-tert-Butoxycarbonyl-3-[(6-difluoromethoxy-1-methyl-2-oxo-1,2,3,4-tetrahydroquinolin-7-yl)methyl]amino-2-phenylpiperidine (Compound 36)

This compound was prepared from compound **35** and compound **9** in a procedure analogous to that used to make compound **11.** ¹H-NMR (270 MHz) δ (CDCl₃) 7.62 - 7.53 (2H, m), 7.38 - 7.23 (3H, m), 6.95 - 6.91 (2H, m), 6.42 (1H, t, *J* = 74.4 Hz), 5.52 - 5.43 (1H, m), 4.02 - 3.88 (3H, m), 3.29 (3H, s), 3.12 - 2.94 (2H, m), 2.90 - 2.82 (2H, m), 2.67 - 2.58 (2H, m), 2.00 - 1.40 (4H, m), 1.40 (9H, s) ppm.

### (iv). (2S,3S)-3-[(6-Difluoromethoxy-1-methyl-2-oxo-1,2,3,4-tetrahydroquinolin-7-yl)methyl]amino-2-phenylpiperidine dihydrochloride (Compound 37)

This compound was prepared from compound **36** in a procedure analogous to that used to make compound **26.** mp 238 - 240°C. IR vₘₐₓ (KBr) 3440, 2930, 2763, 2475, 1681, 1521, 1431, 1116, 1044 cm⁻¹. ¹H-NMR (270 MHz) δ (free base; CDCl₃) 7.42 - 7.22 (5H, m), 6.83 (1H, s), 6.75 (1H, s), 6.25 (1H, t, *J* = 74.3 Hz), 4.03 - 3.98 (1H, m), 3.62 (1H, d, *J* = 13.9 Hz), 3.42 - 3.33, (1H, m), 3.38 (1H, d, *J* = 13.9 Hz), 3.19 (3H, s), 2.98 - 2.76 (4H, m), 2.65 - 2.55 (2H, m), 2.20 - 1.50 (4H, m) ppm. Analysis: % Calculated for C₂₃H₂₇F₂N₃O₂•2HCl: C; 56.56 %, H, 5.98 %, N, 8.60 %. Found: C; 56.28 %, H; 6.05 %, N, 8.39 %.

### Example 10

### Preparation of (2S,3S)-3-[[6-Methoxy-1-(2,2,2-trifluoroethyl)-2-oxo-1,2,3,4-tetrahydroquinolin-7-yl]methyl]amino-2-phenylpiperidine dihydrochloride (Compound 41)

### (i). 6-Methoxy-1-(2,2,2-trifluoroethyl)-2-oxo-1,2,3,4-tetrahydroquinoline (Compound38)

This compound was prepared from compound **29** and CH₃SO₃CH₂CF₃ in a procedure analogous to that used to make compound **30.** ¹H-NMR (270MHz) δ (CDCl₃) 6.98 (1H, d, *J* = 8.4 Hz), 6.80 - 6.72 (2H, m), 4.61 (2H, q, *J* = 8.4 Hz), 3.80 (3H, s), 2.95 - 2.87 (2H, m), 2.75 - 2.67 (2H, m) ppm.

### (ii). 6-Methoxy-1-(2,2,2-trifluoroethyl)-2-oxo-1,2,3,4-tetrahydroquinoline-7-carboxaldehyde (Compound 39)

This compound was prepared from compound **38** in a procedure analogous to that used to make compound **3.** ¹H-NMR (270MHz) δ (CDCl₃) 10.43 (1H, s), 7.53 (1H, s), 6.86 (1H, s), 4.68 (2H, q, *J* = 8.4 Hz), 3.95 (3H, s), 3.05 - 2.96 (2H, m), 2.78 - 2.69 (2H, m) ppm.

### (iii). (2S,3S)-1-tert-Butoxycarbonyl-3-[[6-methoxy-1-(2,2,2-trifluoroethyl)-2-oxo-1,2,3,4-tetrahydroquinolin-7-yl]methyl]amino-2-phenylpiperidine (Compound 40)

This compound was prepared from compound **39** and compound **9** in a procedure analogous to that used to make compound **11.** ¹H-NMR (270MHz) δ (CDCl₃) 7.60 - 7.50 (2H, m), 7.35 - 7.18 (3H, m), 6.98 (1H, s), 6.63 (1H, s), 5.53 - 5.38 (1H, m), 4.70 - 4.50 (2H, m), 3.98 - 3.70 (3H, m), 3.72 (3H, s), 3.08 - 2.80 (4H, m), 2.70 - 2.62 (2H, m), 1.90 - 1.40 (4H, m), 1.39 (9H, s) ppm.

### (iv). (2S,3S)-3-[[6-Methoxy-1-(2,2,2-trifluoroethyl)-2-oxo-1,2,3,4-tetrahydroquinolin-7-yl]methyl]amino-2-phenylpiperidine dihydrochloride (Compound 41)

This compound was prepared from compound **40** in a procedure analogous to that used to make compound **26.** mp 243 - 245°C. IR vₘₐₓ (KBr) 3450, 2940, 2785, 2700, 1679, 1578, 1427, 1260, 1169, 1154, 1037 cm⁻¹. ¹H-NMR (270 MHz) δ (free base; CDCl₃) 7.42 - 7.22 (5H, m), 6.87 (1H, s), 6.53 (1H, s), 4.82 - 4.48 (2H, m), 3.95 (1H, d, *J* = 2.2 Hz), 3.65 (1H, *J* = 14.3 Hz), 3.54 (3H, s), 3.41 (1H, d, *J* = 14.3 Hz), 3.40 - 3.30 (1H, m), 2.95 - 2.80 (4H, m), 2.72 - 2.62 (2H, m), 2.20 -1.95 (2H, m), 1.73 - 1.45 (2H, m) ppm. Analysis: % Calculated for C₂₄H₂₈F₃N₃O₂•2HCl: C; 55.39 %, H, 5.81 %, N, 8.07 %. Found: C; 55.05 %, H; 5.87 %, N, 8.08 %.

### Example 11

### Preparation of (2S,3S)-3-[[1-Methyl-6-(2,2,2-trifluoroethoxy)-2-oxo-1,2,3,4-tetrahydroquinolin-7-yl]methyl]amino-2-phenylpiperidine dihydrochloride (Compound 45)

### (i). 1-Methyl-6-(2,2,2-trifluoroethoxy)-2-oxo-1,2,3,4-tetrahydroquinoline(Compound 42)

This compound was prepared from compound **21** and CH₃SO₃CH₂CF₃ in a procedure analogous to that used to make compound **23.** ¹H-NMR (270MHz) δ (CDCl₃) 6.95 - 6.78 (3H, m), 4.33 (2H, q, *J* = 8.1 Hz), 3.34 (3H, s), 2.93 - 2.84 (2H, m), 2.68 - 2.59 (2H, m) ppm.

### (ii). 1-Methyl-6-(2,2,2-trifluoroethoxy)-2-oxo-1,2,3,4-tetrahydroquinoline-7-carboxaldehyde (Compound 43)

This compound was prepared from compound **42** in a procedure analogous to that used to make compound **3.** This was employed in the next step without further purification.

### (iii). (2S,3S)-1-tert-Butoxycarbonyl-3-[[1-methyl-6-(2,2,2-trifluoroethoxy)-2-oxo-1,2,3,4-tetrahydroquinolin-7-yl]methyl]amino-2-phenylpiperidine (Compound 44)

This compound was prepared from compound **43** and compound **9** in a procedure analogous to that used to make compound **11.** ¹H-NMR (270MHz) δ (CDCl₃) 7.64 - 7.52 (2H, m), 7.36 - 7.22 (3H, m), 6.91 (1H, s), 6.63 (1H, s), 5.52 - 5.42 (1H, m), 4.29 (2H, q, *J* = 8.4 Hz), 4.02 - 3.90 (1H, m), 3.93 - 3.78 (2H, m), 3.29 (3H, s), 3.12 - 2.88 (2H, m), 2.90 - 2.80 (2H, m), 2.67 - 2.57 (2H, m), 1.98 - 1.50 (4H, m), 1.40 (9H, s) ppm.

### (iv). (2S,3S)-3-[[1-Methyl-6-(2,2,2-trifluoroethoxy)-2-oxo-1,2,3,4-tetrahydroquinolin-7-yl]methyl]amino-2-phenylpiperidine dihydrochloride (Compound 45)

This compound was prepared from compound **44** in a procedure analogous to that used to make compound **26.** mp 239 - 240°C. IR vₘₐₓ (KBr) 3440, 2955, 2775, 1650, 1520, 1471, 1454, 1292, 1245, 1158 cm⁻¹. ¹H-NMR (270 MHz) δ (free base; CDCl₃) 7.38 - 7.20 (5H, m), 6.67 (1H, s), 6.55 (1H, s), 4.13 (2H, q, *J* = 8.4 Hz), 3.95 (1H, d, *J* = 1.8 Hz), 3.60 (1H, *J* = 14.3 Hz), 3.41 (1H, d, *J* = 14.3 Hz), 3.38 - 3.27 (1H, m), 3.19 (3H, s), 2.96 - 2.77 (4H, m), 2.65 - 2.57 (2H, m), 2.20 - 2.07 (1H, m), 2.00 - 1.37 (3H, m) ppm. Analysis: % Calculated for C₂₄H₂₈F₃N₃O₂•2HCl•2H₂O: C; 51.80 %, H, 6.16 %, N, 7.55 %. Found: C; 51.45 %. H; 5.91 %, N, 7.38 %.

### Example 12

### Preparation of (2S,3S)-3-[(6-Methoxy-1-methyl-2-thioxo-1,2,3,4-tetrahydroquinolin-7-yl)methyl]amino-2-phenylpiperidine dihydrochloride (Compound 49)

### (i). 6-Methoxy-1-methyl-2-thioxo-1,2,3,4-tetrahydroquinoline (Compound 46)

To a stirred solution of compound **15** (350 mg, 1.83 mmol) in toluene (5 ml) was added Lawesson's Reagent (407 mg, 1.01 mmol), and refluxed for 1.5 hours. The solvent was evaporated, and the residue was purified by a column chromatography on silica gel to give compound **46** (363 mg, 1.75 mmol, 96%) as a white solid. ¹H-NMR (270MHz) δ (CDCl₃) 7.06 (1H, d, *J* = 8.8 Hz), 6.79 (1H, dd, *J* = 8.8, 2.9 Hz), 6.72 (1H, d, *J* = 2.9 Hz), 3.89 (3H, s), 3.81 (3H, s), 3.21 - 3.14 (2H, m), 2.81 - 2.74 (2H, m) ppm.

### (ii). 6-Methoxy-1-methyl-2-thioxo-1,2,3,4-tetrahydroquinoline-7-carboxaldehyde (Compound 47)

This compound was prepared from compound **46** in a procedure analogous to that used to make compound **3.** ¹H-NMR (270MHz) δ (CDCl₃) 10.44 (1H, s), 7.60 (1H, s), 6.83 (1H, s), 3.96 (3H, s), 3.92 (3H, s), 3.24 - 3.16 (2H, m), 2.91 - 2.83 (2H, m) ppm.

### (iii). (2S,3S)-1-tert-Butoxycarbonyl-3-[(6-methoxy-1-methyl-2-thioxo-1,2,3,4-tetrahydroquinolin-7-yl)methyl]amino-2-phenylpiperidine (Compound 48)

This compound was prepared from compound **47** and compound **9** in a procedure analogous to that used to make compound **11.** ¹H-NMR (270MHz) δ (CDCl₃) 7.60 - 7.53 (2H, m), 7.36 - 7.23 (3H, m), 7.04 (1H, s), 6.60 (1H, s), 5.53 - 5.45 (1H, m), 3.99 - 3.89 (1H, m), 3.85 (3H; s), 3.82 (2H, s), 3.73 (3H, s), 3.19 - 3.12 (2H, m), 3.11 - 2.92 (2H, m), 2.78 - 2.70 (2H, m), 1.90 - 1.50 (4H, m), 1.40 (9H, s) ppm.

### (iv). (2S,3S)-3-[(6-Methoxy-1-methyl-2-thioxo-1,2,3,4-tetrahydroquinolin-7-yl)methyl]amino-2-phenylpiperidine dihydrochloride (Compound 49)

This compound was prepared from compound **48** in a procedure analogous to that used to make compound **26.** mp 245 - 246°C. IR vₘₐₓ (KBr) 3445, 2930, 2680, 1561, 1477, 1434, 1259, 1105 cm⁻¹. ¹H-NMR (270 MHz) δ (free base; CDCl₃) 7.33 - 7.21 (5H, m), 6.82 (1H, s), 6.50 (1H, s), 3.92 (1H, d, *J* = 2.2 Hz), 3.75 (3H, s), 3.66 (1H, d, *J* = 13.9 Hz), 3.55 (3H, s), 3.43 (1H, d, *J* = 13.9 Hz), 3.33 - 3.24 (1H, m), 3.17 - 3.10 (2H, m), 2.88 - 2.67 (4H, m), 2.20 - 2.11 (1H, m), 2.03 - 1.80 (1H, m), 1.75 - 1.55 (1H, m), 1.54 - 1.42 (1H, m) ppm. Analysis: % Calculated for C₂₃H₂₉N₃OS•2HCl•0.1H₂O: C; 58.46 %, H, 6.88 %, N, 8.82 %. Found: C; 58.74 %, H; 6.69 %, N, 8.93 %.

### Example 13

### Preparation of (2S,3S)-3-[(7-Methoxy-1-methyl-2-oxo-2,3,4,5-tetrahydro-1H-benzazepin-8-yl)methyl]amino-2-phenylpiperidine dihydrochloride (Compound 54)

### (i). 7-Methoxy-2-oxo-2,3,4,5-tetrahydro-1H-benzazepine (Compound 50)

To a stirred solution of 6-methoxy-1-tetralone (1.0 g, 5.7 mmol) in CH₂Cl₂ (10 ml) was added cone. H₂SO₄ (5 ml) at 0°C, and then NaN₃(1.0 g) was added gradually over 30 minutes. The mixture was warmed up to room temperature, and stirred for 3hours. The mixture was cooled, made basic with NaOH aq., and extracted with CH₂Cl₂. The organic layers were combined, dried (MgSO₄), filtered, and concentrated. the residue was purified by a column chromatography on silica gel to give compound **50** (0.10 g, 0.52 mmol, 9.2%) as a white solid. ¹H-NMR (270MHz) δ (CDCl₃) 7.65 (1H, br. s), 6.94 - 6.88 (1H, m), 6.78 - 6.68 (2H, m), 3.81 (3H, s), 2.77 (2H, t, *J* = 7.0 Hz), 2.37 - 2.15 (4H, m) ppm.

### (ii). 7-Methoxy-2-oxo-2,3,4,5-tetrahydro-1H-benzazepine-8-carboxaldehyde (Compound 51)

This compound was prepared from compound **50** in a procedure analogous to that used to make compound **3.** This was employed in the next step without further purification.

### (iii). 7-Methoxy-1-methyl-2-oxo-2,3,4,5-tetrahydro-1H-benzazepine-8-carboxaldehyde (Compound 52)

This compound was prepared from compound 51 in a procedure analogous to that used to make compound **30.** ¹H-NMR (270MHz) δ (CDCl₃) 10.43 (1H, s), 7.63 (1H, s), 6.85 (1H, s), 3.97 (3H, s), 3.33 (3H, s), 2.82 - 2.74 (2H, m), 2.38 - 2.13 (4H, m) ppm.

### (iv). (2S,3S)-1-tert-Butoxycarbonyl-3-[(7-methoxy-1-methyl-2-oxo-2,3,4,5-tetrahydro-1H-benzazepin-8-yl)methyl]amino-2-phenylpiperidine (Compound 53)

This compound was prepared from compound **52** and compound **9** in a procedure analogous to that used to make compound **11.** ¹H-NMR (270MHz) δ (CDCl₃) 7.62 - 7.55 (2H, m), 7.38 - 7.22 (3H, m), 7.01 (1H, s), 6.60 (1H, s), 5.53 - 5.45 (1H, m), 4.00 - 3.72 (3H, m), 3.72 (3H, s), 3.27 (3H, s), 3.12 - 2.92 (2H, m), 2.70 - 2.60 (2H, m), 2.30 - 2.05 (4H, m), 1.92 - 1.40 (4H, m), 1.40 (9H, s) ppm.

### (v). (2S,3S)-3-[(7-Methoxy-1-methyl-2-oxo-2,3,4,5-tetrahydro-1H-benzazepin-8-yl)methyl]amino-2-phenylpiperidine dihydrochloride (Compound 54)

This compound was prepared from compound **53** in a procedure analogous to that used to make compound **26.** mp 236 - 238°C. IR vₘₐₓ (KBr) 3445, 2935, 2740, 1656, 1508, 1435, 1248, 1166 cm⁻¹. ¹H-NMR (270 MHz) δ (free base; CDCl₃) 7.43 - 7.25 (5H, m), 6.82 (1H, s), 6.50 (1H, s), 4.12 - 4.08 (1H, m), 3.70 (1H, d, *J* = 13.9 Hz), 3.58 - 3.43 (2H, m), 3.52 (3H, s), 3.22 (3H, s), 3.00 - 2.86 (2H, m), 2.68 - 2.58 (2H, m), 2.30 - 2.00 (6H, m), 1.75 - 1.54 (2H, m) ppm. Analysis: % Calculated for C₂₄H₃₁N₃O₂•2HCl•0.5H₂O: C, 60.63 %, H, 7.21 %, N, 8.84 %. Found: C, 60.95 %, H, 7.11 %, N, 8.87 %.

### Example 14

### Preparation of (2S,3S)-3-[(7-Methoxy-1-methyl-2-oxo-1,2,3,4-tetrahydroquinolin-6-yl)methyl]amino-2-phenylpiperidine dihydrochloride (Compound 59)

### (i). 7-Methoxy-2-oxo-1,2,3,4-tetrahydroquinoline (Compound 55)

This compound was prepared according to the procedure (*Chem. Pharm. Bull*., **9,** 970 (1961)).

### (ii). 7-Methoxy-1-methyl-2-oxo-1,2,3,4-tetrahydroquinoline (Compound 56)

This compound was prepared from compound **55** in a procedure analogous to that used to make compound **30.** ¹H-NMR (270 MHz) δ (CDCl₃) 7.06 (1H, d, *J* = 8.8 Hz), 6.57 - 6.48 (2H, m), 3.81 (3H, s), 3.33 (3H, s), 2.86 - 2.78 (2H, m), 2.68 - 2.58 (2H, m) ppm.

### (iii). 7-Methoxy-1-methyl-2-oxo-1,2,3,4-tetrahydroquinoline-6-carboxaldehyde (Compound 57)

This compound was prepared from compound **56** in a procedure analogous to that used to make compound **3.** ¹H-NMR (270MHz) δ (CDCl₃) 10.35 (1H, s), 7.65 (1H, s), 6.52 (1H, s), 3.96 (3H, s), 3.41 (3H, s), 2.93 - 2.85 (2H, m), 2.70 - 2.62 (2H, m) ppm.

### (iv). (2S,3S)-1-tert-Butoxycarbonyl-3-[(7-methoxy-1-methyl-2-oxo-1,2,3,4-tetrahydroquinolin-6-yl)methyl]amino-2-phenylpiperidine (Compound 58)

This compound was prepared from compound **57** and compound **9** in a procedure analogous to that used to make compound 11. ¹H-NMR (270MHz) δ (CDCl₃) 7.62 - 7.55 (2H, m), 7.36 - 7.22 (3H, m), 6.97 (1H, s), 6.44 (1H, s), 5.53 - 5.46 (1H, m), 3.99 - 3.89 (1H, m), 3.85 - 3.69 (2H, m), 3.72 (3H, s), 3.35 (3H, s), 3.10 - 2.91 (2H, m), 2.84 - 2.76 (2H, m), 2.64 - 2.58 (2H, m), 1.88 -1.52 (4H, m), 1.40 (9H, s) ppm.

### (v) (2S,3S)-3-[(7-Methoxy-1-methyl-2-oxo-1,2,3,4-tetrahydroquinolin-6-yl)methyl]amino-2-phenylpiperidine dihydrochloride (Compound 59)

This compound was prepared from compound **58** in a procedure analogous to that used to make compound **26.** mp 243 - 244°C. IR vₘₐₓ (KBr) 3450, 2940, 2785, 2700, 1679, 1578, 1427, 1260, 1169, 1154 cm⁻¹. ¹H-NMR (270 MHz) δ (free base; CDCl₃) 7.38 - 7.23 (5H, m), 6.72 (1H, s), 6.32 (1H, s), 3.91 (1H, d, *J* = 2.2 Hz), 3.61 (1H, d, *J* = 13.6 Hz), 3.50 (3H, s), 3.39 (1H, d, *J* = 13.6 Hz), 3.35 - 3.27 (1H, m), 3.32 (3H, s), 2.88 - 2.57 (6H, m), 2.20 - 2.12 (1H, m), 2.02 - 1.85 (1H, m), 1.70 - 1.55 (1H, m), 1.49 - 1.38 (1H, m) ppm. *Anal. Calc*. for C₂₃H₂₉N₃O₂•2HCl: C, 61.06 %, H, 6.91 %, N, 9.29 %. Found: C, 60.67 %, H, 6.97 %, N, 9.57 %.

### Example 15

### Preparation of (2S,3S)-3-[(7-Methoxy-1-methyl-2-oxo-4-trifluoromethyl-1,2,3,4-tetrahydroquinolin-6-yl)methyl]amino-2-phenylpiperidine dihydrochloride (Compound 65)

### (i). 7-Methoxy-4-trifluoromethyl-2-quinolone (Compound 60)

This compound was prepared according to the procedure (*J. Org. Chem.* **45,** 2285 (1980)).

### (ii). 7-Methoxy-1-methyl-4-trifluoromethyl-2-quinolone (Compound 61)

This compound was prepared from compound **60** in a procedure analogous to that used to make compound **30.** ¹H-NMR (270 MHz) δ (CDCl₃) 7.82 - 7.75 (1H, m), 6.95 - 6.83 (3H, m), 3.94 (3H, s), 3.72 (3H, s) ppm.

### (iii). 7-Methoxy-1-methyl-2-oxo-4-trifluoromethyl-1,2,3,4-tetrahydroquinoline (Compound 62)

The solution of **61** (200 mg, 0.78 mmol) in methanol (6 ml) was hydrogenated over 10% Pd-C (0.1 g) at atmospheric pressure for 22 hours. The catalyst was filtered off, and washed with methanol. The filtrate was concentrated to give **62** (190 mg, 0.75 mmol, 96%) as a white solid. ¹H-NMR (270 MHz) δ (CDCl₃) 7.19 (1H, d, *J* = 8.1 Hz), 6.65 - 6.58 (2H, m), 3.84 (3H, s), 3.60 - 3.42 (1H, m), 3.35 (3H, s), 3.07 - 2.83 (2H, m) ppm.

### (iv). 7-Methoxy-1-methyl-2-oxo-4-trifluoromethyl-1,2,3,4-tetrahydroquinoline-6-carboxaldehyde (Compound 63)

This compound was prepared from compound **62** in a procedure analogous to that used to make compound **3**. ¹H-NMR (270MHz) δ (CDCl₃) 10.35 (1H, s), 7.77 (1H, s), 6.58 (1H, s), 4.00 (3H, s), 3.65 - 3.51 (1H, m), 3.43 (3H, s), 3.07 (1H, dd, *J* = 16.9, 2.2 Hz), 2.91 (1H, dd, *J* = 16.9, 7.3 Hz) ppm.

### (v). (2S,3S)-1-tert-Butoxycarbonyl-3-[(7-methoxy-1-methyl-2-oxo-4-trifluoromethyl-1,2,3,4-tetrahydroquinolin-6-yl)methyl]amino-2-phenylpiperidine (Compound 64)

This compound was prepared from compound **63** and compound **9** in a procedure analogous to that used to make compound 11. ¹H-NMR (270MHz) δ (CDCl₃) 7.61 - 7.52 (2H, m), 7.36 - 7.22 (3H, m), 7.12 and 7.52 (total 1H, each s), 6.46 (1H, s), 5.52 - 5.42 (1H, m), 4.00 - 3.89 (1H, m), 3.88 - 3.71 (2H, m), 3.75 (3H, s), 3.52 - 3.37 (1H, m), 3.36 (3H, s), 3.09 - 2.76 (4H, m), 1.90 - 1.45 (4H, m), 1.40 (9H, s) ppm.

### (vi). (2S,3S)-3-[(7-Methoxy-1-methyl-2-oxo-1,2,3,4-tetrahydroquinolin-6-yl)methyl]amino-2-phenylpiperidine dihydrochloride (Compound 65)

This compound was prepared from compound **64** in a procedure analogous to that used to make compound **26.** mp 255 - 256°C. IR vₘₐₓ (KBr) 3430, 2935, 2660, 1680, 1626, 1416, 1371, 1340, 1124, 1113 cm⁻¹. ¹H-NMR (270 MHz) δ (free base; CDCl₃) 7.37 - 7.22 (5H, m), 6.81 and 6.78 (total 1H, each s), 6.36 and 6.31 (total 1H, each s), 3.93 - 3.88 (1H, m), 3.70 - 3.53 (4H, m), 3.46 - 3.24 (6H, m), 3.02 - 2.75 (4H, m), 2.17 - 2.05 (1H, m), 2.03 - 1.38 (3H, m) ppm. Analysis: % Calculated for C₂₄H₂₈F₃N₃O₂•2HCl: C, 55.39 %, H, 5.81 %, N, 8.07 %. Found: C, 55.03 %, H, 5.99 %, N, 7.91 %.

### Example 16

### Preparation of (2S,3S)-3-[(6-Methoxy-1-methyl-2-oxo-4H-3,1-benzoxazin-7-yl)methyl]-amino-2-phenylpiperidine dihydrochloride (Compound 70)

### (i). 6-Methoxy-4H-3,1-benzoxazin-2-one (Compound 66)

This compound was prepared according to the procedure (*J. Med. Chem*. , **30,** 295 (1987)).

### (ii). 6-Methoxy-1-methyl-4H-3,1-benzoxazin-2-one (Compound 67)

This compound was prepared from compound **66** in a procedure analogous to that used to make compound **30.** ¹H-NMR (270 MHz) δ (CDCl₃) 6.90 - 6.83 (2H, m), 6.71 - 6.67 (1H, m), 5.16 (2H, s), 3.80 (3H, s), 3.35 (3H, s) ppm.

### (iii). 6-Methoxy-1-methyl-2-oxo-4H-3,1-benzoxazine-7-carboxaldehyde (Compound 68)

This compound was prepared from compound **67** in a procedure analogous to that used to make compound **3.** ¹H-NMR (270MHz) δ (CDCl₃) 10.46 (1H, s), 7.39 (1H, s), 6.81 (1H, s), 5.22 (2H, s), 3.94 (3H, s), 3.40 (3H, s) ppm.

### (iv). (2S,3S)-1-tert-Butoxycarbonyl-3-[(6-methoxy-1-methyl-2-oxo-4H-3,1-benzoxazin-7-yl)methyl]amino-2-phenylpiperidine (Compound 69)

This compound was prepared from compound **68** and compound **9** in a procedure analogous to that used to make compound **11.** ¹H-NMR (270MHz) δ (CDCl₃) 7.61 - 7.52 (2H, m), 7.35 - 7.22 (3H, m), 6.85 (1H, s), 6.57 (1H, s), 5.55 - 5.43 (1H, m), 5.14 (2H, s), 4.00 - 3.92 (1H, m), 3.91 - 3.87 (2H, m), 3.70 (3H, s), 3.32 (3H, s), 3.09 - 2.92 (2H, m), 1.92 - 1.50 (41H, m), 1.40 (9H, s) ppm.

### (v). (2S,3S)-3-[(6-Methoxy-1-methyl-2-oxo-4H-3,1-benzoxazin-7-yl)methyl]amino-2-phenylpiperidine dihydrochloride (Compound 70)

This compound was prepared from compound **69** in a procedure analogous to that used to make compound **26.** mp 235 - 237°C. IR vₘₐₓ (KBr) 3420, 2935, 2665, 1728, 1564, 1508, 1481, 1429, 1302, 1037 cm⁻¹. ¹H-NMR (270 MHz) δ (free base; CDCl₃) 7.35 - 7.20 (5H, m), 6.61 (1H, s), 6.47 (1H, s), 5.11 (2H, s), 3.90 - 3.87 (1H, m), 3.65 (1H, d, *J* = 14.3 Hz), 3.53 (3H, s), 3.44 (1H, d, *J* = 14.3 Hz), 3.30 - 3.18 (1H, m), 3.21 (3H, s), 2.86 - 2.74 (2H, m), 2.18 - 2.07 (1H, m), 1.98 - 1.38 (3H, m) ppm. Analysis: % Calculated for C₂₂H₂₇N₃O₃•2HCl: C, 58.15 %, H, 6.43 %, N, 9.25 %. Found: C, 57.83 %, H, 6.36 %, N, 9.18 %.

### Example 17

### Preparation of (2S,3S)-3-[(6-Methoxy-1-methyl-2-oxo-4H-3,1-benzothiazin-7-yl)methyl]-amino-2-phenylpiperidine dihydrochloride (Compound 75)

### (i). 6-Methoxy-4H-3,1-benzothiazin-2-one (Compound 71)

This compound was prepared according to the procedure (*J. Med. Chem*. **30,** 295 (1987)).

### (ii). 6-Methoxy-1-methyl-4H-3,1-benzothiazin-2-one (Compound 72)

This compound was prepared from compound **71** in a procedure analogous to that used to make compound **30.** ¹H-NMR (270 MHz) δ (CDCl₃) 6.98 (1H, d, *J* = 8.8 Hz), 6.84 (1H, dd, *J* = 8.8, 2.9 Hz), 6.75 (1H, d, *J*=2.9 Hz), 3.93 (2H, s), 3.81 (3H, s), 3.42 (3H, s) ppm.

### (iii). 6-Methoxy-1-methyl-2-oxo-4H-3,1-benzothiazine-7-carboxaldehyde (Compound 73)

This compound was prepared from compound **72** in a procedure analogous to that used to make compound **3**. ¹H-NMR (270MHz) δ (CDCl₃) 10.45 (1H, s), 7.52 (1H, s), 6.86 (1H, s), 3.99 (2H, s), 3.96 (3H, s), 3.43 (3H, s) ppm.

### (iv). (2S,3S)-1-tert-Butoxycarbonyl-3-[(6-methoxy-1-methyl-2-oxo-4H-3,1-benzothiazin-7-yl)methyl]amino-2-phenylpiperidine (Compound 74)

This compound was prepared from compound **73** and compound **9** in a procedure analogous to that used to make compound **11.** ¹H-NMR (270MHz) δ (CDCl₃) 7.62 - 7.54 (2H, m), 7.37 - 7.22 (3H, m), 6.96 (1H, s), 6.62 (1H, s), 5.54 - 5.42 (1H, m), 4.02 - 3.90 (1H, m), 3.91 (2H, s), 3.82 (2H, s), 3.72 (3H, s), 3.38 (3H, s), 3.10 - 2.93 (2H, m), 1.93 - 1.50 (4H, m), 1.40 (9H, s) ppm.

### (v). (2S,3S)-3-[(6-Methoxy-1-methyl-2-oxo-4H-3,1-benzothiazin-7-yl)methyl]amino-2-phenylpiperidine dihydrochloride (Compound 75)

This compound was prepared from compound **74** in a procedure analogous to that used to make compound **26**.

mp 252 - 254°C. IR vₘₐₓ (KBr) 3450, 2935, 2655, 1647, 1562, 1514, 1470, 1450, 1434, 1416, 1269, 1166, 1038 cm⁻¹. ¹H-NMR (270 MHz) δ (free base; CDCl₃) 7.36 - 7.19 (5H, m), 6.72 (1H, s), 6.52 (1H, s), 3.93 - 3.87 (3H, m), 3.64 (1H, d, *J* = 14.3 Hz), 3.54 (3H, s), 3.43 (1H, d, *J* = 14.3 Hz), 3.31 - 3.22 (1H, m), 3.28 (3H, s), 2.87 - 2.74 (2H, m), 2.18 - 2.07 (1H, m), 2.00 - 1.40 (3H, m) ppm. Analysis: % Calculated for C₂₂H₂₇N₃O₂S•2HCl: C, 56.17 %, H, 6.21 %, N, 8.93 %. Found: C, 55.81 %, H, 6.37 %, N, 8.67 %.

### Example 18

### Preparation of (2S,3S)-3-((7-Methoxy-4-methyl-3-oxo-3,4-dihydro-1,4-benzothiadin-6-yl)methyl)amino-2-phenylpiperidine dihydrochloride (Compound 79)

### (i). 7-Methoxy-4-methyl-3-oxo-3,4-dihydro-1,4-benzothiadine (Compound 76)

This compound was prepared according to the procedure (*Indian J. Chem. sect. B*, **29B**, 297(1990)).

### (ii). 7-Methoxy-4-methyl-3-oxo-3,4-dihydro-1,4-benzothiadine-6-carboxaldehyde (Compound 77)

This compound was prepared from compound **76** in a procedure analogous to that used to make compound **3**. ¹H-NMR (270 MHz) δ (CDCl₃) 10.40 (1H, s), 7.53 (1H, s), 6.99 (1H, s), 3.93 (3H, s), 3.46 (2H, s), 3.45 (3H, s) ppm.

### (iii). (2S,3S)-1-tert-Butoxycarbonyl-3-((7-methoxy-4-methyl-3-oxo-3,4-dihydro-1,4-benzothiadin-6-yl)methyl)amino-2-phenylpiperidine (Compound 78)

This compound was prepared from compound **77** and compound **9** in a procedure analogous to that used to make compound **11.** ¹H-NMR (270 MHz) δ (CDCl₃) 7.63 - 6.75 (7H, m), 5.48 (1H, br), 4.06 - 2.94 (14H, m), 1.95 - 1.20 (4H, m), 1.40 (9H, s) ppm. This was employed in the next step without further purification.

### (iv). (2S,3S)-3-((7-Methoxy-4-methyl-3-oxo-3,4-dihydro-1,4-benzothiadin-6-yl)methyl)amino-2-phenylpiperidine dihydrochloride (Compound 79)

This compound was prepared from compound **78** in a procedure analogous to that used to make compound **26**. mp 263 - 269°C. ¹H-NMR (270 MHz) δ (free base; CDCl₃) 7.39 - 6.99 (7H, m), 3.92- 2.75 (6H, m), 3.52 (3H, s), 3.36 (2H, s), 3.28 (3H, s), 2.20 - 1.40 (4H, m) ppm.

### Example 19

### Preparation of (2S,3S)-3-((7-Methoxy-4-methyl-3-oxo-3,4-dihydro-1,4-benzoxadin-6-yl)methyl)amino-2-phenylpiperidine dihydrochloride (Compound 83)

### (i). 7-Methoxy-4-methyl-3-oxo-3,4-dihydro-1,4-benzoxadine (Compound 80)

This compound was prepared according to the procedure set forth in U.S. Patent No. 4552956.

### (ii). 7-Methoxy-4-methyl-3-oxo-3,4-dihydro-1,4-benzoxadine-6-carboxaldehyde (Compound 81)

This compound was prepared from compound **80** in a procedure analogous to that used to make compound **3.** ¹H-NMR (270 MHz) δ (CDCl₃) 10.35 (1H, s), 7.45 (1H, s), 6.62 (1H, s), 4.70 (2H, s), 3.91 (3H, s), 3.38 (3H, s) ppm.

### (iii). (2S,3S)-1-tert-Butoxycarbonyl-3-((7-methoxy-4-methyl-3-oxo-3,4-dihydro-1,4-benzoxadin-6-yl)methyl)amino-2-phenylpiperidine (Compound 82)

This compound was prepared from compound **81** and compound **9** in a procedure analogous to that used to make compound **11.** ¹H-NMR (270 MHz) δ (CDCl₃) 7.63 - 6.49 (7H, m), 5.50 (1H, br), 4.56 (2H, s), 4.00 - 2.90 (6H, m), 3.67 (3H, s), 3.30 (3H, s), 1.90 - 1.30 (4H, m), 1.40 (9H, s) ppm. This was employed in the next step without further purification.

### (iv). (2S,3S)-3-((7-Methoxy-4-methyl-3-oxo-3,4-dihydro-1,4-benzoxadin-6-yl)methyl)amino-2-phenylpiperidine dihydrochloride (Compound 83)

This compound was prepared from compound **82** in a procedure analogous to that used to make compound **26.** mp 263 - 267°C. ¹H-NMR (270 MHz) δ (free base; CDCl₃) 7.36 - 6.39 (7H, m), 4.55 (2H, s), 3.92 - 2.72 (6H, m), 3.49 (3H, s), 3.21 (3H, s), 2.20 - 1.37 (4H, m) ppm.

### Example 20

### Preparation of (2S,3S)-3-[(6-Methoxy-1,3,3-trimethyl-2-thioxo-2,3-dihydroindol-5-yl) methyl]amino-2-phenylpiperidine dihydrochloride (Compound 87)

### (i). 6-Methoxy-1,3,3-trimethyl-2-thioxo-2,3-dihydroindol (Compound 84)

This compound was prepared from compound **2** in a procedure analogous to that used to make compound **46.** ¹H-NMR (270MHz) δ (CDCl₃) 7.19 (1H, d, *J* = 8.4 Hz), 6.72 - 6.55 (2H, m), 3.85 (3H, s), 3.63 (3H, s), 1.41 (6H, s) ppm.

### (ii). 6-Methoxy-1,3,3-trimethyl-2-thioxo-2,3-dihydroindol-5-carboxaldehyde (Compound 85)

This compound was prepared from compound **84** in a procedure analogous to that used to make compound **3.** ¹H-NMR (270MHz) δ (CDCl₃) 10.42 (1H, s), 7.76 (1H, s), 6.60 (1H, s), 4.01 (3H, s), 3.68 (3H, s), 1.42 (6H, s) ppm.

### (iii). (2S,3S)-1-tert-Butoxycarbonyl-3-[(6-methoxy-1,3,3-trimethyl-2-thioxo-2,3-dihydroindol-5-yl)methyl]amino-2-phenylpiperidine (Compound 86)

This compound was prepared from compound **85** and compound **9** in a procedure analogous to that used to make compound **11.** ¹H-NMR (270MHz) δ (CDCl₃) 7.64 - 7.56 (2H, m), 7.37 - 7.22 (3H, m), 7.11 (1H, s), 6.51 (1H, s), 5.57 - 5.44 (1H, m), 3.98 - 3.70 (3H, m), 3.76 (3H, s), 3.64 (3H, s), 3.13 - 2.92 (2H, m), 1.90 - 1.40 (4H, m), 1.41 (9H, s), 1.38 (3H, s), 1.37 (3H, s) ppm.

### (iv). (2S,3S)-3-[(6-Methoxy-1,3,3-trimethyl-2-thioxo-2,3-dihydroindol-5-yl)methyl]amino-2-phenylpiperidine dihydrochloride (Compound 87)

This compound was prepared from compound **86** in a procedure analogous to that used to make compound **26.** mp 234 - 236°C, IR vₘₐₓ (KBr) 3435, 2970, 2934, 2685, 1627, 1559, 1447, 1430, 1370, 1278, 1056 cm⁻¹. ¹H-NMR (270 MHz) δ (free base; CDCl₃) 7.37 - 7.23 (5H, m), 6.89 (1H, s), 6.40 (1H, s), 3.91 (1H, d, *J* = 2.2 Hz), 3.67 (1H, d, *J* = 13.9 Hz), 3.62 (3H, s), 3.57 (3H, s), 3.42 (1H, d, J = 13.9 Hz), 3.33 - 3.22 (1H, m), 2.88 - 2.73 (2H, m), 2.19 - 1.35 (4H, m), 1.35 (3H, s), 1.34 (3H, s) ppm. Analysis: % Calculated for C₂₄H₃₁N₃OS•2HCl: C; 59.74 %, H, 6.89 %, N, 8.71 %. Found: C; 60.02 %, H; 6.91 %, N, 8.64 %.

### Example 21

### Preparation of (2S,3S)-3-[(7-Methoxy-1-methyl-2-thioxo-1,2,3,4-tetrahydroquinolin-6-yl) methyl]amino-2-phenylpiperidine dihydrochloride (Compound 91)

### (i). 7-Methoxy-1-methyl-2-thioxo-1,2,3,4-tetrahydroquinolin (Compound 88)

This compound was prepared from compound **55** in a procedure analogous to that used to make compound **46.** ¹H-NMR (270MHz) δ (CDCl₃) 7.08 (1H, d, *J* = 8.1 Hz), 6.71 (1H, d, *J* = 2.6 Hz), 6.64 (1H, dd, *J* = 8.1, 2.6 Hz), 3.89 (3H, s), 3.83 (3H, s), 3.23 - 3.14 (2H, m), 2.78 - 2.68 (2H, m) ppm.

### (ii). 7-Methoxy-1-methyl-2-thioxo-1,2,3,4-tetrahydroquinolin-6-carboxaldehyde (Compound 89)

This compound was prepared from compound **88** in a procedure analogous to that used to make compound **3.** ¹H-NMR (270MHz) δ (CDCl₃) 10.37 (1H, s), 7.65 (1H, s), 6.69 (1H, s), 3.98 (3H, s), 3.95 (3H, s), 3.24 - 3.16 (2H, m), 2.82 - 2.74 (2H, m) ppm.

### (iii). (2S,3S)-1-tert-Butoxycarbonyl-3-[(7-methoxy-1-methyl-2-thioxo-1,2,3,4-tetrahydroquinolin-6-yl)methyl]amino-2-phenylpiperidine (Compound 90)

This compound was prepared from compound **89** and compound **9** in a procedure analogous to that used to make compound **11**. ¹H-NMR (270MHz) δ (CDCl₃) 7.60 - 7.53 (2H, m), 7.37 - 7.23 (3H, m), 6.99 (1H, s), 6.56 (1H, s), 5.53 - 5.43 (1H, m), 3.99 - 3.88 (1H, m), 3.90 (3H, s), 3.85 - 3.65 (2H, m), 3.73 (3H, s), 3.18 - 3.11 (2H, m), 3.10 - 2.90 (2H, m), 2.72 - 2.63 (2H, m), 1.90 - 1.50 (4H, m), 1.41 (9H, s) ppm.

### (iv). (2S,3S)-3-[(7-Methoxy-1-methyl-2-thioxo-1,2,3,4-tetrahydroquinolin-6-yl) methyl]amino-2-phenylpiperidine dihydrochloride (Compound 91)

This compound was prepared from compound 90 in a procedure analogous to that used to make compound **26.** mp. 219 - 221°C. IR vₘₐₓ (KBr) 3435, 2940, 2660, 1626, 1558, 1464, 1430, 1416, 1369, 1338, 1103 cm⁻¹. ¹H-NMR (270 MHz) δ (free base; CDCl₃) 7.42 - 7.23 (5H, m), 6.80 (1H, s), 6.45 (1H, s), 4.05 - 4.01 (1H, m), 3.87 (3H ,s), 3.66 (1H, d, *J* = 13.9 Hz), 3.52 (3H, s), 3.50 - 3.40 (1H, m), 3.43 (1H, d, *J* = 13.9 Hz), 3.18 - 3.10 (2H, m), 2.97 - 2.83 (2H, m), 2.68 - 2.58 (2H, m), 2.20 - 2.00 (2H, m), 1.80 - 1.50 (2H, m) ppm. Analysis: % Calculated for C₂₃H₂₉N₃OS•2HCl•0.5H₂O: C; 57.85 %, H, 6.75 %, N, 8.80 %. Found: C; 57.81 %, H; 6.52 %, N, 8.68 %.

### Example 22

### Preparation of (2S,3S)-3-((1,6-Dimethoxy-2-oxo-1,2,3,4-tetrahydroquinolin-7-yl)methyl)amino-2-phenylpiperidine dihydrochloride (Compound 95)

### (i). 1,6-Dimethoxy-2-oxo-1,2,3,4-tetrahydroquinoline (Compound 92)

This compound was prepared according to the procedure (*Tetrahedron*, **43,** 2577(1987)).

### (ii). 1,6-Dimethoxy-2-oxo-1,2,3,4-tetrahydroquinoline-7-carboxaldehyde (Compound 93)

This compound was prepared from compound **92** in a procedure analogous to that used to make compound **3.**

### (iii). (2S,3S)-1-tert-Butoxycarbonyl-3-((1,6-dimethoxy-2-oxo-1,2,3,4-tetrahydroquinolin-7-yl)methyl)amino-2-phenylpiperidine (Compound 94)

This compound was prepared from compound **93** and compound **9** in a procedure analogous to that used to make compound **11.** This was employed in the next step without further purification.

### (v). (2S,3S)-3-((1,6-Dimethoxy-2-oxo-1,2,3,4-tetrahydroquinolin-7-yl)methyl)amino-2-phenylpiperidine dihydrochloride (Compound 95)

This compound was prepared from compound **94** in a procedure analogous to that used to make compound **26.**

### Example 23

### Preparation of (2S,3S)-3-[(1-Difluoromethyl-6-methoxy-2-oxo-1,2,3,4-tetrahydroquinolin-7-yl)methyl]amino-2-phenylpiperidine dihydrochloride (Compound 99)

### (i). 6-Methoxy-2-oxo-1,2,3,4-tetrahydroquinoline-7-carboxaldehyde (Compound 96)

This compound was prepared from compound **29** in a procedure analogous to that used to make compound **3.** ¹H-NMR (270 MHz) δ (CDCl₃) 10.40 (s, 1H), 7.80 (br, 1H), 7.22 (s, 1H), 6.84 (s, 1H), 3.92 (s, 3H), 3.03 (t, 2H, *J* = 7Hz), 2.64 (t, 2H, *J* = 7Hz).

### (ii). 1-Difluoromethyl-6-methoxy-2-oxo-1,2,3,4-tetrahydroquinoline-7-carboxaldehyde (Compound 97)

This compound was prepared from compound **96** and CF₂HCl in a procedure analogous to that used to make compound **30.** ¹H-NMR (270 MHz) δ (CDCl₃) 10.42 (s, 1H), 7.92 (s, 1H), 7.70 (t, 1H, *J* = 59.7Hz), 6.86 (s, 1H), 3.94 (s, 3H), 3.04 - 2.95 (m, 2H), 2.75 - 2.67 (m, 2H).

### (iii). (2S,3S)-1-tert-Butoxycarbonyl-3-[(1-difluoromethyl-6-methoxy-2-oxo-1,2,3,4-tetrahydroquinolin-7-yl)methyl]amino-2-phenylpiperidine (Compound 98)

This compound was prepared from compound **97** and compound **9** in a procedure analogous to that used to make compound **11.** ¹H-NMR (270 MHz) δ (CDCl₃) 7.71 (t, 1H, *J* = 60.1 Hz), 7.62 - 7.52 (m, 2H), 7.43 (s, 1H), 7.35 - 7.20 (m, 3H), 6.63 (s, 1H), 5.45 - 5.32 (m, 1H), 4.02 - 3.92 (m, 1H), 3.81 (s, 2H), 3.71 (s, 3H), 3.12 - 2.95 (m, 2H), 2.93 - 2.84 (m, 2H), 2.69 - 2.61 (m, 2H), 1.93 - 1.50 (m, 4H), 1.39 (s, 9H).

### (iv). (2S,3S)-3-[(1-Difluoromethyl-6-methoxy-2-oxo-1,2,3,4-tetrahydroquinolin-7-yl)methyl]amino-2-phenylpiperidine dihydrochloride (Compound 99)

This compound was prepared from compound **98** in a procedure analogous to that used to make compound **26.** ¹H-NMR (270 MHz) δ (CDCl₃) 7.69 (t, 1H, *J* = 60.1Hz), 7.42 - 7.15 (m, 6H), 6.50 (s, 1H), 3.92 (d, 1H, *J* = 1.8 Hz), 3.67 - 3.26 (m, 3H), 3.46 (s, 3H), 2.97 - 2.50 (m, 6H), 2.20 - 1.35 (m, 4H).

### Example 24

### Preparation of (2S,3S)-3-(6-Ethoxy-1-methyl-2-oxo-1,2,3,4-tetrahydroquinolin-7-yl)methylamino-2-phenylpiperidine dihydrochloride

The title compound is prepared in a manner analogous to that procedure disclosed in Example 6 above.

### Example 25

### Preparation of (2S,3S)-3-(6-methoxy-1-methyl-2-oxo-1,2,3,4-tetrahydroquinolin-7-yl)methylamino-2-(4-fluorophenyl)-piperidine dihydrochloride

The title compound is prepared in a manner analogous to that procedure step forth above in Example 5 using (*2S*,*3S*)-2-(4-fluorophenyl)-pipendin-3-amine as starting material.

### Example 26

### Preparation of (2S,3S)-3-[(6-Methoxy-1,3-dimethyl-2-oxo-1,2,3,4-tetrahydroquinolin-7-yl)methyl]amino-2-phenylpiperidine dihydrochloride

The title compound is prepared in a manner analogous to that set forth above in Example 15 using 7-methoxy-1,3-dimethyl-2-oxo-1,2,3,4-tetrahydroquinoline as starting material.

### Example 27

### Preparation of 6-Methoxy-7-[(2-phenyl-piperidin-3-ylamino)-methyl]-3,4-dihydro-1H-quinolin-2-one

### (i) 6-hydroxy-2-oxo-1,2,3,4-tetrahydroquinoline

*p*-Aminophenol [0.5 gm (4.58 mmol)] was dissolved in 30 ml of each methylene chloride and saturated aqueous bicarbonate solution and stirred for 5 minutes at ambient temperature. 3-Chloropropionyl chloride [0.49 ml (5.04 mmol.)] was added over 10 min. and the reaction mixture was stirred at ambient temperature for 4 hrs. A large amount of precipitate was observed. The solids were filtered and dried to afford 0.82 gm ( 90 %) of an off-white solid. MS APCI m/e 200 (p+1). This product (0.82 gm (4.1 mmol.)) was combined with 1.6 gm (12.3 mmol.) aluminum chloride as a mixture of solids. The mixture was then heated in an oil bath at 210 °C for 10 minutes or until the gas evolution ceases. The reaction mixture was then allowed to cool to ambient temperature and then quenched in an ice/water mixture. The aqueous phase was extracted with ethyl acetate which was separated, dried over sodium sulfate and evaporated in vacuo to a light brown solid 0.58 gm (87 %) MS APCI m/e 164 (p+1).

### (ii) Preparation of 6-Methoxy-2-oxo-1,2,3,4-tetrahydroquinoline

A solution of 0.58 gm (3.56 mmol.) 6-hydroxy-2-oxo-1,2,3,4-tetrahydroquinoline was prepared in 10 ml acetone followed by the addition of 1.46 gm (10.58 mmol.) potassium carbonate and 0.51 ml (5.36 mmol.) dimethyl sulfate. The reaction mixture was stirred at ambient temperature for 16 hours and then evaporated in vacuo. The residue was partitioned between saturated aqueous bicarbonate solution and methylene chloride. The organic phase was dried over sodium sulfate and evaporated. The residue was purified by silica gel chromatography eluting with 96/4 methylene chloride/methanol to afford 0.53 gm (85 %) of the desired product as a white solid. MS APCI m/e 178 (p+1).

### (iii) Preparation 6-Methoxy-2-oxo-1,2,3,4-tetrahydro-quinoline-7-carbaldehyde

A mixture of 0.29 gm (2.19 mmol) aluminum chloride in 5 ml of methylene chloride was prepared under N₂ and stirred for 15 minutes and then cooled to 0° C. The mixture was treated with 0.2 gm (1.13 mmol) 6-methoxy-3,4-dihydro-1H-quinolin-2-one in 5 ml of methylene chloride. The reaction mixture was stirred for 10 minutes at this temperature and was then cooled to -5 °C. Dichloromethylmethyl ether 0.28 ml (3.07 mmol) was added over a 5 minute period and the green reaction mixture was slowly warmed to room temperature and stirred for 6 hours. The reaction mixture was diluted with 2N HCl and extracted with methylene chloride (4 X 10 ml). The combined organics were dried over sodium sulfate, filtered and concentrated to yield an off white solid. The crude product was chromatographically purified on silica gel, eluting with 7/3 ethyl acetate / hexane. There was obtained 125 mg (54 %) of an off white solid. MS APCI m/e 206 (p+1).

### (iv) 6-Methoxy-7-[(2-phenyl-piperidin-3-ylamino)-methyl]-3,4-dihydro-1H-quinolin-2-one

To a flame dried round bottom flask fitted with Dean-Stark trap, condenser and nitrogen atmosphere was placed: 66 mg (0.37 mmol) cis-(2S,3S)-3-amino, 2-phenylpiperidine and 77 mg (0.37 mmol) 6-methoxy-2-oxo-1,2,3,4-tetrahydro-quinoline-7-carbaldehyde in 15 ml of toluene containing 3A molecular sieves. The reaction mixture was heated under reflux for 6 hours and monitored by mass spectrum analysis for the presence of the imine intermediate. The reaction mixture was allowed to cool to ambient temperature and then evaporated in vacuo. The residue was taken up in approximately 15 ml of dichloroethane and was treated with 102 mg (0.48 mmol.) sodium triacetoxybrohydride and then stirred for 16 hrs under nitrogen at ambient temperature. The reaction mixture was then washed with aqueous saturated sodium bicarbonate solution, washed with brine and then dried and evaporated in vacuo. The residue was purified by column chromatography on silica gel eluting with 95/5 CHCl₃/methanol containing 3 drops of conc. NH₄OH solution. There was obtained 100 mg of the free base (75 %) which was converted to the above named product as the hydrochloride in the following manner: Treatment of methanol with 3 equivalents (53 µl, 0.82 mmol) acetyl chloride afforded a methanolic HCl solution which was stirred for 10 minutes. The free base was added in methanol and the mixture was again stirred for 10 minutes and then evaporated in vacuo. The residue was taken up in the minimum amount of methanol and treated with ether until a cloudy precipitate form. Upon standing the di-HCl salt was obtained in 46 % overall yield (75 mg). Mp 233-235° C MS, APCI m/e 366 (p+1).

### Example 28 (microsomal biotransformation)

### 4-hydroxy-6-methoxy-1-methyl-7-[(2-phenyl-piperidin-3-yl-amino)-methyl]-3,4-dihydro-1H-quinolin-2-one

The title compound can be produced from the dihydrochloride salt of 6-methoxy-1-methyl-7-[(2-phenyl-piperidin-3-ylamino)-methyl]-3,4-dihydro-1H-quinolin-2-one (i.e., substrate) by using a recombinant insect cell microsomal reaction mixture. The reaction contained the following components:

| | |
|---|---|
| 87.5 mL | 100 mM potassium hydrogen phosphate buffer (pH 7.4) |
| 25 mL | cofactor solution |
| 1.25 mL | 30 mM parent compound (di HCl salt) dissolved in distilled water |
| 10 mL | baculovirus-infected insect cell microsomes co-expressing human P450 (CYP2D6) and human NADPH-cytochrome P450 reductase |
| 1.25 mL | catalase, 900,000 U/mL (e.g., Sigma C-30) |

| 100 mM potassium hydrogen phosphate buffer (final pH 7.4) | |
|---|---|
| 8.1 mL | 100 mM K₂HPO₄ in distilled water |
| 1.9 mL | 100 mM KH₂PO₄ in distilled water |

| cofactor solution | |
|---|---|
| 100 mg | NADP⁺ (e.g., Sigma N-0505) |
| 1.875 g | isocitric acid (e.g., Sigma I-1252) |
| 4.95 mL | isocitric dehydrogenase (e.g., Sigma I-2002) |
| 20.05 mL | 125 mM MgCl₂ in distilled water |

Reaction components were added to a sterile 300-mL Erienmeyer flask with foam plug closure. The flask was incubated vertically on a rotary shaker (1-in. throw) at 150 rpm and 37°C. The progress of the reaction was monitored by analysis of periodic samples using reverse phase high performance liquid chromatography (HPLC Method 1).

| HPLC Method 1 | |
|---|---|
| Column | Symmetry C18, 3.9 x 150 mm. |
| Mobile phase | linear gradient from 2-30 min.; (10-60)% acetonitrile : (90-40)% aqueous buffer [20 mM acetic acid in distilled water, adjusted to pH 4.0 with 1N H₂SO₄]. |
| Flow rate | 1 mL/min. |
| Injection vol. | 25 µL |
| Monitor | UV absorbance at 262 nm; photodiode array at 210-400 nm (4.8 nm slit). |
| Run Time | 30 min. |

Using HPLC Method 1, the title compound had a retention time of approximately 12.6 minutes. The parent compound had a retention time of approximately 15.9 minutes. To stop the reaction and prepare the sample for analysis, a 200 mL sample was added to 200 mL methanol, mixed, cooled on ice for 15 min., and centrifuged (Eppendorf Model 5417C) at 14,000 rpm for 5 minutes to remove precipitated proteins. The reaction was complete by 6.5 hours after initiation. The harvested reaction mixture was added to 375 mL of methanol and refrigerated (4°C) overnight to precipitate proteins. The 500 mL of methanolic mix was filtered by vacuum through a glass fiber filter (Whatman GF/B) to remove solids. The precipitated solids retained on the filter were resuspended with 100 mL of methanol and refiltered. The clarified methanolic filtrates were pooled and stripped of methanol by vacuum distillation at 40°C. The residual aqueous solution, 150 mL, was filtered by vacuum through a 5.5-cm dia. glass fiber filter (Fisher G2) and the filtrate loaded onto a C18 resin SPE (solid phase extraction) cartridge [Waters Sep-Pak 35cc (10g) C18; prepared according to manufacturer's directions]. The loaded SPE cartridge was washed with 80 mL distilled water to remove unbound material. Adsorbed material was eluted off of the resin with a series of three 15 mL aliquots of solutions of methanol in water of increasing organic solvent strength (10, 20, 30, 40, 45, 50, 60, 70, 80, and 100% methanol). The title compound eluted in the third 60%, three 70%, and first 80% methanol fractions. These five fractions (75 mL) were pooled and 10 mL of 100 mM potassium phosphate buffer (pH 7.4) was added. The resultant solution was stripped of methanol by vacuum distillation at 40°C. The aqueous residue was then taken to dryness *in vacuo* (Savant Speed-Vac). The 5.7 mg of dried material containing the title compound was dissolved in 2 mL of a 1:9 MeOH/H₂O solution. From this solution the title compound was isolated using HPLC Method 2.

| HPLC Method 2 | |
|---|---|
| Column | Symmetry C18, 7.8 x 300 mm. |
| Mobile phase | isocratic.; 20% acetonitrile : 80% aqueous buffer [20 mM acetic acid in distilled water, adjusted to pH 4.0 with 1 N H₂SO₄]. |
| Flow rate | 4 mL/min. |
| Injection vol. | 75 µL |
| Monitor | UV absorbance at 262 nm; photodiode array at 210-400 nm (4.8 nm slit). |
| Run Time | 15 min. |

The title compound eluted at approximately 3.7 min. Eluting fractions containing the title compound were saved, pooled, and added to 5 mL 100 mM potassium phosphate buffer (pH 7.4) to yield a volume of 58 mL. The resultant mixture was stripped of acetonitrile by vacuum distillation at 40°C. The aqueous residue was loaded onto a C18 resin SPE cartridge [Waters Sep-Pak 6cc (1g)C18; prepared according to the manufacturer's directions]. The loaded SPE cartridge was washed with 40 mL of distilled water and then with 10 mL of a 10% solution of methanol in water to remove unbound and unwanted material. The title compound was eluted with 15 mL of 100% methanol. This methanol eluate was taken to dryness *in vacuo* (Savant Speed-Vac). The dried material consisted of 1.6 mg of the title compound. This represents an overall recovered molar yield of 10.8%.

The title compound had UV-light absorbance maxima at ≤ 210, 262 nm, and a shoulder beginning at 290 nm. MS (APCI⁺): 396.3 (M+H); ¹H-NMR (free base, 400MHz, CDCl₃) δ 1.10 (3H, t, 7.0 Hz), 1.42-1.45 (1H, m), 1.88-1.95 (1H, m), 2.02-2.12 (1H, m), 2.84 (2H, m), 3.21 (3H, s), 3.27 (1H, m), 3.44 (1H, d, J=14.1Hz), 3.64 (1H, d, J=14.1Hz), 3.54 (3H, s), 3.67 (1H, m), 3.90 (1H, d, 2.1Hz), 4.79 (1H, t), 6.72 (1H, s), 6.75 (1H, s), 7.30-7.31 (5H, m).

### Example 29 (microbial biotransformation)

### 4-hydroxy-6-methoxy-1-methyl-7-[(2-phenyl-piperidin-3-ylamino)-methyl]-3,4-dihydro-1H-quinolin-2-one

An alternative method to produce the title compound from the dihydrochloride salt of 6-methoxy-1-methyl-7-[(2-phenyl-piperidin-3-ylamino)-methyl]-3,4-dihydro-1H-quinolin-2-one (i.e., substrate) is by using microbial biotransformation. Twenty-five mL of IOWA Medium (anhydrous dextrose, 20 g; yeast extract, 5 g; dipotassium hydrogen phosphate, 5 g; sodium chloride, 5 g; soybean flour, 5 g; distilled water, 1 L; adjusted to pH 7.2 with 1N sulfuric acid) were added to each of thirty-five 125-mL DeLong flasks with stainless steel Morton closures and the resulting combinations were steam-sterilized for 30 minutes at 15 psig and 121°C. Five flasks (inoculum stage) were each aseptically inoculated with 0.25 mL of a cryogenically stored (-80°C) axenic stock of *Streptomyces albulus* (ATCC 12757) mycelium. The inoculated flasks were incubated vertically on a rotary shaker (2-in. throw) at 210 rpm and 29°C for 2 days. Then, 2.5 mL of broth from the inoculum stage was aseptically transferred to each of the remaining 30 flasks (biotransformation stage). The inoculated biotransformation flasks were incubated vertically on a rotary shaker (2-in. throw) at 210 rpm and 29°C for 1 day. The dihydrochloride salt of 6-methoxy-1-methyl-7-[(2-phenyl-piperidin-3-ylamino)-methyl]-3,4-dihydro-1H-quinolin-2-one (i.e., substrate) was dissolved in distilled water (7 mg/mL) and sterilized by filtration through a sterile nylon membrane (0.2 µ porosity). To each of the 30 biotransformation flasks, 0.5 mL of the resulting substrate solution was aseptically added to give an initial substrate concentration of 125 mcg/mL (105 mg total in 30 flasks). The dosed flasks were reincubated vertically on the rotary shaker at 210 rpm and 29°C for an additional 7 days. The progress of the biotransformation to title compound was monitored by periodic analysis of 1-mL samples using HPLC Method 1 (see example 1a). At the end of the 7-day biotransformation period, the broth contents of all the flasks was removed and pooled with a small volume rinse of each of the flasks with distilled water (850 mL total). The culture broth was filtered through a layer of cheesecloth and cotton and the clarified filtrate was saved. This filtrate was brought to a volume of 900 mL with distilled water. A series of solid phase extraction (SPE) steps of decreasing scale was used to concentrate the title compound, since taking the title compound to dryness under some circumstances can cause decomposition to a dehydrated product, 6-methoxy-1-methyl-7-[(2-phenyl-piperidin-3-ylamino)-methyl]-1H-quinolin-2-one. [This decomposition product had a retention time of approximately 15.5 min. when analyzed using HPLC Method 1.] The filtrate containing the title compound was loaded under pressure of nitrogen gas (30 psig) onto a C18 resin SPE cartridge [Biotage KP-C18-HS, FLASH 40S, 55g; prepared according to the manufacturer's directions]. The loaded SPE cartridge was washed with 1000 mL distilled water to remove unbound material. Then, the loaded cartridge was washed with 1000 mL of a 10% methanol solution (1:9 MeOH/H₂O) followed by 100 mL of a 20% methanol solution and by 100 mL of a 50% methanol solution to remove unwanted material. The title compound was eluted with 500 mL of 100% methanol. This eluate containing the title compound was combined with 50 mL of 10 mM potassium phosphate buffer (10 mM K₂HPO₄ in H₂O; adjusted to pH 7.0 with H₃PO₄). The resulting solution was distilled under reduced pressure at 45°C to remove methanol. To the aqueous residue 50 mL of 10 mM potassium phosphate buffer (pH 7) was added and then this solution, 160 mL, was loaded onto a C18 resin SPE cartridge [Waters Sep-Pak 35cc (10g) C18; prepared according to the manufacturer's directions]. Compounds bound to the resin were eluted with methanol in water solutions of increasing organic solvent strength (10, 20, 30, 40, 50, 55, 60, 65, 70, 80, 100% MeOH). The title compound eluted from the SPE resin in the 55-65% methanol solution fractions. Those fractions containing only the title compound were saved and pooled (135 mL total). To this pool 15 mL of 10 mM potassium phosphate buffer (pH 7) was added and the resultant solution was distilled under reduced pressure at 45°C to remove methanol. To the aqueous residue 5 mL of 10 mM potassium phosphate buffer (pH 7) was added and this mixture (46 mL) was loaded onto a C18 resin SPE cartridge [Waters Sep-Pak 20cc (5g) C18; prepared according to the manufacturer's directions]. The title compound was eluted with 10 mL of 80:20 MeOH/10 mM K₂HPO₄ (pH7), followed by 20 mL of 100% methanol. The eluant solutions were combined and the volume was reduced under a stream of N₂ gas. The residual 11 mL of liquid containing the title compound was loaded onto a C18 resin SPE cartridge [Waters Sep-Pak 6cc (1g). prepared according to the manufacturer's directions]. The loaded cartridge was washed with 10 mL of a 20% methanol solution in water to remove unwanted material. The title compound was eluted with 10 mL of a 50% methanol solution and 5 mL of a 90% methanol solution. The eluant solutions containing the title compound were pooled and the methanol was stripped using a flow of N₂ gas. The title compound was isolated from the aqueous residue using HPLC Method 3.

| HPLC Method 3 | |
|---|---|
| Column | Luna 5µ C18(2), 21.2 x 250 mm; preceded by Luna 5 µ C8(2), 21.2 x 60 mm guard column. |
| Mobile phase | isocratic; 25% acetonitrile : 75% 10 mM K₂HPO₄, adjusted to pH 7 with H₃PO₄ |
| Flow rate | 14 mL/min. |
| Monitor | UV absorbance at 262 nm; photodiode array at 210-400 nm (4.8 nm slit). |
| Run Time | 12 min. |

The title compound had a retention time of 4.7 minutes. Eluting HPLC mobile phase fractions containing the title compound were collected (total of 240 mL), stripped of acetonitrile by distillation under vacuum at 40°C, and loaded onto a C18 resin SPE cartridge [Waters 6cc (1g) C18; prepared according to the manufacturer's directions]. The loaded SPE cartridge was washed with 40 mL distilled water followed by 25 mL of a 10% methanol solution to remove unwanted material. The title compound was eluted with 10 mL of 100% methanol. This eluate was concentrated to 2.1 mL under a stream of N₂ gas. Three mL of 10 mM potassium phosphate buffer (pH 7) was added to prevent decomposition by dehydration to 6-methoxy-1-methyl-7-[(2-phenyl-piperidin-3-ylamino)-methyl]-1H-quinolin-2-one. Analysis of the resultant solution using HPLC Method 1, determined that it contained 8.7 mg of the title compound. This represents an overall recovered molar yield of 9.5%.

The title compound had UV-light absorbance maxima at ≤ 210, 262 nm, and a shoulder beginning at 290 nm; MS (APCI⁺): 396.2 (M+H) This was determined from the 2.1 mL of methanolic solution prior to the addition of 3 mL of 10 mM potassium phosphate buffer.

The title compound can be prepared similarly using other microorganisms in place of *Streptomyces albulus* ATCC 12757, especially *Mortierella isabellina* ATCC 38063.

The chemical structures of the compounds prepared in Examples 1 to 29 are summarized in the following table.

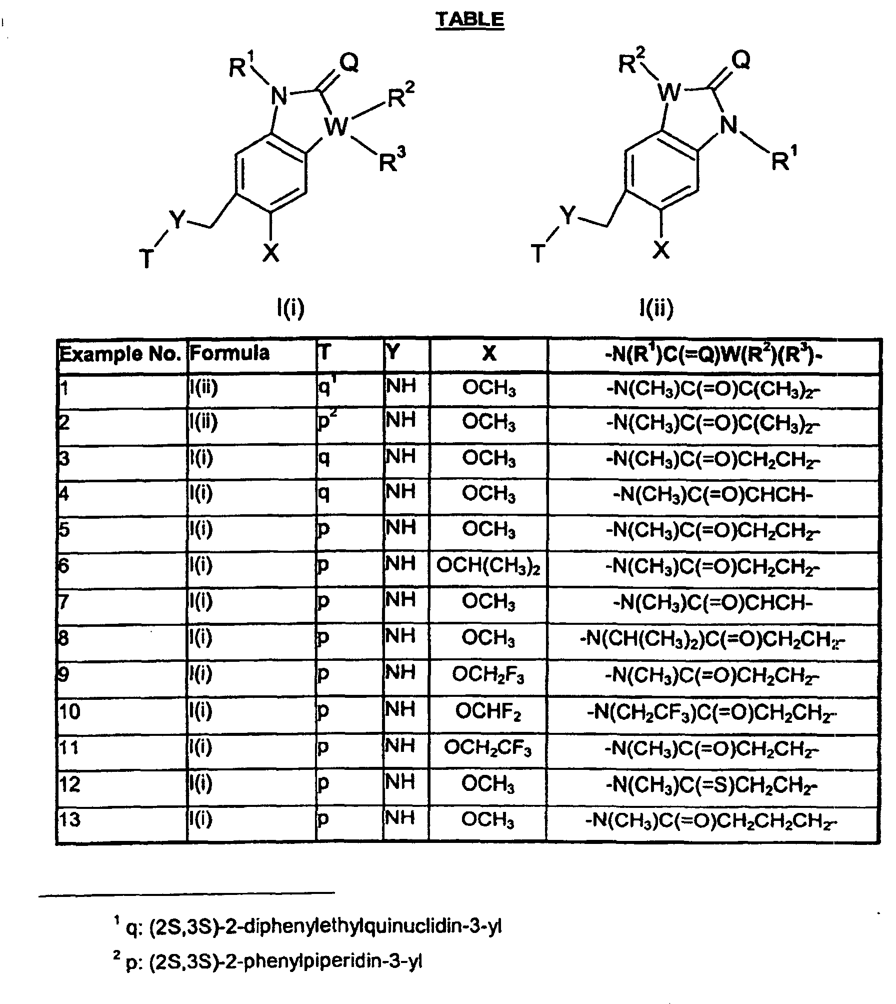

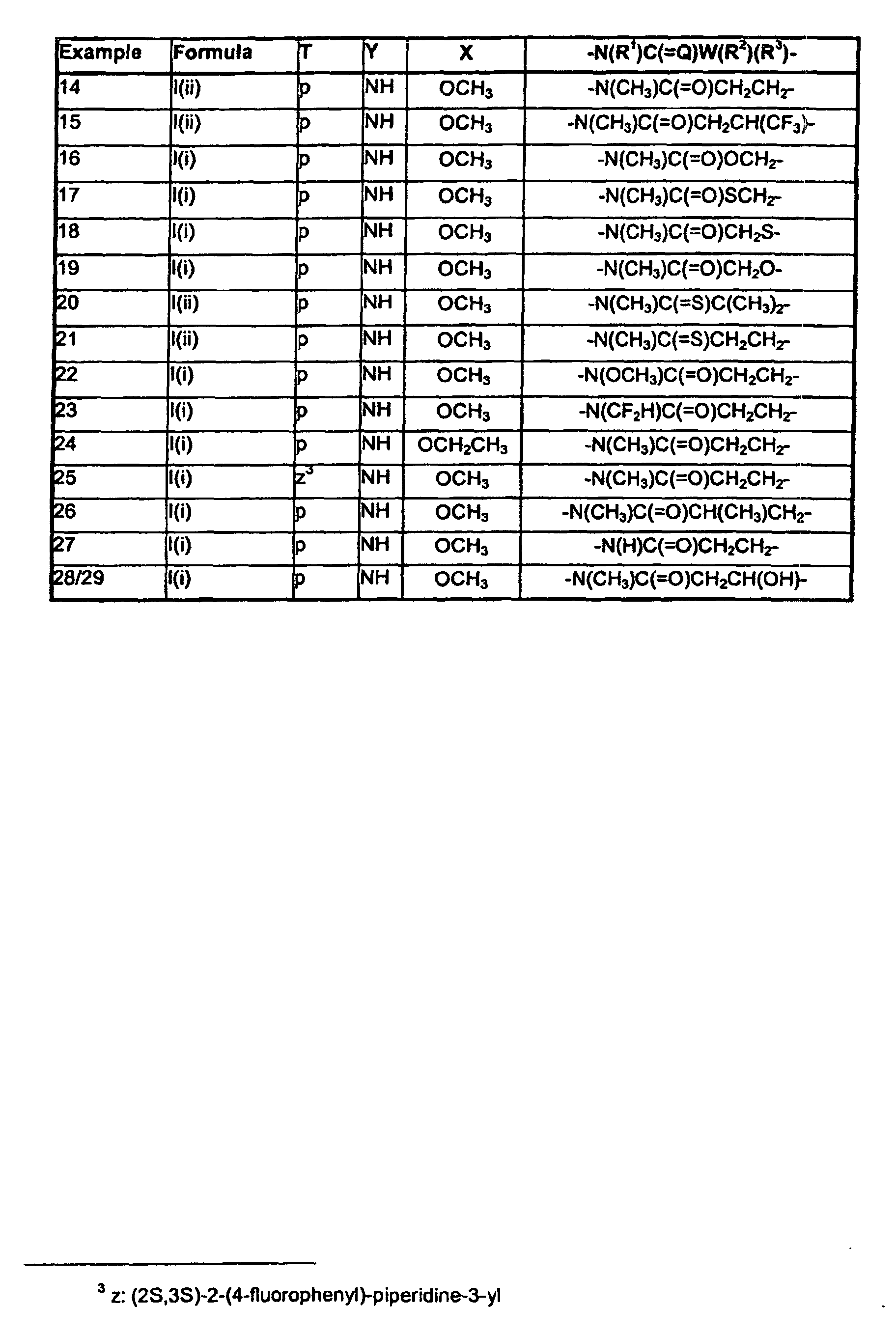

## Claims

1. The use of a compound of the chemical formula (I): 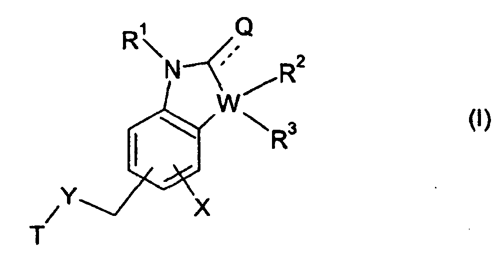 wherein W is methylene, ethylene, propylene, vinylene, -CH₂O-, -OCH₂-, -CH₂S-, or -SCH₂-;
R¹, R² and R³ are independently hydrogen, (C₁-C₃)alkyl, (C₁-C₃)alkoxy(C₁-C₃)alkyl, or halo(C₁-C₃)alkyl, provided that when W is methylene, R² and R³ cannot both be hydrogen;
or one of R² or R³ may be hydroxy;
X is halo, (C₁-C₃)alkoxy, (C₁-C₃)alkyl, halo(C₁-C₃)alkoxy, or (C₁-C₃)alkenyl;
Y is -N H- or -O-;
Q is oxygen or sulfur and is double bonded to the carbon to which it is attached or Q is CH₃ and is single bonded to the carbon to which it is attached;
T is (*2S*,*3S*)-2-diphenylmethylquinuclidin-3-yl, (*2S*,*3S*)-2-diphenylmethyl-1-azanorboman-3-yl; or (*2S*,*3S*)-2-phenylpiperidin-3-yl, wherein the phenyl group of said (2S, 3S)-2-phenylpiperidine-3-yl may optionally be substituted with one or more substituents, preferably with from zero to three substituents independently selected from halo, (C₁-C₆)alkyl optionally substituted with from one to seven fluorine atoms, (C₁-C₆)alkoxy optionally substituted with from one to seven fluorine atoms, amino, cyano, nitro, (C₁-C₆)alkylamino and di(C₁-C₆)alkylamino; and
the dashed line represents an optional double bond;
with the proviso that R¹ cannot be (C₁-C₃)alkoxy-CH₂- or halo-CH₂-;
or a pharmaceutically acceptable salt thereof,
in the manufacture of a medicament for treating a disorder or condition selected from obsessive-compulsive disorder, panic disorder, social phobia, agoraphobia, post traumatic stress disorder, borderline personality disorder, inflammation of the urinary tract, conduct disorder, disruptive behavior disorder, bipolar disorder, movement disorders associated with Tourette's syndrome, akinetic-rigid syndrome, movement disorders associated with Parkinson's disease, tardive dyskinesia, eating disorders selected from anorexia nervosa and bulimia nervosa, attention deficit hyperactivity disorder, chronic fatigue syndrome, premature ejaculation, premenstrual syndrome, premenstrual dysphoric disorder, gastroesophageal reflux disease, fibromyalgia, post-herpetia neuralgia, cystitis and irritable bowel syndrome, in a mammal.

2. Use according to Claim 1 wherein, in the compound of formula (I),
Y is -NH-;
T is (2S,3S)-2-phenylpiperidin-3-yl, where the phenyl group of said (2S,3S)-2-phenylpiperidine-3-yl may optionally be substituted with fluoro;
Q is oxygen and is double bonded to the carbon atom to which it is attached;
X is methoxy or ethoxy;
R¹ is hydrogen, methyl, or halo(C₁-C₂)alkyl;
W is methylene, ethylene, or vinylene; and
R² and R³ are independently hydrogen or methyl, or one of R² or R³ may be hydroxy, when W is ethylene, or R² and R³ are both methyl, when W is methylene, or R² and
R³ are both hydrogen, when W is vinylene.

3. Use according to Claim 2 wherein the compound of formula (I) is selected from the following compounds and their pharmaceutically acceptable salts:

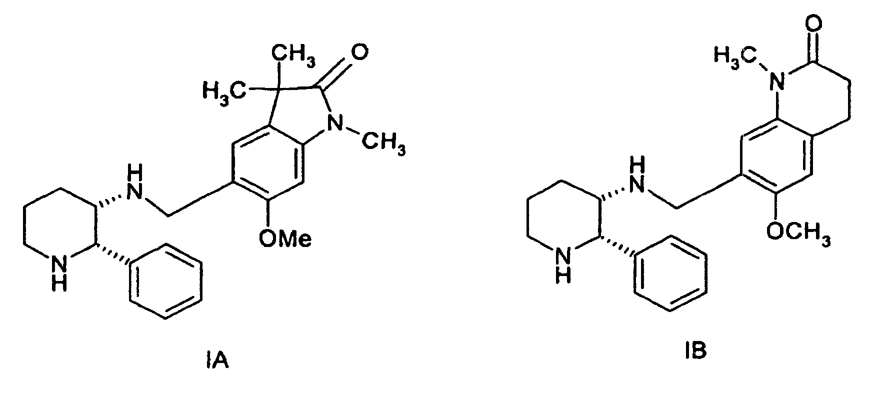

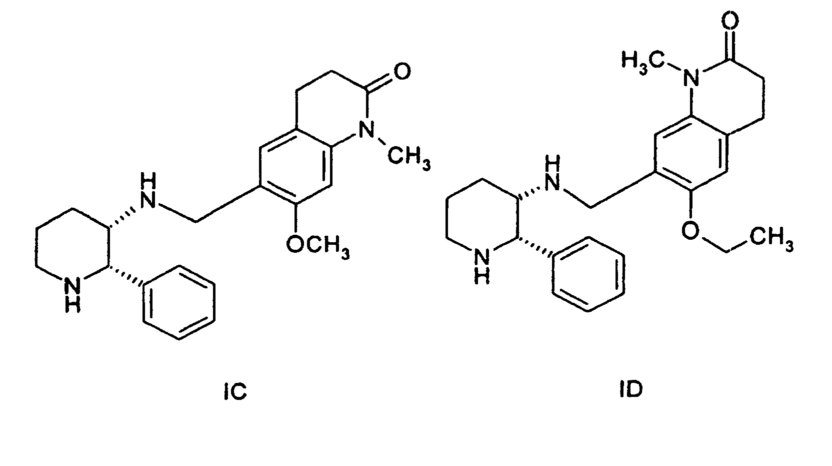

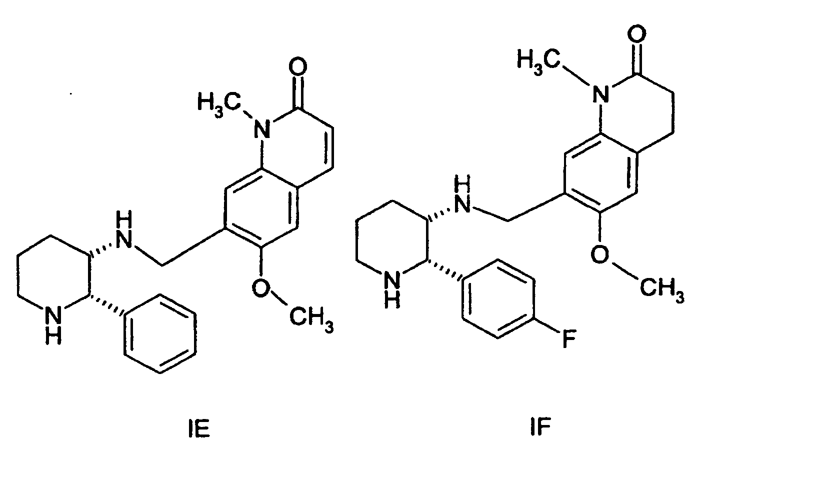

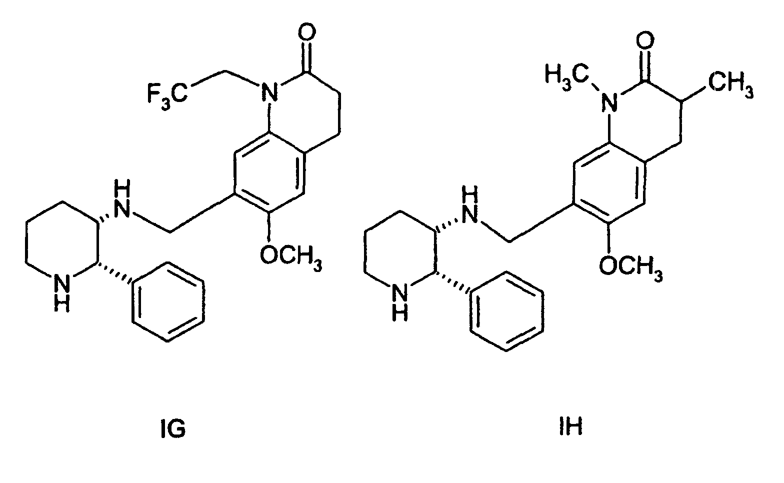

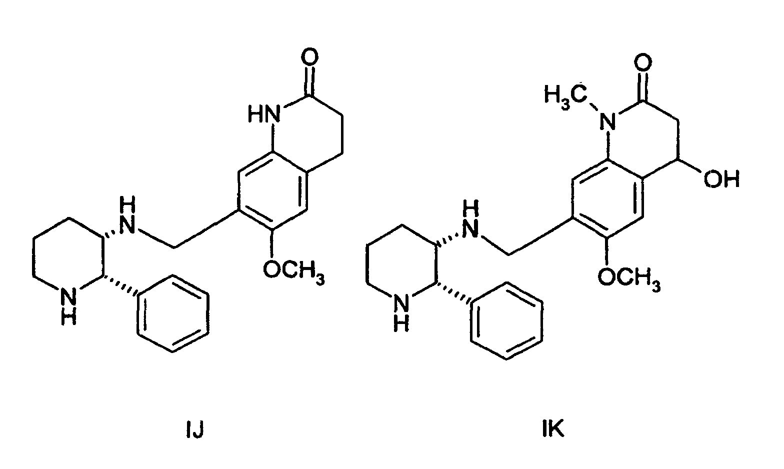

4. A compound of formula (I) as shown in Claim 1 having the formula ID 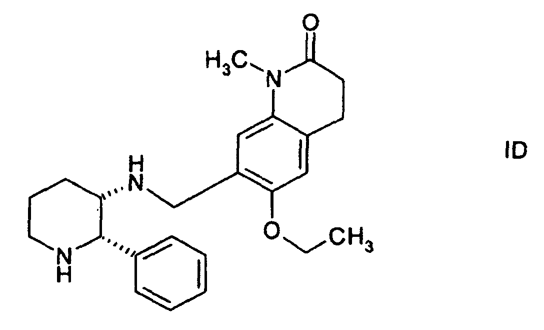 or a pharmaceutically acceptable salt thereof.

5. A compound of formula (I) as shown in Claim 1 having the formula IF 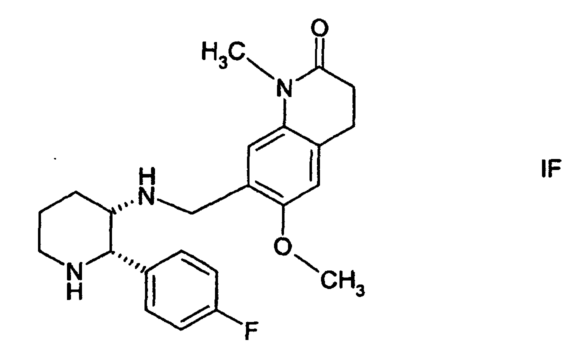 or a pharmaceutically acceptable salt thereof.

6. A compound of formula (I) as shown in Claim 1 having the formula IG 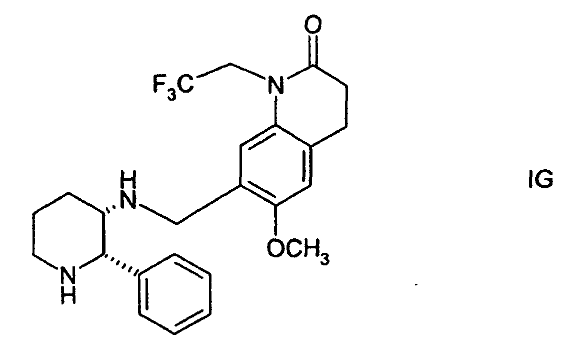 or a pharmaceutically acceptable salt thereof.

7. A compound of formula (I) as shown in Claim 1 having the formula IH 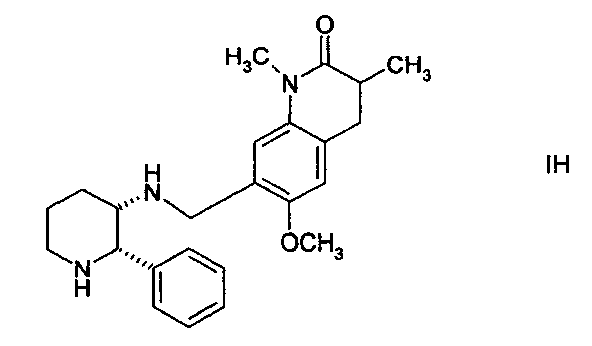 or a pharmaceutical acceptable salt thereof.

8. A compound of formula (I) as shown in Claim 1 having the formula IJ 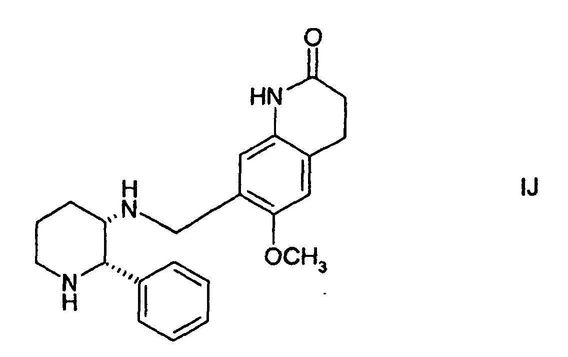 or a pharmaceutically acceptable salt thereof.

9. A compound of formula (I) as shown in Claim 1 having the formula IK 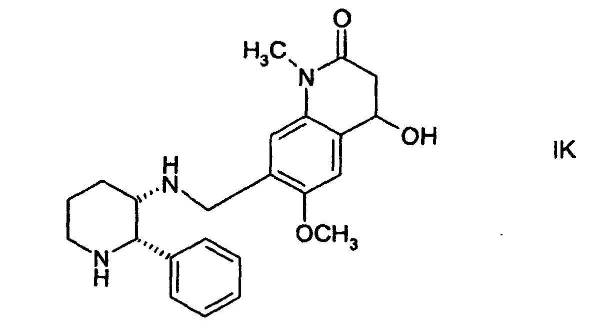 or a pharmaceutically acceptable salt thereof.

10. The use of a compound according to any of Claims 4 to 9 in the manufacture of a medicament for treating a disorder or condition selected from dysthymia, major depressive order, pediatric depression, generalized anxiety disorder, obsessive-compulsive disorder, panic disorder, phobias, social phobia, agoraphobia, post traumatic stress disorder, borderline personality disorder, cardiovascular disorders, ophthalmic disorders, inflammation of the urinary tract, conduct disorder, disruptive behavior disorder, bipolar disorder, movement disorders associated with Tourette's syndrome, akinetic-rigid syndrome, movement disorders associated with Parkinson's disease, tardive dyskinesia, memory disorders, eating disorders selected from anorexia nervosa and bulimia nervosa; attention deficit hyperactivity disorder, chronic fatigue syndrome, premature ejaculation, premenstrual syndrome, premenstrual dysphoric disorder, chemical dependencies and addictions, gastroesophageal reflux disease, fibromyalgia, post-herpetia neuralgia, cystitis and irritable bowel syndrome, in a mammal.

11. A pharmaceutical composition comprising a compound according to any of Claims 4 to 9 or a pharmaceutically acceptable salt thereof and a pharmaceutically acceptable carrier.

12. A precursor compound which is selected from the group consisting of
5-methoxy-3,3-dimethyl-2-oxo-2,3-dihydro-1H-indole-6-carbaldehyde;
6-ethoxy-1-methyl-2-oxo-1,2,3,4-tetrahydroquinoline-7-carbaldehyde; and
6-methoxy-1,3-dimethyl-2-oxo-1,2,3,4-tetrahydroquinoline-7-carbaldehyde.

## Patentansprüche

1. Verwendung einer Verbindung der chemischen Formel (I): 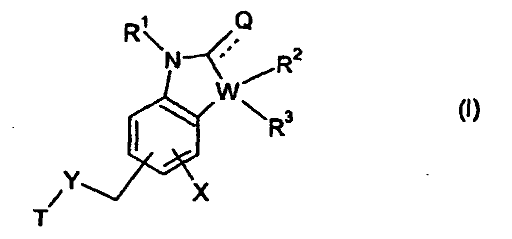 worin W Methylen, Ethylen, Propylen, Vinylen, -CH₂O-, -OCH₂-, -CH₂S- oder -SCH₂- bedeutet;
R¹, R² und R³ unabhängig voneinander Wasserstoff, (C₁-C₃)Alkyl, (C₁-C₃)Alkoxy(C₁-C₃)alkyl oder Halogen(C₁-C₃)alkyl bedeuten, wobei, wenn W Methylen ist, R² und R³ nicht beide Wasserstoff sein können;
oder einer der Reste R² oder R³ Hydroxy sein kann;
X Halogen, (C₁-C₃)Alkoxy, (C₁-C₃)Alkyl, Halogen(C₁-C₃)-alkoxy oder (C₁-C₃)Alkenyl bedeutet;
Y -NH- oder -O- bedeutet;
Q Sauerstoff oder Schwefel bedeutet und über eine Doppelbindung an den Kohlenstoff, an dem es befestigt ist, gebunden ist, oder Q CH₃ bedeutet und über eine Einfachbindung an den Kohlenstoff, an dem es befestigt ist, gebunden ist;
T (2S,3S)-2-Diphenylmethylchinuclidin-3-yl, (2S,3S)-2-Diphenylmethyl-1-azanorbornan-3-yl oder (2S,3S)-2-Phenylpiperidin-3-yl bedeutet, wobei die Phenylgruppe von (2S,3S)-2-Phenylpiperidin-3-yl optional mit einem oder mehreren Substituenten, vorzugsweise mit null bis drei Substituenten, die unabhängig voneinander aus Halogen, optional mit ein bis sieben Fluoratomen substituiertem (C₁-C₆)Alkyl, optional mit ein bis sieben Fluoratomen substituiertem (C₁-C₆)Alkoxy, Amino, Cyano, Nitro, (C₁-C₆)Alkylamino und Di(C₁-C₆)alkylamino ausgewählt sind, substituiert sein kann; und
die gestrichelte Linie für eine optionale Doppelbindung steht;
mit dem Vorbehalt, dass R¹ nicht (C₁-C₃)Alkoxy-CH₂- oder Halogen-CH₂- sein kann;
oder eines pharmazeutisch akzeptablen Salzes derselben; bei der Herstellung eines Medikaments zur Behandlung einer Erkrankung oder eines Zustandes, der ausgewählt ist aus obsessiven Zwangsstörungen, Panikstörungen, sozialen Phobien, Agoraphobie, posttraumatischen Stresserkrankungen, Borderline-Persönlichkeitsstörungen, Entzündungen des Harntrakts, Verhaltensstörungen, Zerstörungsverhaltensstörungen, bipolaren Störungen, akinetic-rigid-Syndrom, Bewegungsstörungen, die mit dem Tourette-Syndrom in Verbindung stehen, Bewegungsstörungen, die mit Parkinson-Krankheit in Verbindung stehen, tardiver Dyskinesie, Essstörungen, die aus Anorexia nervosa und Bulimia nervosa ausgewählt sind, Aufmerksamkeitsdefizithyperaktivitätsstörungen, chronischem Erschöpfungssyndrom, vorzeitiger Ejakulation, prämenstruellem Syndrom, prämenstruellen Dysphoriestörungen, Gastroösophagus-Reflux-Krankheit, Fibromyalgie, Post-Herpes-Neuralgie, Cystitis und Reizdarmsyndrom bei einem Säuger.

2. Verwendung nach Anspruch 1, wobei in der Verbindung der Formel (I)
Y -NH- bedeutet;
T (2S,3S)-2-Phenylpiperidin-3-yl bedeutet, wobei die Phenylgruppe von (2S,3S)-2-Phenylpiperidin-3-yl optional mit Fluor substituiert sein kann;
Q Sauerstoff bedeutet und über eine Doppelbindung an das Kohlenstoffatom, an dem es befestigt ist, gebunden ist;
X Methoxy oder Ethoxy bedeutet;
R¹ Wasserstoff, Methyl oder Halogen(C₁-C₂)alkyl bedeutet;
W Methylen, Ethylen oder Vinylen bedeutet; und
R² und R³ unabhängig voneinander Wasserstoff oder Methyl bedeuten oder einer der Reste von R² oder R³ Hydroxy sein kann, wenn W Ethylen ist, oder R² und R³ beide Methyl sind, wenn W Methylen ist, oder R² und R³ beide Wasserstoff bedeuten, wenn W Vinylen ist.

3. Verwendung nach Anspruch 2, wobei die Verbindung der Formel (I) aus den folgenden Verbindungen und deren pharmazeutisch akzeptablen Salzen ausgewählt ist:

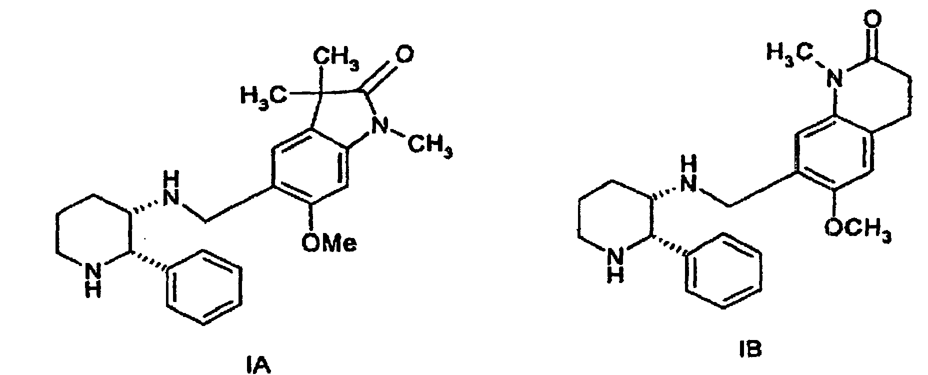

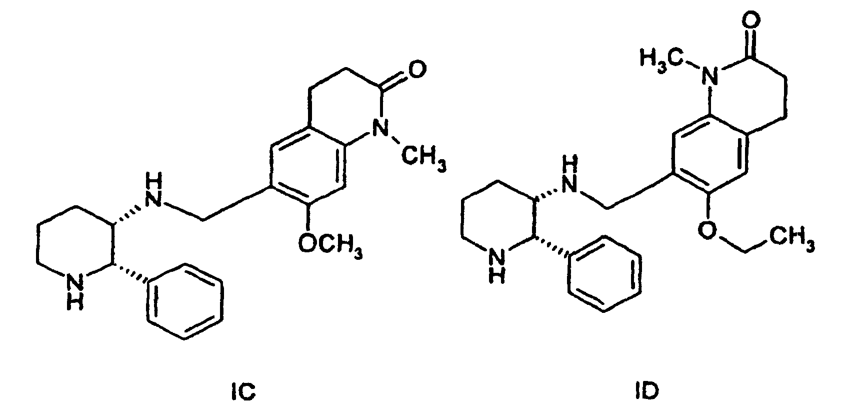

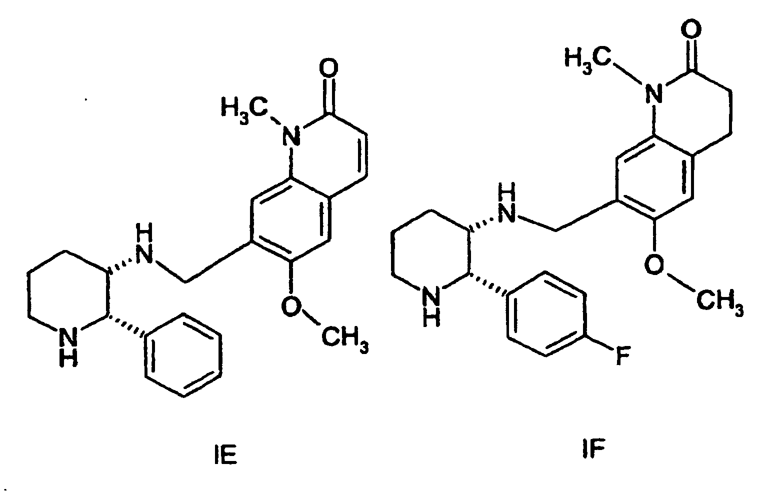

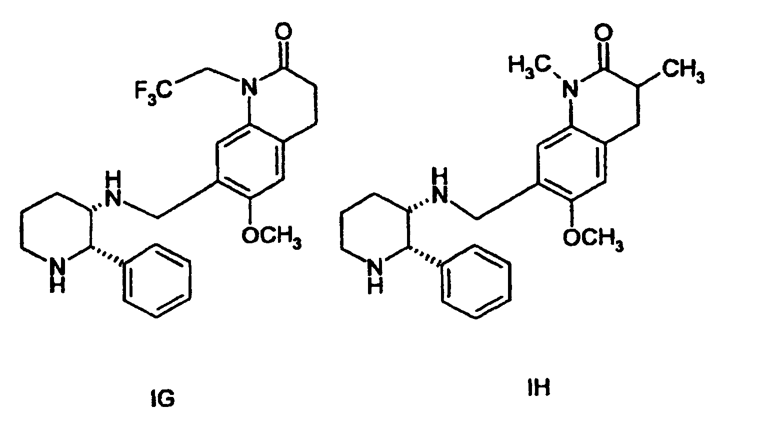

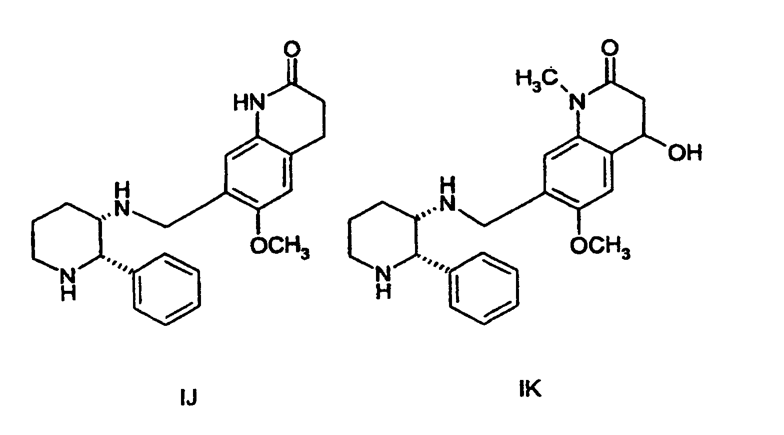

4. Verbindung der Formel (I), die in Anspruch 1 angegeben ist, mit der Formel ID 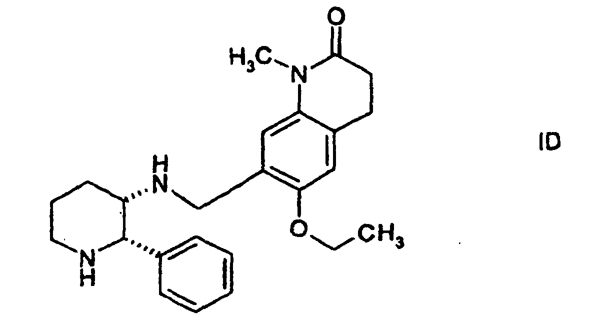 oder ein pharmazeutisch akzeptables Salz derselben.

5. Verbindung der Formel (I), die in Anspruch 1 angegeben ist, mit der Formel IF 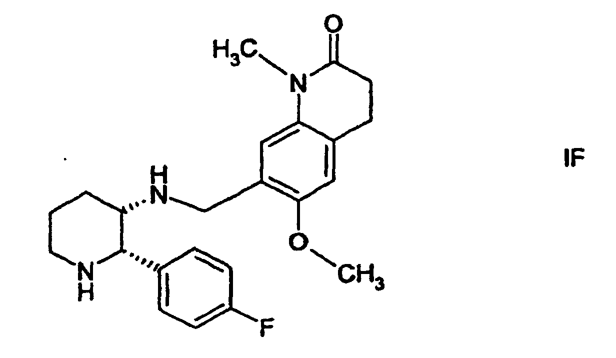 oder ein pharmazeutisch akzeptables Salz derselben.

6. Verbindung der Formel (I), die in Anspruch 1 angegeben ist, mit der Formel IG 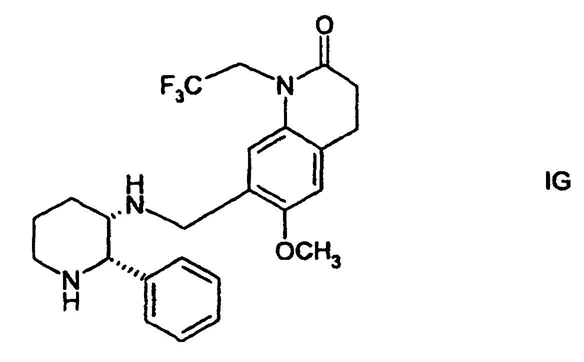 oder ein pharmazeutisch akzeptables Salz derselben.

7. Verbindung der Formel (I), die in Anspruch 1 angegeben ist, mit der Formel IH 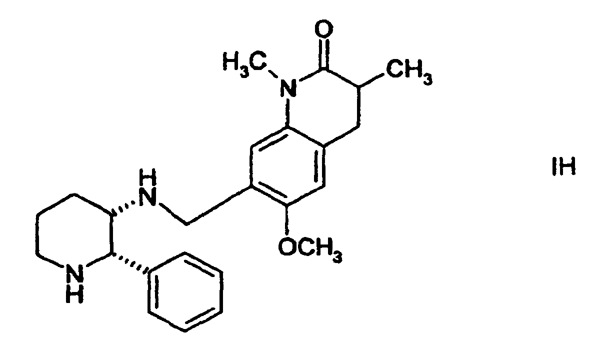 oder ein pharmazeutisch akzeptables Salz derselben.

8. Verbindung der Formel (I), die in Anspruch 1 angegeben ist, mit der Formel IJ 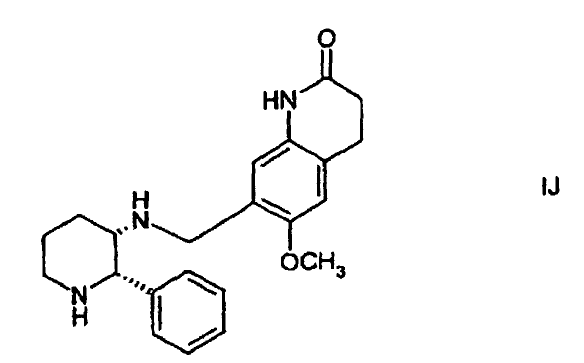 oder ein pharmazeutisch akzeptables Salz derselben.

9. Verbindung der Formel (I), die in Anspruch 1 angegeben ist, mit der Formel IK 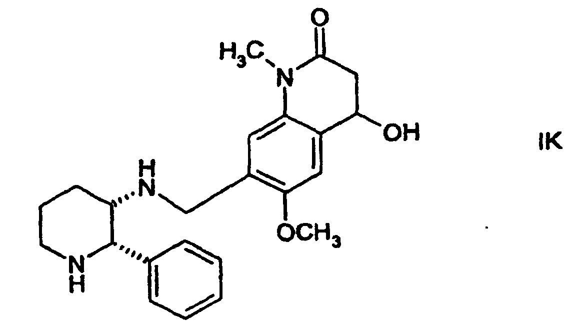 oder ein pharmazeutisch akzeptables Salz derselben.

10. Verwendung einer Verbindung nach einem der Ansprüche 4 bis 9 bei der Herstellung eines Medikaments zur Behandlung einer Erkrankung oder eines Zustandes, die ausgewählt sind aus Dysthymie, starker Depression, pädiatrischer Depression, generalisierten Angststörungen, obsessiven Zwangsstörungen, Panikstörungen, Phobien, sozialen Phobien, Agoraphobie, posttraumatischen Stresserkrankungen, Borderline-Persönlichkeitsstörungen, kardiovaskulären Erkrankungen, ophthalmologischen Erkrankungen, Entzündungen des Harntrakts, Verhaltensstörungen, Zerstörungsverhaltensstörungen, bipolaren Störungen, Bewegungsstörungen, die mit dem Tourette-Syndrom in Verbindung stehen, akinetic-rigid-Syndrom, Bewegungsstörungen, die mit Parkinson-Krankheit in Verbindung stehen, tardiver Dyskinesie, Gedächtnisstörungen, Essstörungen, die aus Anorexia nervosa und Bulimia nervosa ausgewählt sind, Aufmerksamkeitsdefizithyperaktivitätsstörungen, chronischem Erschöpfungssyndrom, vorzeitiger Ejakulation, prämenstruellem Syndrom, prämenstruellen Dysphoriestörungen, chemischen Abhängigkeiten und Suchtzuständen, Gastroösophagus-Reflux-Krankheit, Fibromyalgie, Post-Herpes-Neuralgie, Cystitis und Reizdarmsyndrom bei einem Säuger.

11. Pharmazeutische Zusammensetzung, die eine Verbindung nach einem der Ansprüche 4 bis 9 oder ein pharmazeutisch akzeptables Salz derselben und einen pharmazeutisch akzeptablen Träger umfasst.

12. Vorläuferverbindung, die aus der aus
5-Methoxy-3,3-dimethyl-2-oxo-2,3-dihydro-1H-indol-6-carbaldehyd,
6-Ethoxy-1-Methyl-2-oxo-2,2,3,4-tetrahydrochinolin-7-carbaldehyd und
6-Methoxy-1,3-dimethyl-2-oxo-1,2,3,4-tetrahydrochinolin-7-carbaldehyd
bestehenden Gruppe ausgewählt ist.

## Revendications

1. Utilisation d'un composé de formule chimique (I) : 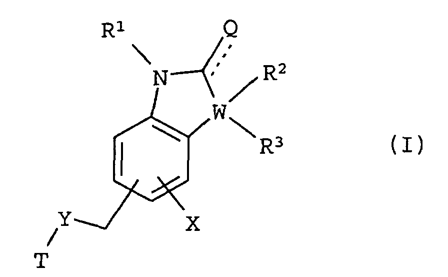 dans laquelle W représente un groupe méthylène, éthylène, propylène, vinylène, -CH₂O-, -OCH₂-, -CH₂S- ou -SCH₂- ;
R¹, R² et R³ représentent indépendamment un atome d'hydrogène, un groupe alkyle en C₁ à C₃, (alkoxy en C₁ à C₃) (alkyle en C₁ à C₃) ou halogénalkyle en C₁ à C₃, sous réserve que, lorsque W représente un groupe méthylène, R² et R³ ne puissent représenter l'un et l'autre un atome d'hydrogène ;
ou bien un des groupes R² et R³ peut représenter un groupe hydroxy ;
X représente un groupe halogéno, alkoxy en C₁ à C₃, alkyle en C₁ à C₃, halogénalkoxy en C₁ à C₃ ou alcényle en C₁ à C₃ ;
Y représente un groupe -NH- ou -O- ;
Q représente un atome d'oxygène ou de soufre et est doublement lié à l'atome de carbone auquel il est fixé, ou bien Q représente un groupe CH₃ et est lié par une liaison simple à l'atome de carbone auquel il est fixé ;
T représente un groupe (2S,3S)-2-diphénylméthylquinuclidine-3-yle, (2S,3S)-2-diphénylméthyl-1-aza-norbornane-3-yle ou (2S,3S)-2-phénylpipéridine-3-yle, dans lequel le groupe phényle dudit groupe (2S,3S)-2-phénylpipéridine-3-yle peut être facultativement substitué avec un ou plusieurs substituants, de préférence avec zéro à trois substituants choisis indépendamment entre des substituants halogéno, alkyle en C₁ à C₆ facultativement substitué avec 1 à 7 atomes de fluor, alkoxy en C₁ à C₆ facultativement substitué avec 1 à 7 atomes de fluor, amino, cyano, nitro, alkylamino en C₁ à C₆ et di(alkyle en C₁ à C₆) amino ; et
la ligne discontinue représente une double liaison facultative ;
sous réserve que R¹ ne puisse représenter un groupe (alkoxy en C₁ à C₃)-CH₂- ou halogéno-CH₂- ;
ou d'un de ses sels pharmaceutiquement acceptables,
dans la production d'un médicament destiné au traitement d'un trouble ou d'une affection choisi entre le trouble obsessionnel compulsif, la panique, la névrose phobique, l'agoraphobie, le syndrome de stress post-traumatique, un trouble de la personnalité à la limite de la normale, l'inflammation du tractus urinaire, un trouble de la conduite, un trouble de comportement perturbé, un trouble bipolaire, des troubles du mouvement associés au syndrome de Tourette, le syndrome de rigidité akinésique, des troubles du mouvement associés à la maladie de Parkinson, la dyskinésie tardive, des troubles de l'alimentation choisis entre l'anorexie mentale et la boulimie mentale, un trouble d'hyperactivité avec déficit d'attention, le syndrome de fatigue chronique, l'éjaculation prématurée, le syndrome prémenstruel, un trouble dysphorique prémenstruel, la maladie de reflux gastro-oesophagien, la fibromyalgie, les névralgies post-herpétiques, la cystite et le syndrome du côlon irritable, chez un mammifère.

2. Utilisation suivant la revendication 1, dans laquelle, dans le composé de formule (I) :
Y représente un groupe -NH- ;
T représente un groupe (2S,3S)-2-phénylpipéridine-3-yle, dans lequel le groupe phényle dudit groupe (2S,3S)-2-phénylpipéridine-3-yle peut être facultativement substitué avec un substituant fluoro ;
Q représente un atome d'oxygène et est doublement lié à l'atome de carbone auquel il est fixé ;
X représente un groupe méthoxy ou éthoxy ;
R¹ représente un atome d'hydrogène, un groupe méthyle ou halogénalkyle en C₁ à C₂ ;
W représente un groupe méthylène, éthylène ou vinylène ; et
R² et R³ représentent indépendamment un atome d'hydrogène ou un groupe méthyle, ou bien un des groupes R² et R³ peut représenter un groupe hydroxy, lorsque W représente un groupe éthylène, ou bien R² et R³ représentent l'un et l'autre un groupe méthyle, lorsque W représente un groupe méthylène, ou bien R² et R³ représentent l'un et l'autre un atome d'hydrogène, lorsque W représente un groupe vinylène.

3. Utilisation suivant la revendication 2, dans laquelle le composé de formule (I) est choisi parmi les composés suivants et leurs sels pharmaceutiquement acceptables :

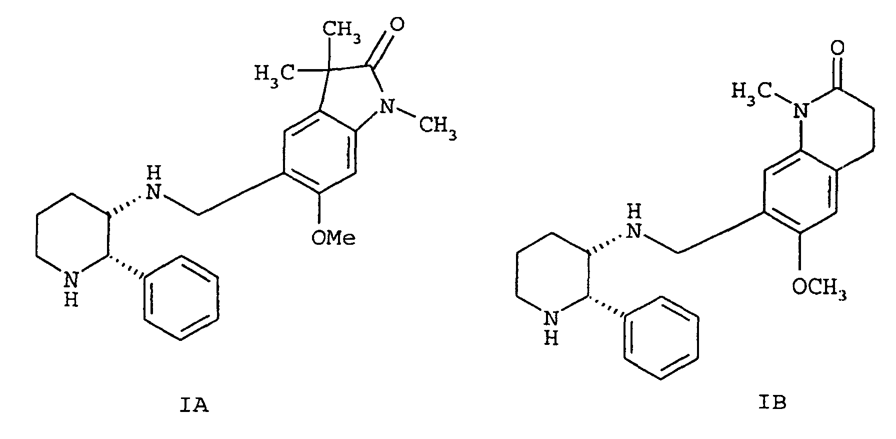

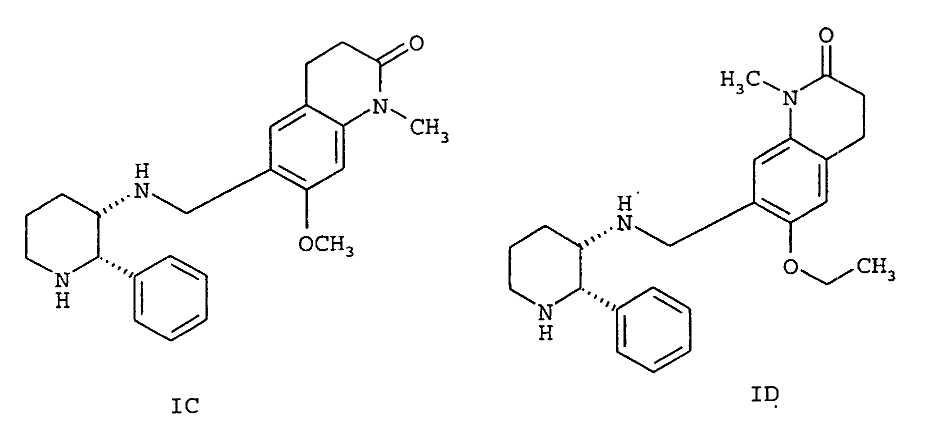

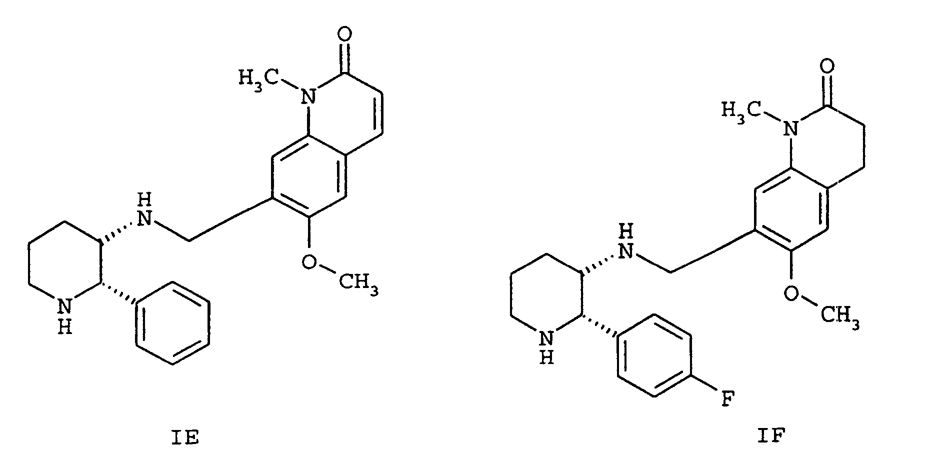

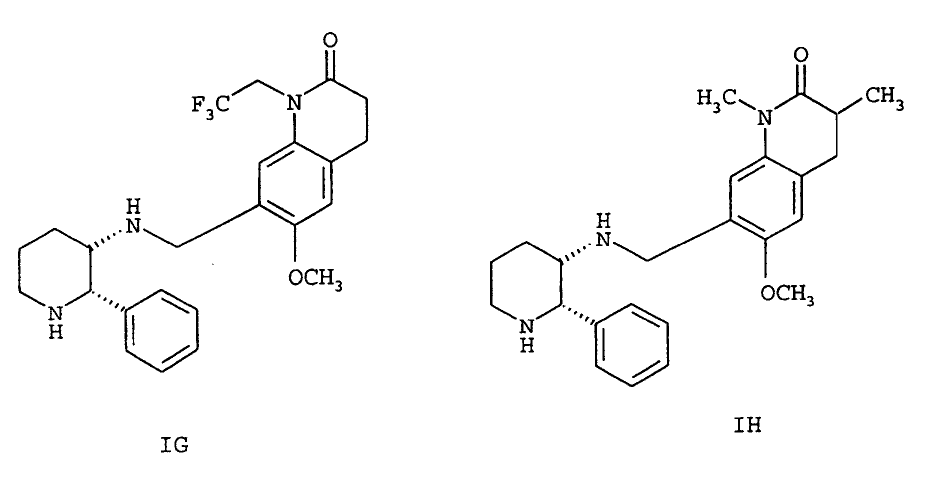

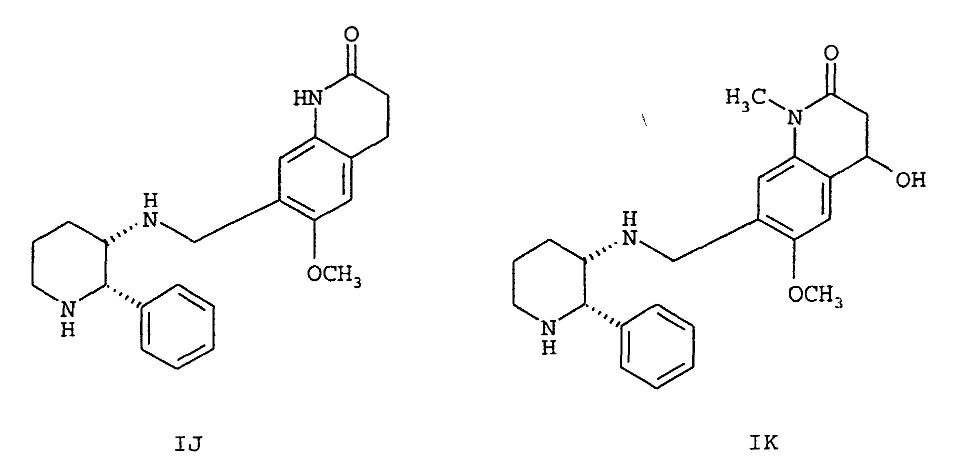

4. Composé de formule (I) suivant la revendication 1, répondant à la formule ID 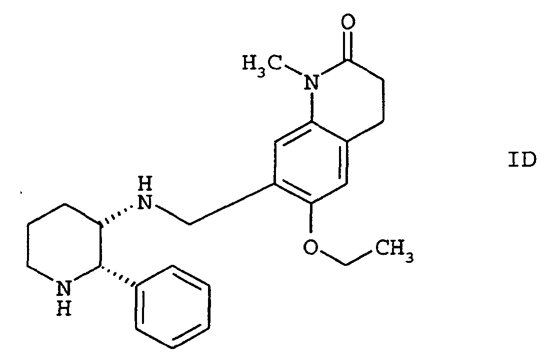 ou un de ses sels pharmaceutiquement acceptables.

5. Composé de formule (I) tel que représenté dans la revendication 1, répondant à la formule IF 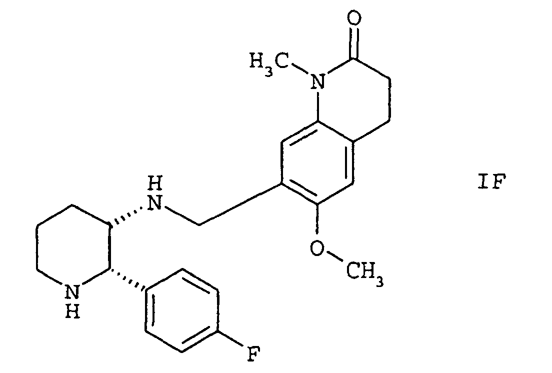 ou un de ses sels pharmaceutiquement acceptables.

6. Composé de formule (I) tel que représenté dans la revendication 1, répondant à la formule IG 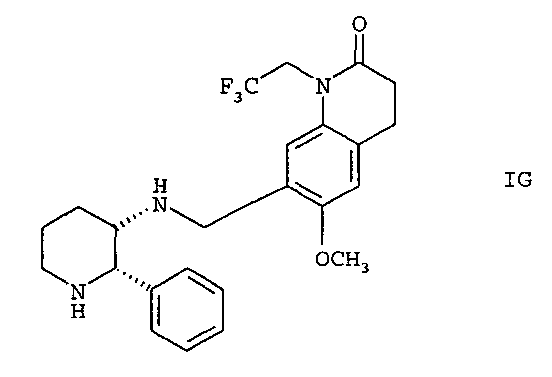 ou un de ses sels pharmaceutiquement acceptables.

7. Composé de formule (I) tel que représenté dans la revendication 1, répondant à la formule IH 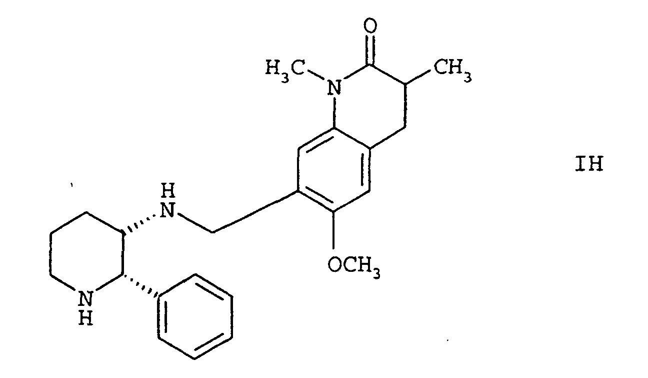 ou un de ses sels pharmaceutiquement acceptables.

8. Composé de formule (I) tel que représenté dans la revendication 1, répondant à la formule IJ 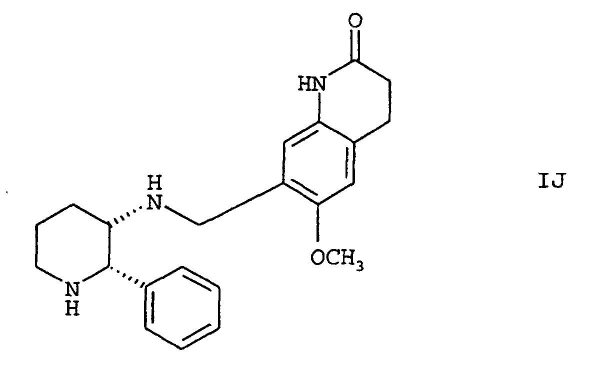 ou un de ses sels pharmaceutiquement acceptables.

9. Composé de formule (I) tel que représenté dans la revendication 1, répondant à la formule IK 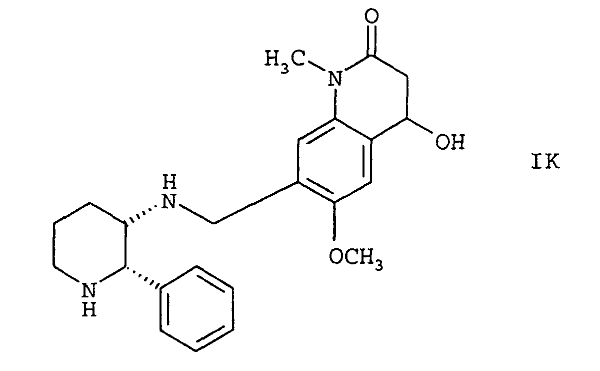 ou un de ses sels pharmaceutiquement acceptables.

10. Utilisation d'un composé suivant l'une quelconque des revendications 4 à 9 dans la production d'un médicament destiné au traitement d'un trouble ou d'une affection choisi entre la dysthymie, un trouble dépressif majeur, la dépression pédiatrique, un trouble d'anxiété généralisée, un trouble obsessionnel compulsif, la panique, les phobies, la névrose phobique, l'agoraphobie, le syndrome de stress post-traumatique, un trouble de la personnalité à la limite de la normale, des troubles cardio-vasculaires, des troubles ophtalmiques, une inflammation du tractus urinaire, un trouble de la conduite, un trouble de comportement perturbé, un trouble bipolaire, des troubles du mouvement associés au syndrome de Tourette, le syndrome de rigidité akinésique, des troubles du mouvement associés à la maladie de Parkinson, la dyskinésie tardive, des troubles de la mémoire, des troubles de l'alimentation choisis entre l'anorexie mentale et la boulimie mentale, un trouble d'hyperactivité avec déficit d'attention, le syndrome de fatigue chronique, l'éjaculation prématurée, le syndrome prémenstruel, un trouble dysphorique prémenstruel, des dépendances et accoutumances chimiques, la maladie de reflux gastro-oesophagien, la fibromyalgie, les névralgies post-herpétiques, la cystite et le syndrome du côlon irritable, chez un mammifère.

11. Composition pharmaceutique comprenant un composé suivant l'une quelconque des revendications 4 à 9 ou un de ses sels pharmaceutiquement acceptables et un support pharmaceutiquement acceptable.

12. Composé précurseur qui est choisi dans le groupe consistant en
5-méthoxy-3,3-diméthyl-2-oxo-2,3-dihydro-1H-indole-6-carbaldéhyde ;
6-éthoxy-1-méthyl-2-oxo-1,2,3,4-tétrahydroquinoléine-7-carbaldéhyde ; et
6-méthoxy-1,3-diméthyl-2-oxo-1,2,3,4-tétrahydroquinoléine-7-carbaldéhyde.
